# EUROPEAN PATENT APPLICATION

(11) **EP 3 567 050 A1**
(43) Date of publication of application: **13.11.2019**
(21) Application number: 18170969.2
(22) Date of filing: 07.05.2018
(51) Int. Cl.: C07K 16/00, C12N 15/10, C40B 40/08

(54) **A COMBINED TANDEM- AND DUAL MANUFACTURING SYSTEM FOR HUMAN ANTIBODY ENGINEERING OPERATING WITH B-CELL RECEPTOR PRESENTATION**

(71) Applicant: Genovie AB, 371 33 Karlskrona (SE)
(72) Inventor: Jarvis, Reagan Micheal, 371 33 Karlskrona (SE); Hill, Ryan Edward, 371 33 Karlskrona (SE); Pase, Luke Benjamin, 371 33 Karlskrona (SE); Uchtenhagen, Hannes, 371 33 Karlskrona (SE)
(74) Representative: Chas. Hude A/S

(57) **Abstract**

The present invention relates to a tandem-manufacturing system for generating antibody sequences, comprising
a. an input part,
b. a first tandem-manufacturing part,
c. an output part, and
d. a second tandem-manufacturing part.

## Description

### Field of the Invention

The present invention relates to the provision of an antibody engineering system (AES) that presents a near-native human antibody repertoire in the B-cell receptor (BCR) context on the surface of human engineered BCR-presenting cells (eBPC). The antibody candidate sequence libraries are prepared and manipulated in the context of efficient recombinase-mediated cassette exchange (RMCE) vector libraries within an antibody open reading frame (ORF) construction platform (AOCP). These vector libraries are used to integrate antibodies into matched eBPC that comprise an eBPC platform (eBPCP), such that each antibody is integrated once into each eBPC in a pool of cells, and is presented on the surface of the eBPC as a BCR. This AES represents a genuinely fully-human ex vivo antibody platform that permits sensitive selection of antibody candidates against label-free epitopes, through leveraging BCR signal transduction to report productive BCR engagement by a target epitope on single eBPC.

### Introduction to the invention

Antibodies secreted by B-cells fulfil a central function in the adaptive immune system of all jawed vertebrates. Binding with high affinity and specificity to a region (epitope) on their target (antigen), they neutralize circulating viruses and toxins and mark invading cellular pathogens for destruction. Within each individual a highly diverse pool of antibodies is generated that has potential reactivity against a virtually unlimited diversity of foreign (non-self) antigens that an individual may encounter, while avoiding harmful responses against self. The combination of high affinity, specificity, effector functions and breadth of potential antigens has made antibodies artificially raised against therapeutic targets a crucial therapeutic tool, addressing unmet clinical needs in autoimmunity and cancer. There exist a multitude of ways to generate antibodies ex vivo, each technique with different strengths and weaknesses. The present invention relates to an AES that enables generation of full-length human antibodies without the need for post-selection modification, and which may utilise BCR signalling readouts for selection of candidate IgH/IgL chain pairs against label-free and non-immobilised epitopes as well as against contextual epitopes and challenging transmembrane proteins.

### Antibodies

Antibodies, also called immunoglobulins (Ig), are proteins that are formed by two copies of a heterodimeric unit. The two proteins comprising the heterodimeric unit are called antibody heavy (IgH) and light chain (IgL), respectively. Both IgH and IgL are composed of a variable (IgHV/IgLV) and constant (IgHC/IgLC) region formed by immunoglobulin superfamily (IgSF) domains. Both variable regions and the IgLC contain one IgSF domain, whereas the IgHC are longer, comprising of three IgSF domains. In combination a specific and highly variable section of both IgHV and IgLV forms the binding interface (paratope) that binds to the epitope. The IgHV and IgLV regions are encoded by three and two gene segments, respectively, which are combinatorically assembled together with the IgHC/IgLC gene segments.

### The antibody BCR complex

From genesis each B-cell is committed to producing a single specific antibody. Production and secretion of the antibody is triggered through epitope-dependent activation of the B-cell by the antigen in the context of B-cell encounter with said antigen. This assures that only antibodies that defend against a current threat are being secreted in large amounts. To achieve this, each B-cell carries a membrane-bound form of its antibody on the surface. In its membrane-bound form the antibody is also called B-cell receptor (BCR) and the IgHCs contain a type 1 transmembrane domain followed by a short cytoplasmic tail. The antibodies are presented on the surface in complex with two transmembrane proteins, CD79a and CD79b (formerly called Ig-alpha and Ig-beta). CD79a/b contain signalling motifs called immunoreceptor tyrosine-based activation motifs (ITAMs), which propagate phosphorylation signals following antibody-antigen binding that are required for B-cell activation.

The epitope engagement of BCR is also central to the maturation of antibody affinity within the B-cell system as a whole. Starting with the naturally derived repertoire of IgH/IgL chain sequences, a single B-cell clone may encounter a target weakly, which induces proliferation. Each daughter cell in each generation of this clonal lineage may present a sequence-diversified version of the original BCR/antibody, imparted through a process call somatic hypermutation. Within a cell lineage, BCR with favourable engagement with cognate epitope are further selected by survival and proliferative cues, while unfavourable engagement is eliminated through lack of survival and proliferative cues. Repeated encounter of epitope across generations of individual 'clonal' lineages thus results in the selection of antibody-producing cells that have stronger engagement with cognate epitopes.

### Antibody diversity

Each B-cell expresses a single recombined antibody and each antibody possess potential for unique ligand specificity, determined by the structure of its paratope. The structural diversity of this region is largely confined to three short hairpin loops on each chain, called complementarity determining regions (CDR), which collectively determine specificity and affinity of the antigen-binding site.

This structural diversity is underpinned by extraordinary sequence diversity across antibodies, which is achieved in three modes. First, the random selection of gene segments for antibody chain recombination provides extensive basal combinatorial sequence diversity. For example, the IgHV regions are formed randomly from 44 unique variable (V), 27 unique diversity (D) and 7 unique joining (J) germline gene segments. Light chain recombination follows the same principle but lacks the D segments and two separate complete manifestations of the antibody light chain exist, called kappa and lambda. Either can pair with any given IgHV, significantly increasing overall diversity. Here and below both kappa and lambda are jointly referred to in abbreviations as "L" for light chain. The second mode to generate sequence diversity occurs within the CDR3 loops. Whereas the both the CDR1 and CDR2 sequences are encoded in the germline V segments, the CDR3 is generated by random deletion of template nucleotides and addition of non-template nucleotides, at the junctions between recombining V,(D) and J gene segments. The two modes ensure the extraordinary diversity in the antibody repertoire that each individual possesses from birth. In the third mode, called somatic-hypermutation, antibodies that bind to a foreign antigen are affinity-matured through iterative antibody-specific mutagenesis and selected through survival signals stemming from the BCR as described above. This mode assures that antibodies with very high affinity even against difficult targets can be generated, and that antibody secretion is largely limited to productive antibodies.

In addition to the described diversity concerning epitope binding, there are several different classes of IgHC regions (IgM, IgD, IgA, IgG, IgE) with a number of subclasses. These do not affect the antigen-binding site but have differing roles in immune defence.

### Therapeutic antibodies

The potential for high affinity, specificity and the wide range of potential targets, combined with their inherent immunological effector functions has made antibodies a highly attractive therapeutic tool. Antibody platforms enable the relatively rapid development of highly specific therapeutics binding with nano- to picomolar affinity to theoretically any extracellular target. Accordingly, therapeutic antibodies have revolutionized the treatment of some autoimmune diseases and are transforming the treatment of several types of tumours. Their development and use has grown during the last decade as key biotechnological challenges have been overcome, and increasingly sophisticated concepts such as bi-specific antibodies and antibody-drug conjugates have been developed. While currently mainly limited to single-chain T-cell receptors, it is likely that future generations of chimeric antigen receptor T-cells (CAR-T) will also rely at least partially on antibody-based receptors. Most but not all therapeutic antibodies are of the IgG class, the most abundant type of antibody in circulation, and of subclass IgG1 due to its beneficial balance of effector functions and physiochemical properties.

### Generating therapeutic antibodies

Antibodies against therapeutic targets are not rationally designed; instead sufficiently large repertoires of diverse antibodies are screened for binding to the target epitope. Candidate sequences that recognize the antigen are then selected for affinity maturation and eventually expressed in a mammalian cell line, most commonly, Chinese hamster ovary (CHO) cells for pre-clinical and clinical testing. The clinical target is most commonly a protein, such as a surface receptor, which may for instance be antagonized through antibody binding. To serve as an antigen in antibody discovery, a recombinant soluble version of this protein generally has to be produced. This recombinant antigen can then be immobilized on plates, labelled for staining or formulated as an immunogen for immunization.

Most therapeutic antibodies currently used in the clinic were generated in mice following immunization. However, murine antibodies are recognized as foreign by the human immune system and require extensive modification to convert them into therapeutics tolerated in humans. In this reformatting, the variable regions are grafted onto human constant regions (chimeric antibodies) or only the murine CDRs are grafted onto a fully human scaffold (humanized antibodies). This reduces the risk of the antibodies being rejected by the patients, however, particularly the generation of the more useful humanised antibodies is complex, and additional modifications are often needed to restore affinity after humanisation. More recent efforts are therefore largely aimed at avoiding non-human sequences as much as possible from the onset. The resulting platforms can broadly characterized as non-immune libraries, recombinant antibody library platforms that do not require immunization, or immune libraries, platforms that either rely on immunized or naturally infected human donors or the immunization of transgenic animals, which express human antibody genes.

### Non-immune recombinant antibody libraries

In this type of system, of which phage-display is the most prominent, a large antibody plasmid library is generated *in vitro* and used in a recombinant selection system that allows very high-throughput manipulation of very large libraries. The antibody sequences for this library can be obtained from human donors, or fully synthetically assembled from germline gene segments and random CDR regions or be a combination of the two. Human-derived libraries have the advantage of being highly enriched for functional antibody pairs, but generally have only limited capacity to produce antibodies against self-proteins as a result of evolved molecular tolerance. This is an important limitation, as many clinical antibody targets are human transmembrane proteins with regulatory functions.

The generated antibody library is integrated into a host/carrier, for example a bacteriophage, so that each individual phage expresses only a single member of the library on its surface. The phage library is produced in bacteria and the brought in contact with the antigen and enriched for particles that bind the antigen. The specific antibody genes expressed by the selected particles are recovered and used for successive enrichment cycles ('biopanning'). Secondary sequence diversification can be integrated into these selection cycles allowing affinity maturation of enriched candidates. Alternatives to phage-display use yeast or insect cells as host and each system have its distinct advantages and drawbacks.

### Immune antibody library platforms

Immune libraries rely on the ability of the immune system to generate high-affinity antibodies against essentially any foreign target. This approach ranges from the isolation of affinity-matured antibodies from immunized or infected human donors to the use of mice that have been genetically engineered to express a fully human set of antibody genes.

It should be noted that recombinant selection platforms such as phage-display and immune-libraries are not mutually exclusive. Antibody sequences isolated from immunized animals or human donors during an immune reaction can be used as starting material for a recombinant selection library in order to identify and affinity mature the sequences obtained from such starting material.

### Synthetic sequence diversity

In principle the more diverse antibody sequences a library contains the more likely it is to contain rare examples recognizing a specific antigen with high affinity. Examples of some libraries can comprise over 1x10^10 different sequences, can randomly pair all human IgHV/IgLV gene segments and fully randomize all six CDR regions. However, equally important is the functional diversity of the library and how it is distributed as not all sequences diversity is equally functionally relevant. For example, not all amino acids are believed to be equally important in forming a high affinity antigen-binding site. Furthermore, the CDR3 of the heavy chain (CDRH3) and to a lesser extent that of the light chain (CDRL3) often play an outsized role in epitope binding. Finally, introducing sites for post-translational modifications may harm functionality and complicated molecular engineering often produces a significant amount of non-functional sequences that increase the non-productive content of a given library.

### Bottlenecks in existing antibody selection systems

Sophisticated antibody libraries of high molecular quality and diversity are now well established. However, several important bottlenecks remain. Phage-display, and several other related approaches, are based on singe-chain antibody fragments. This facilitates high-throughput selection and manipulation of libraries with very high diversities. However, selected candidates must later be re-formatted into full-length antibodies and expressed in human cells as required by most downstream applications. This type of latestage manipulation is inefficient, as not all antibodies respond equally well to such reformatting, thus potentially failing late in the process, or requiring costly and time-consuming adaptation. Systems relying on humanized mice do return full-length IgG of high affinity that are selected in the context of mammalian expression. However, this system generally precludes the use of toxic antigens or antigens that have high homology to their murine othologs. Moreover, it is difficult to generate antibodies in mice or other animals against contextual or unstable epitopes that cannot be delivered as an effective immunogen within the animal physiology.

Efforts to develop fully human antibody library selection systems expressing full-length native antibodies have so far been held back by technical limitations. Most importantly, genome integration of antibody candidate libraries has been inefficient and difficult to control, which posed too severe limitations on library size and quality to be broadly useful. Moreover, the mode of presenting antibody candidates at the cell surface has generally been achieved through inefficient surface-capture methods.

Another major bottleneck is the requirement for a soluble, stable recombinant form of the antigen. Not all proteins are easily converted to this form, which is the case particularly for clinical relevant targets such an ion channels or G-coupled receptors.

### Summary

Human antibodies are powerful therapeutic entities, and major advances in biotechnology over the past decades has lead to the development of various platforms that can generate humanised or fully human antibodies. However, there remains a lack of a truly fully-human ex vivo platform that can deliver fully-human antibodies without the need for further downstream engineering and selection. Moreover, many systems that leverage mouse or other animal physiology to generate antibodies against label-free epitopes, limit the generation of antibodies against contextual, toxic or unstable epitopes. Thus, there is an unmet need for an ex vivo antibody engineering platform that is able to generate fully-human antibodies, ex vivo, in a label-free context.

### Detailed description of the invention

The present invention addresses the above-mentioned needs. In particular, the present invention relates to the provision of an antibody engineering system (AES), comprising two distinct platforms. The Antibody ORF Construction Platform (AOCP) comprises as collection of vectors, primers and subsequent amplicons that permit the construction vector libraries encoding IgH and IgL chain ORF that may be inserted into and expressed by the second platform, comprising an engineered BCR presenting cell platform (eBPCP). The eBPCP comprises engineered human cells that contain genomic integration sites matched to the ACOP integration vectors, permitting the rapid integration of IgH and/or IgL chain ORF to the genome of said cells. This process results in the presentation of a BCR encoded by the integrated IgH and IgL chain ORF, at the cell surface. The AOCP vectors and eBPCP integration sites are designed in such a way that each cell within a library created by IgH/IgL integration from an AOCP library, expresses a single IgH/IgL pair as a BCR. The engagement, or lack of engagement, of this BCR on the surface with a target epitope (tEp) can be used to select a subpopulation of BCR-expressing cells with desirable tEp engagement properties. The AOCP is then used again to recover IgH/IgL sequences from selected eBPCP cells, either as a terminal output defining the obtained IgH/IgL chain sequences with desirable tEp engagement properties, and/or to reform these into integration vectors in differing modes for recycling of the overall AES in antibody engineering workflows. Thus, the invention provides a combined AES that is based on full-length human antibodies that are presented in a native BCR context and may be selected on the basis of tEp engagement with said BCR through measurement of physical interaction, or signal transduction from the BCR, or lack thereof.

The present invention is based on improved reproducibility and efficiency of presenting libraries of IgH/IgL chain sequences in that native BCR context for selection purposes, and being able to recover and recycle such selection for antibody engineering workflows. This is achieved by a combined system whereby a series of synthetic genomic receiver sites (GRS), representing recombinase-mediated cassette exchange (RMCE) constructs, are incorporated into the genomes of engineered BCR-presenting cells (eBPC). These sites permit efficient and reproducible integration of IgH/lg chain sequences, as single copies, provided via matched integration vector libraries (IVL) that harbour matched RCME sequences. Coupled with componentry to recover these IgH/IgL sequences from selected cells, and regenerate IVLs rapidly with sequence-diversification, the present invention represents an in vitro AES based on human cells that outputs full-length human antibodies.

The provision of two parallel subsystems further permits flexibility of the AES in terms of selecting the most efficient processes for different phases of an antibody engineering workflow. The overall AES may transition between a Tandem Site Subsystem (TSS) mode, and a Dual Site Subsystem (DSS) mode. A TSS operates with constructs that pair IgH/IgL sequences in a single unit within eBPC and IVL, whereas a DSS operates with IgH and IgL sequences in paralleled units within eBPC and IVL. The TSS is thus used for high-content screening processes where maximum diversity within a paired IgH/IgL sequence repertoire should be sampled, while a DSS is used for different antibody engineering operations where it is desirable to treat IgH and IgL chain sequences separately. Universal manufacturing modules and output parts shared by the TSS and DSS subsystems enables transition between TSS and DSS modes within the present invention.

### Antibody Engineering System (AES) Architecture and Operation

The combined AES is comprised of paired AOCP and eBPCP, which are be divided into system parts, which themselves operate in two overlapping subsystems. The two overlapping subsystems are described as
a) Tandem Site Subsystem (TSS)
b) Dual Site Subsystem (DSS)
wherein the TSS operates with AOCP and eBPCP genetic constructs that manage IgH and IgL as a single unit, while the DSS operates IgH and IgL genetic constructs as a pair of units.

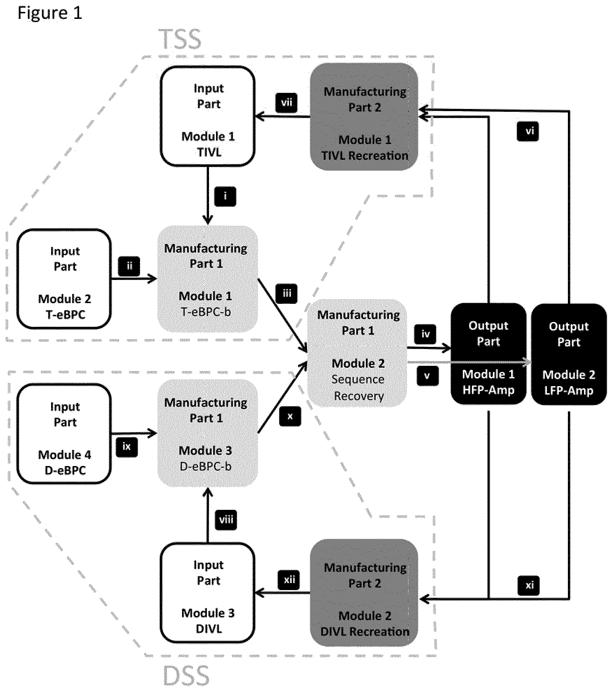

Figure 1 delineates the parts of the AES, defines which parts operate as TSS and DSS, and further which parts and modules are shared and universal. These parts are divided into modules comprising one or more components that operate within a TSS or DSS, or between these subsystems. The parts comprising the overall AES are
a) Input Part (IP)
b) Manufacturing Part 1 (MP1)
c) Output Part (OP)
d) Manufacturing Part 2 (MP2)
wherein;
a) Comprises two AOCP input modules representing integration vector libraries encoding IgH/IgL chain sequences, and two eBPCP input modules representing the cells into which each the tandem and dual library vectors may be integrated;
b) Comprises two modules, each for handling the eBPCP generation and selection in either TSS or DSS modes, and a further universal module for recovery of sequences from the TSS or DSS cells;
c) Represents two separate modules that generate sequence outputs in either high-fidelity or low-fidelity modes. The former can be treated as a terminal output to the system, whereas either may be used to insert to Manufacturing Part 2;
d) Comprises two separate modules for recreation of input integration libraries from the sequences recovered in the Output part, in either DSS or TSS modes, and thus may recreate selected AOCP input modules for the input part.

The componentry of each module will be described in detail in subsequent sections.

### Input Part (IP)

The IP represent the primary input to the AES, and take two distinct forms with regard to the TSS and DSS. The TSS or DSS inputs may be combined to generate new selectable BCR-expressing eBPC libraries (eBPC-b libraries, see MP2) for specific AES workflows. The formation of such cell libraries allows specific constraints to be placed on the antibody IgH/IgL chain sequence repertoire submitted for selection in specific AES workflows. Alternatively, combination of the TSS or DSS inputs can be used to generate eBPC-b libraries that may be outgrown and kept in longer-term storage. Such cell populations would represent a resource comprising a high-diversity IgH/IgL chain sequence repertoire that may be repeatedly drawn upon for different AES workflows.

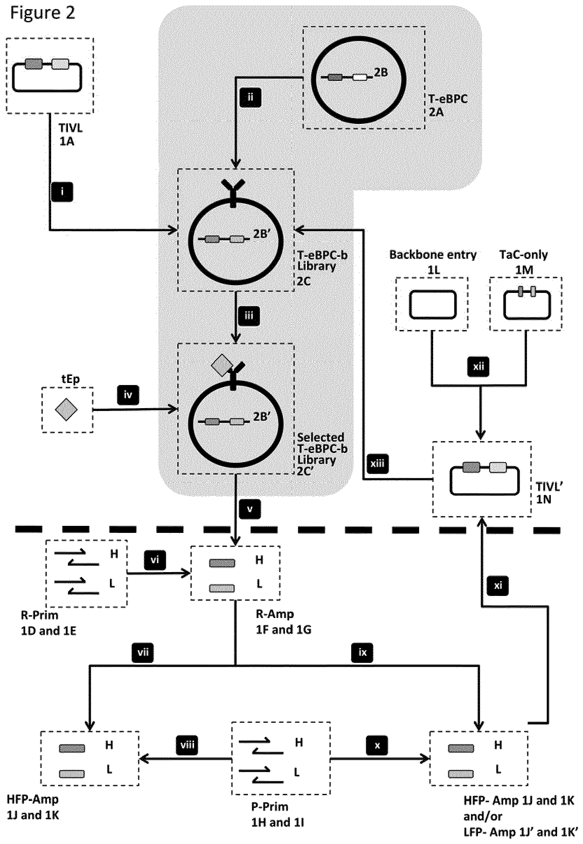

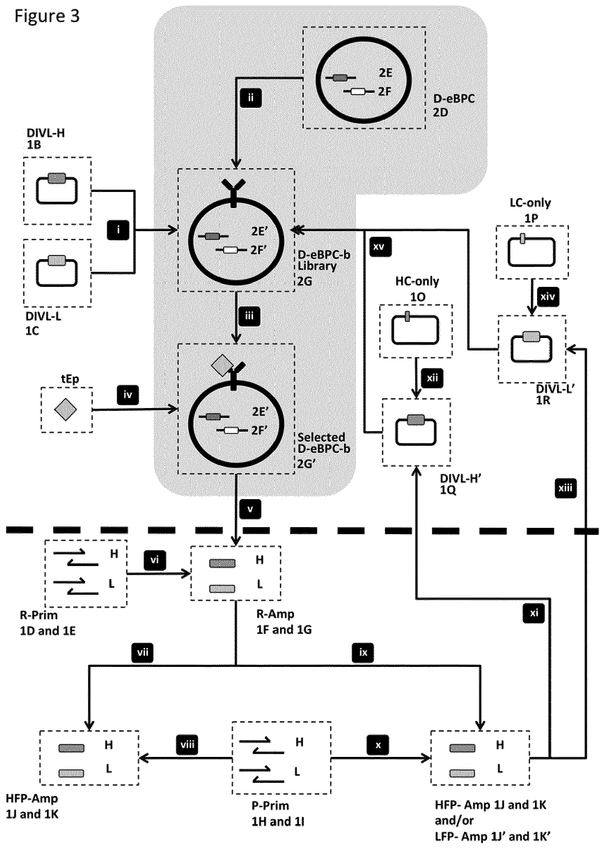

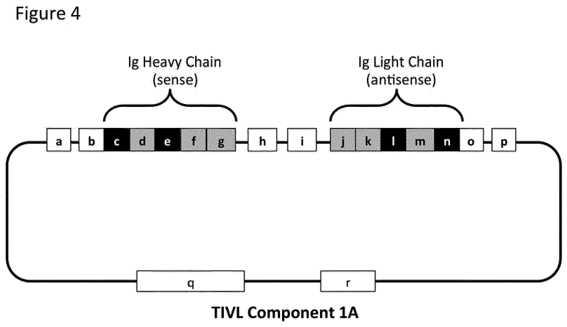

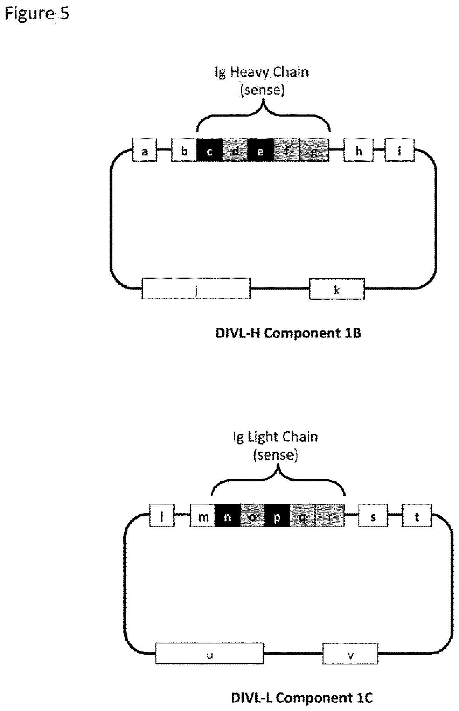

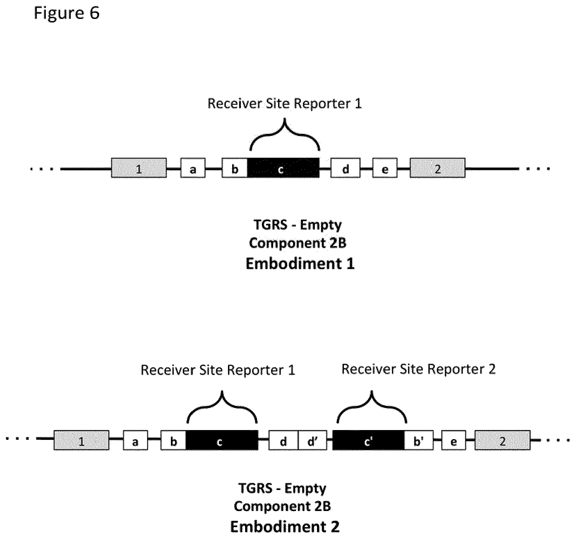

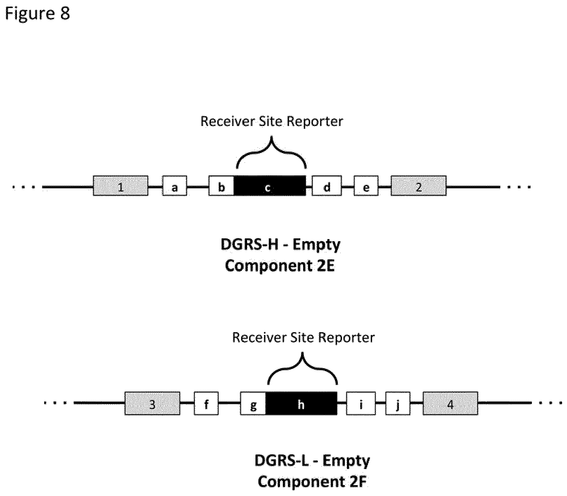

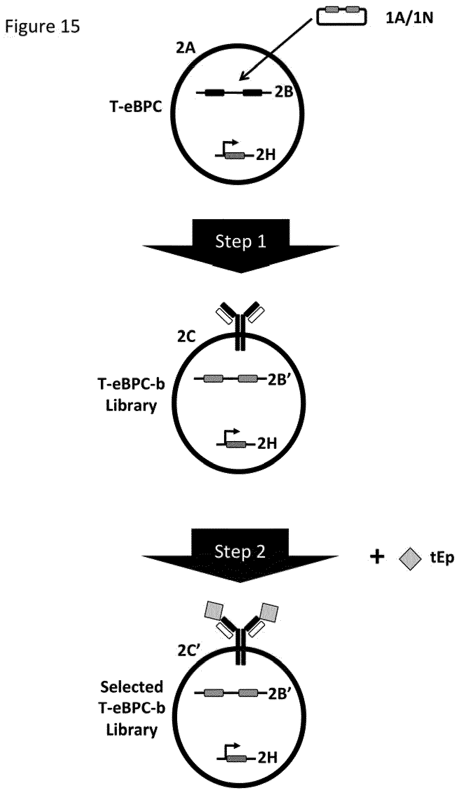

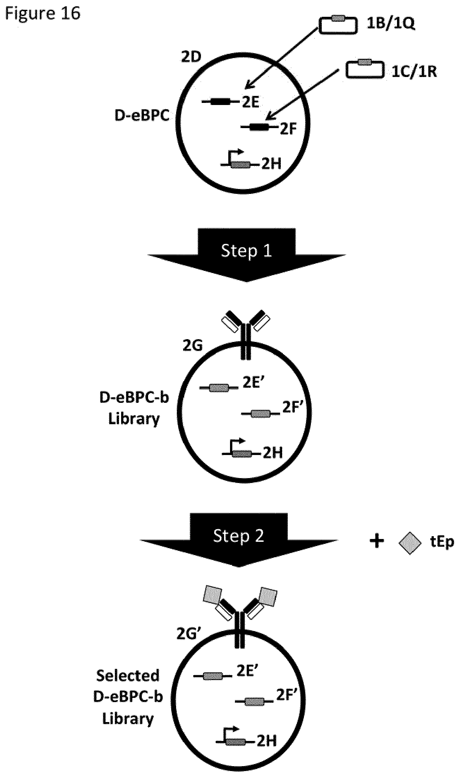

The IP is comprised of
a) IP Module 1
b) IP Module 2
c) IP Module 3
d) IP Module 4
wherein;
a) Represents a module comprising a single component, a tandem integration vector library (**TIVL; component 1A, see** **Figure 2** **and** **4**).
b) Represents a multi-component module comprising a Tandem-engineered BCR-presenting cell (**T-eBPC; component 2A, see** **Figure 2** **and** **Figure 15**). A T-eBPC further contains a Tandem Genomic Receiver Site (**TGRS; Component 2B, see** **Figure 2** **and** **6**), and optionally a synthetic reporter construct to report BCR engagement (**Component 2H; see** **Figure 15**).
c) Represents module comprising two complimentary Dual Integration Vector Library DIVL) components, a Dual Integration Vector Library Heavy (**DIVL-H; component 1B, see** **Figure 3** **and** **5**), and Dual integration vector library Light (**DIVL-L; component 1C, see** **Figure 3** **and** **5**).
d) Represents a multi-component module comprising a Dual-engineered BCR-presenting cell (**D-eBPC; component 2D, see** **Figure 3** **and** **Figure 16**). A D-eBPC further contains a pair of Dual Genomic Receiver Sites (DGRS), a Dual Genomic Receiver Site Heavy (**DGRS-H; Component 2E, see** **Figure 3** **and** **8**), and a Dual Genomic Receiver Site Light (**DGRS-L; Component 2F, see** **Figure 3** **and** **8**). A D-eBPC optionally includes a synthetic reporter construct to report BCR engagement (**Component 2H; see** **Figure 16**).

IP Module 1 and IP Module 2 are paired inputs that can be combined to generate a library of Tandem eBPC presenting a BCR (T-eBPC-b), whereas IP Module 3and IP Module 4 are paired inputs that can be combined to generate a library of Dual eBPC presenting a BCR (D-eBPC-b). Therefore, the TIVL and DIVL inputs define the IgH/IgL sequence diversity that is contained within the T-eBPC-b and D-eBPC-b, respectively. Thus, the TIVL and DIVL inputs are selected to represent the desired antibody sequence diversity and architecture. That is the IgH/IgL chain sequences may be restricted on a number of parameters, including
a) Native IgHV and IgLV gene segment usage
b) Synthetic IgHV and IgLV gene segment usage
c) CDRH3/CDRL3 sequence length
d) CDRH3/CDRL3 sequence diversity
e) CDRH3/CDRL3 amino acid biases
wherein, each of the above criteria will affect the downstream application (a and b), the overall achievable sequence diversity presented by a given TIVL or DIVL (a to e), and the suitability of folding and presentation of antibodies as a BCR (a to e).

### Manufacturing Part 1 (MP1)

The manufacturing part comprises of processing model in which the IVLs and eBPCs of the IP are combined to generate eBPC-b libraries, and the selection and processing of those libraries. The generation of the selectable cell libraries is followed by a selection procedure on the basis of tEp engagement. Selected cells are then partitioned for recovery of IgH/IgL chain sequences presented by the selected cells. This procedure may be conducted in two separate modes as determined by the selection of inputs (see Figure 1).

MP1 Module 1 represents combination and processing of TSS inputs, generating T-eBPC-b libraries. MP1 Module 2 represents DSS inputs, thus generating D-eBPC-b libraries. Once each library is generated within their respective modules, they must be submitted to a selection procedure on the basis of tEp. Such a procedure can include rounds of both negative and positive selection to obtain a subpopulation of one or more eBPC-b. This selected eBPC-b are then submitted to MP1 Module 2, representing a sequence recovery procedure. The components of MP1 Module 2 are designed such that the procedure for recovering IgH and/or IgL chain sequences from selected T-eBPC-b and selected D-eBPC-b is universal, and thus generates universal outputs vide infra.

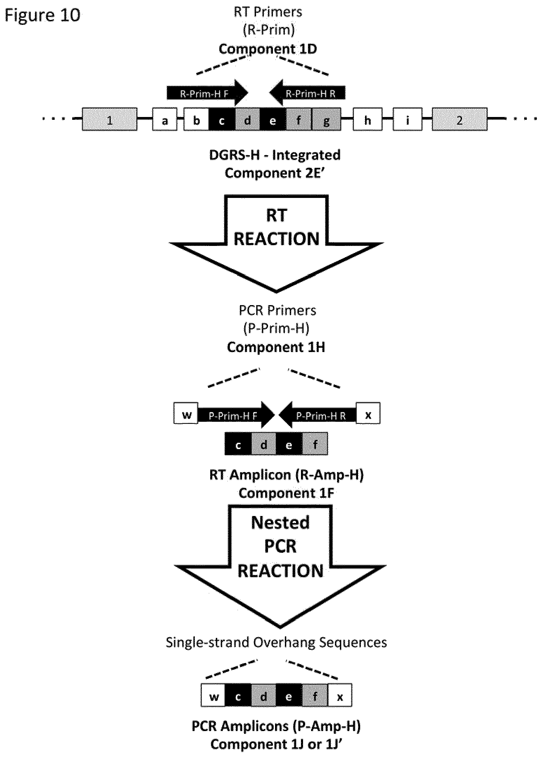

The MP1 is comprised of
a) MP1 Module 1
b) MP1 Module 2
c) MP1 Module 3
wherein;
a) Represents a functional module wherein combining of inputs from IP Module 1 and IP Module 2 results in the creation of a library of T-eBPC expressing BCR at the cell surface **(T-eBPC-b library; component 2C, see** **Figure 2** **and** **15**). In the process, the IgH/IgL chain sequences encoded by the TIVL are integrated to the TGRS, converting TGRS from an open to an integrated state (**TGRS'; Component 2B', see** **Figure 2****,** **6****,** **7** **and** **15**). This process generates library of T-eBPC-b that expresses single discrete pairs of IgH/IgL chains that were encoded by the input TIVL. This functional MP1 Module 1 also includes the selection of a sub-population of the T-eBPC-b library on the basis of engagement of the BCR expressed by each of the T-eBCP-b library members with tEp (**Selected T-eBCP-b, Component 2C', see** **Figure 2**). This selection may be based on positive or negative selection on detection of detection of engagement or lack of engagement, respectively;
b) Represents the multi-component sequence recovery module designed to recover IgH/IgL chain sequences from both the selected T-eBPC-b (see a) above) and selected D-eBPC-b (see b) below). The sequence recovery proceeds through a two-step reverse transcription (RT) and polymerase chain reaction (PCR). The first step requires two pairs of RT Primers (R-Prim), for RT of IgH transcript **(R-Prim-H; Component 1D**) and IgL transcript **(R-Prim-L; Component 1E)**, respectively. The RT process generates IgH amplicons **(R-Amp-H; component 1F)** and IgL amplicons **(R-Amp-L; Component 1G).** These RT amplicons are then submitted to PCR amplification using PCR primer (P-Prim) pairs targeting the R-Amp-H **(P-Prim-H; Component 1H)** and R-Amp-L **(P-Prim-L; Component 1I),** respectively **(See** **Figure 10****).** Overall the PCR amplicon products recovered from the second step can either be considered as terminal outputs from the AES, or intermediates that may be recycled through further rounds of selection (vide infra).
c) Represents a functional module wherein combining of inputs from IP Module 3 and IP Module 4 results in the creation of a library of D-eBPC expressing BCR at the cell surface **(D-eBPC-b library; component 2G, see** **Figure 3** **and** **16****).** In the process, the IgH/IgL chain sequences encoded by the DIVL-H and DIVL-L are integrated to the DGRS-H and DGRS-L, respectively. This converts the DGRS-H to the integrated state **(DGRS-H'; component 2E', see** **Figure 3****,** **8** **and** **9****)** and DGRS-L to the integrated state **(DGRS-L'; component 2F', see** **Figure 3****,** **8** **and** **9****).** This process generates library of D-eBPC-b that expresses single discrete pairs of IgH/IgL chains that were encoded by the input DIVL-H/DIVL-L. This functional MP1 Module 3 also includes the selection of a sub-population of the D-eBPC-b library on the basis of engagement of the BCR expressed by each of the D-eBCP-b library members with a tEp **(selected D-eBPC-b; Component 2G', see** **Figure 3****).** This selection may be based on positive or negative selection on detection of detection of engagement or lack of engagement, respectively.

The operation of MP1 module 2 results in the generation of PCR amplicons (P-Amp) for IgH and/or IgL chain sequences. Critically, MP1 Module 2 contains two different states in generating P-Amp. A high-fidelity (HF) state, employing high-fidelity proofreading polymerase enzymes, is used to recover sequences faithful to those contained within the selected eBPC-b. A low-fidelity (LF) state, employing low-fidelity non-proofreading enzymes (error-prone PCR) is used to recover IgH and/or IgL chain sequences that are imparted with point mutations when compared to those sequences contained with the selected eBPC-b. Alternatively, the LF state can also be achieved using a polymerase under reaction conditions that induce the enzyme to be error-prone or not, such as addition or subtractions of magnesium salts and/ or the use of excessive nucleotide concentrations. These HF and LF PCR amplicons (HFP-Amp and LFP-Amp, respectively) are employed in different output contexts as described in the next section.

### Output Part (OP)

The OP represents two different forms of PCR amplicon (P-Amp) from the MP1 Module 2. These P-Amp products ultimately represent IgH and/or IgL chain sequences that are derived from selected eBPC-b on the basis of tEp engagement properties. Due to the design of the IP and MP1 componentry, the P-Amp products are identical in nature, whether ultimately derived from operation of TSS or DSS IP and MP1 modules.

The OP Is comprised of
a) OP Module 1
b) OP Module 2
wherein; a) Represents HFP-Amp for IgH chain sequences **(HFP-Amp-H; component 1J, see** **Figure 2** **and** **3****)** and IgL chain sequences **(HFP-Amp-L; component 1K, see** **Figure 2** **and** **3****).** This is considered the terminal output from the AES, and also may be used as intermediates for downstream processing within MP2. b) LFP-Amp for IgH chain sequences **(LFP-Amp-H; component 1J', see** **Figure 2** **and** **3****)** and IgL chain sequences **(LFP-Amp-H; component 1K', see** **Figure 2** **and** **3****).** These are used as intermediates for downstream processing in MP2 **(See** **Figure 2****,** **3** **and** **10****).**

The primary use of the HFP-Amp products (OP Module 1) is as the terminal output of the AES, considering that these products represent the IgH/IgL sequences of a selected eBPC-b that has been selected by tEp engagement. In this sense, the selected HF-Amp products can be used to faithfully recapitulate BCRs and antibodies that have desired tEp engagement properties. HFP-Amp products can also be submitted as inputs to TIVL/DIVL Recreations Modules 1 and 2, respectively, of MP2.

The LFP-Amp products are used as intermediates for the regeneration of TIVL and DIVL with introduced sequence diversity. Considering the purpose of the AES is to provide high-quality affinity and/or functionally optimised (engineered) BCRs and antibodies, more than one round of selection will generally be required on the background of introducing sequence diversity.

In both the HFP-Amp and LFP-Amp state, the outputs represent IgH and/or IgL sequences that span the V to J gene segments, and are appended with extensions to the 5' and 3', encoding sites for single stranded DNA overhangs and Type IIS recognition sites, which mediate directed cloning of these products into TIVL and DIVL contexts. In the LFP-Amp, sequence diversity is included in the amplicons via use of low-fidelity or 'error prone' PCR when amplifying the R-Amp products. The architecture and operation of the amplicon generation and TIVL/DIVL recreation are described in detail in subsequent sections.

### Manufacturing Part 2 (MP2)

The MP2 comprises independent TSS and DSS modules with dedicated componentry to enable the recreation of TIVL and DIVL from the P-Amp products of the OP. These TIVL and/or DIVL are used to recycle the AES by use of these recreated libraries as new inputs to MP1. This in effect recreates eBPC-b with IgH/IgL sequences that are faithful to those obtained by selection of eBPC-b in the previous round, or IgH/IgL that have added sequence diversity through LFP-Amp products.

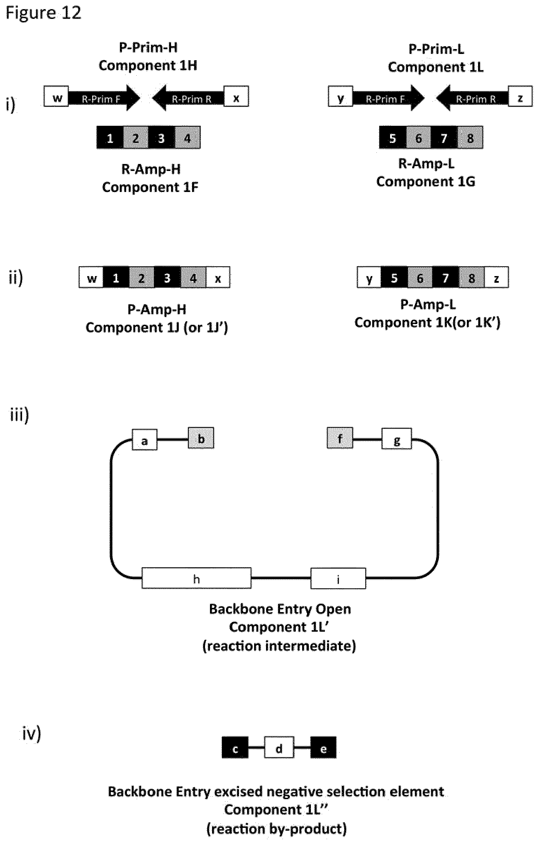

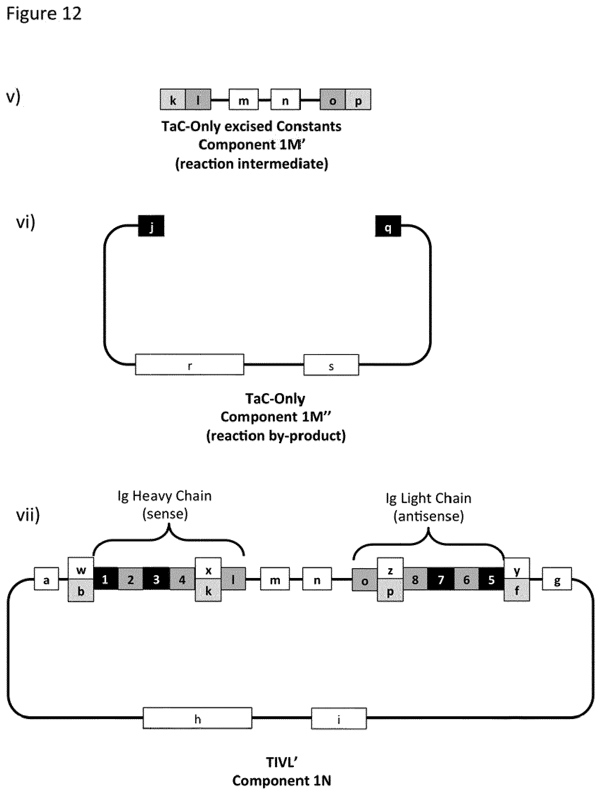

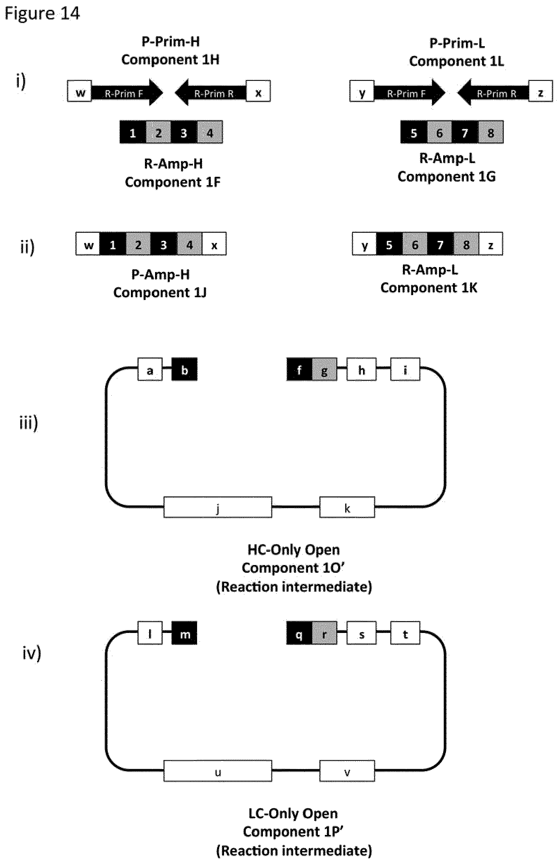

The MP2 is comprised of
a) MP2 Module 1
b) MP2 Module 2
wherein; a) Represents a functional module whereby HFP-Amp and/or LFP-Amp products from the OP are submitted to recreate a TIVL **(TIVL'; Component 1N, see** **Figure 2****, and** **12****).** To create a TIVL', the HFP-Amp and/or LFP-Amp for heavy and light chain products are ligated into an entry backbone vector **(Backbone entry, Component 1L, see** **Figure 2****,** **11** **and** **12****),** along with an insert derived from a second vector encoding IGHC and IGLC and terminator sequences, termed Tandem Constant-Only Vector **(TaC-Only; Component 1M, see** **Figure 2****,** **11** **and** **12****);**
b) Represents a functional module whereby HFP-Amp and/or LFP-Amp products from the OP are submitted to recreate a DIVL-H (**DIVL-H'; Component 1Q, see** **Figure 3****, and** **14**) and a DIVL-L **(DIVL-L'; Component 1R, see** **Figure 3****, and** **14****).** To create a DIVL-H', the HFP-Amp-H and/or LFP-Amp-H product is ligated into a entry backbone vector encoding IGHC, a heavy constant-only vector **(HC-only, Component 1O), see** **Figure 3****,** **13** **and** **14****).** To create a DIVL-L', the HFP-Amp-L and/or LFP-Amp-L product is ligated into an entry backbone vector encoding IGLC, a light constant-only vector **(LC-only, Component 1P), see** **Figure 3****,** **13** **and** **14****).**

It is important to note that the universality of the sequence recovery module (MP1 Module 2), the outputs from the sequence recover that comprise the OP modules are identical in nature whether derived from a T-eBPC-b or D-eBPC-b. This means that TSS-derived sequences, originally delivered by a TIVL, may be recycled through a DSS and DIVL, or vice versa.

### Overview of TSS and DSS Architecture and Function

To fully define the TSS and DSS workflows, and to highlight the distinct and overlapping components of these subsystems, it is useful to consider the overall stepwise workflows within these subsystems. Figures 2 and 3 summarise the processes described in the above sections with regard to the TSS and DSS, respectively. In both figures 2 and 3, the components and processes depicted above the dashed line are exclusive to either TSS or DSS, respectively. All components below the dashed line belong to the universality sequence recovery module that comprises MP1 Module 2. As described above, this universality permits the cycling between TSS and DSS subsystems. The next sections will describe all components of the TSS and DSS in detail.

### TIVL Architecture

A TIVL is a library of one or more vectors that represents the desired IgH and IgL sequence diversity to be inserted to a selected T-eBPC. Thus a TIVL is the primary input to a TSS system; containing the IgH/L sequences to be presented by a T-eBPC-b. A schematic representation of the TIVL plasmid architecture is depicted in Figure 4.

A TIVL comprises **component 1A** of the AES.

A TIVL represents one or more vectors as a library, each vector comprising
a) a 5' genetic element
b) a first Kozak sequence
c) a leader sequence of the IgH chain
d) a IGHV gene segment
e) a CDRH3 sequence
f) a IGHJ gene segment
g) a IGHC gene segment
h) a first transcriptional terminator sequence
i) a second transcriptional terminator sequence
j) a IGLC gene segment
k) a IGLJ gene segment
l) a CDRL3 sequence
m) a IGLV gene segment
n) a leader sequence of the IgL chain
o) a second Kozak sequence
p) a 3' genetic element
q) a positive selection gene
r) an origin of replication.
wherein;
a) Represents the 5' genetic element of the TIVL plasmid, representing the 5' sequence responsible for targeted genomic integration to the **TGRS (component 2B)** contained within a T-eBPC (**component 2A**);
b) Represents a Kozak sequence for efficient translational initiation of the IgH transcript in the T-eBPC-b;
c) Represents the leader sequence of the IgH chain encoded by elements d) to g);
d) Represents an IGHV gene segment encoding a portion of the IgH chain;
e) Represents a CDRH3 sequence encoding a portion of the IgH chain;
f) Represents an IGHJ segment encoding a portion of the IgH chain;
g) Represents an IGHC segment encoding a portion of the IgH chain;
h) Represents a transcriptional terminator sequence for the sense-direction encoded IgH chain, and optional one or more untranslated 3' genetic element(s). These genetic elements may include, but are not limited to, transcript stabilizing or destabilizing elements, and unique identifier sequences
i) Represents the transcriptional terminator sequence for the antisense-direction encoded IgL chain, and optional one or more untranslated 3' genetic element(s). These genetic elements may include, but are not limited to, transcript stabilizing or destabilizing elements, and unique identifier sequences
j) Represents an IGLC segment encoding a portion of the IgL chain;
k) Represents an IGLJ region segment encoding a portion of the IgL chain;
l) Represents an CDRL3 region segment encoding a portion of the IgL chain;
m) Represents an IGLV region segment encoding a portion of the IgL chain;
n) Represents the leader sequence of the IgL chain encoded by elements j) to m);
o) Represents a Kozak sequence for efficient translational initiation of the IgL transcript in the T-eBPC-b
p) Represents the 3' genetic element of the TIVL plasmid, representing the 3' sequence responsible for targeted genomic integration to the TGRS contained within a T-eBPC-b.
q) Represents a positive selection gene for TIVL plasmid selection and propagation.
r) Represents the plasmid origin of replication.

It is important to note that in the presented arrangement, the IgH chain is encoded in the sense (5' to 3') direction, and the IgL chain in the antisense (3' to 5') direction relative to the IgH chain (see Figure 4). This arrangement enables the vector to deliver both IgH and IgL chain sequences to a TGRS without carrying a transcriptional prompter sequence within the TIVL. That is, the TGRS contains promoter sequences encoded in both the sense and antisense directions to drive transcription of the IgH and IgL chain, respectively. These promoter sequences are contained within the 5' and 3' genomic elements of the TGRS (vide infra). This means that the particular TIVL vector arrangement is closely matched to the architecture of the TGRS, which is contained within the T-eBPC. The TIVL recreation components (MP2 Module 1), are designed to efficiently recapitulate the TIVL format with P-Amp products.

The leader sequences described for the IgH and IgL chains within the TIVL are preferably universal consensus sequences. This is to provide universal anchor points for R-Prim **(Components 1D an 1E)** and P-Prim **(Components 1H and 1L)** components. Diversity in leader sequences (appended to native V gene segments, for instance) would require a greater number of primers within the sequence recover components.

The IgH and IgL chain sequence diversity presented by a given TIVL is dependent on a number of parameters. Including the number of V and J gene segments represented in the library for both IgH and IgL chains and the origin of the sequences used for each of the segments. That is to say, that a TIVL library contains a selected number of IGVH, IGHJ, IGLV and IGLJ sequences that are combined to provide a repertoire of antibodies. Preferably, the IgH and IgL gene segment sequences are germline or derived from a native human antibody repertoire, avoiding artificial deviations that could prove immunogenic.

IGHC and IGLC chain sequences should represent a single sequence derived from the native human repertoire, and do not contribute to sequence diversity.

A separate and key determinant of sequence diversity presented by a given TIVL is the nature of the CDRH3 and CDRL3 sequences. Both the length and the degree of sequence diversity within each of the CDR3 sequences included affect the theoretical diversity of a library. The length of the CDRH3 sequence from the consensus C(T/A)(H)R residues in the V regions, to the consensus WG(Q/R/K)G residues on the J regions, is from 30 to 54 nucleotides. The sequence diversity within these variable length sequences is theoretically very high. However, it is preferable to impart positional biases in these sequences for the amino acids encoded.

The CDR3 sequences found in human antibodies are highly diverse but do not randomly represent all twenty amino acids at each position. Rather, each position encodes a subset of amino acids with distinct probabilities and sometimes heavy biases and restrictions. This reflects functional biases towards side-chains that are generally preferable for ligand binding as well as structural constraints present in CDR3 region folding. Knowledge of these probabilities are based on studies of the human antibody CDR3 repertoires and design of the CDR3 sequences in the TIVL can match these probabilities. Furthermore, certain residues such as asparagine and cysteine can be excluded throughout in order to minimize the risk of aggregation and CDR3 glycosylation.

Ultimately, the combination of CDRH3 and CDRL3 sequences with that of the V and J gene segments included in the initial construction of the TIVL, will dictate the IgH and IgL chain sequence diversity presented by the TIVL.

### Dual Integration Vector Library (DIVL) Architecture

The DIVL system comprises libraries analogous to the TIVL described above, with the key difference that the IgH and IgL chains are encoded in separate vectors. This means that the DIVL system comprises two distinct libraries, DIVL-H and DIVL-L, each comprising one or more vectors that contain the desired IgH and IgL sequence diversity to be inserted to a D-eBPC. Thus, DIVL-H and DIVL-L are the primary input to a DSS system; containing the IgH/L sequences to be presented by a D-eBPC-b. A schematic representation of the DIVL plasmid architectures is depicted in Figure 5.

A DIVL-H comprises **component 1B** and a DIVL-L **component 1C** of the AES.

A DIVL-H represents one or more vectors as a library, each vector comprising
a) a 5' genetic element
b) a Kozak sequence
c) a leader sequence for the IgH chain
d) a IGHV gene segment
e) a CDRH3 sequence
f) a IGHJ gene segment
g) a IGHC gene segment
h) optional one or more 3' untranslated genetic elements
i) a 3' genetic element
j) a positive selection gene
k) a origin of replication.
wherein;
a) Represents the 5' genetic element of the DIVL-H plasmid, representing the 5' sequence responsible for targeted genomic integration to the first Dual genomic receiver site heavy chain (**DGRS-H; component 2E**) contained within a D-eBPC (**component 2D);**
b) Represents a Kozak sequence for efficient translational initiation of the IgH transcript in the D-eBPC-b;
c) Represents the leader sequence of the IgH chain encoded by elements d) to g);
d) Represents an IGHV gene segment encoding a portion of the IgH chain;
e) Represents a CDRH3 sequence encoding a portion of the IgH chain;
f) Represents an IGHJ gene segment encoding a portion of the IgH chain;
g) Represents an IGHC gene segment encoding a portion of the IgH chain;
h) Represents an optional one or more untranslated 3' genetic element(s). These genetic elements may include, but are not limited to, transcript stabilizing or destabilizing elements, and unique identifier sequences.
i) Represents the 3' genetic element of the DIVL-H plasmid, representing the 3' sequence responsible for targeted genomic integration to the DGRS-H **(component 2E)** contained within a D-eBPC.
j) Represents a positive selection gene for DIVL-H plasmid selection propagation DIVL-H.
k) Represents the plasmid origin of replication.

A DIVL-L represents one or more vectors as a library, each vector comprising
I) a 5' genetic element
m) a Kozak sequence
n) a leader sequence for the IgL chain
o) a IGLV gene segment
p) a CDRL3 sequence
q) a IGLJ gene segment
r) a IGLC gene segment
s) optional one or more 3' untranslated genetic elements
t) a 3' genetic element
u) a positive selection gene
v) a origin of replication.
wherein;
I) Represents the 5' genetic element of the DIVL-L plasmid, representing the 5' sequence responsible for targeted genomic integration to the second Dual genomic receiver site light-chain (**DGRS-L; component 2F**) contained within a T-eBPC (**component 2D**);
m) Represents a Kozak sequence for efficient translational initiation of the IgL H transcript in the D-eBPC-b;
n) Represents the leader sequence of the IgL chain encoded by elements o) to r);
o) Represents an IGLV gene segment encoding a portion of the IgL chain;
p) Represents a CDRL3 sequence encoding a portion of the IgL chain;
q) Represents an IGLJ gene segment encoding a portion of the IgL chain;
r) Represents an IGLC gene segment encoding a portion of the IgL chain;
s) Represents an optional one or more untranslated 3' genetic element(s). These genetic elements may include, but are not limited to, transcript stabilizing or destabilizing elements, and unique identifier sequences;
t) Represents the 3' genetic element of the DIVL-L plasmid, representing the 3' sequence responsible for targeted genomic integration to the DGRS-L (**component 2E**) contained within a D-eBPC;
u) Represents a positive selection gene for DIVL-H plasmid selection propagation DIVL-H;
v) Represents the plasmid origin of replication.

In this paired library system, the 5' and 3' genetic elements for targeted genomic integration contained within each of the DIVL-H (a, i) and DIVL-L (I, t) must be different between the two vectors, as to provide insulated integration of interposed sequence into the DGRS-H and DGRS-L, respectively, contained within the D-eBPC.

The main utility of dealing with IgH and IgL chain sequence in separate vector contexts in a DIVL, as compared to a single context in the TIVL, is to be able to manipulate the IgH and IgL chain sequences independently. This enables handling AES workflows that aim to systematically diversify either the IgH or IgL chain, while holding the other constant. That is, a D-eBPC may be integrated with a single chain (D-eBPC-x), and repeatedly integrated with DIVLs encoding the complimentary chain (vide infra).

### TIVL and DIVL 5' and 3' genetic elements

In addition, a TIVL/DIVL may also further comprise one or more of the following additional genetic elements
i. Heterospecific recombinase sites
ii. Homologous arms
iii. Eukaryotic promoter
iv. Eukaryotic conditional regulatory element
v. Eukaryotic terminator
vi. Selection marker
vii. Splice acceptor site
viii. Splice donor site
ix. Non-protein coding gene
x. Insulator
xi. Mobile genetic element
xii. Meganuclease recognition site
xiii. Internal ribosome entry site (IRES)
xiv. Viral self-cleaving peptide element
xv. A kozak consensus sequence
xvi. Selection marker of integration
xvii. An antibiotic resistance cassette
xviii. A bacterial origin of replication
xix. A yeast origin of replication
xx. A cloning site

### TGRS Architecture and Function

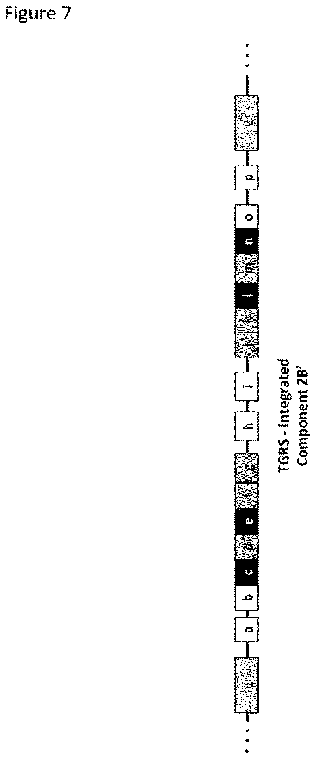

A TGRS is a synthetic sequence cassette inserted into the genome of a T-eBPC, and is designed to be paired with the TIVL such that the IgH and IgL sequences encoded by said TIVL may be integrated in a site-specific manner to the genome of the T-eBPC within the TGRS cassette. This cassette may be considered to occupy two different states. First, the 'open' state is where the cassette encodes a constitutively expressed receiver site reporter gene that indicates the cell has an open IgH/L chain receiver site, with functional promoters **(Component 2B**; see Figure 6). Second, the 'integrated' state is essentially where the reporter gene has been exchanged with IgH and IgL chain sequences **(Component 2B**'; see Figure 7). For clarity, both open and integrated states are described in detail below.

A TGRS (open state) represents a synthetic cassette as a single copy inserted in the genome of a T-eBPC, comprising
a) a 5' genetic element
b) a Kozak sequence
c) a receiver site reporter gene
d) a transcriptional terminator element, and optional one or more 3' untranslated genetic element
e) a second transcriptional terminator element (antisense), and optional one or more 3' untranslated genetic element
f) a second receiver site reporter gene
g) a second a Kozak sequence (antisense)
h) a 3' genetic element
i) a 5' genomic element
j) a 3' genomic element
wherein;
a) Represents the 5' sequence responsible for targeted genomic integration to the TIVL-encoded IgH/L chain pair. Thus sequence element a) of the TGRS is matched with the equivalent 5' genetic element of the TIVL;
b) Represents a Kozak sequence for efficient translational initiation of the reporter gene that constitutively marks the presence of an intact TGRS within a T-eBPC;
c) Represents the reporter gene of the TGRS, loss of which is used to indicate the integration of sequences provide by the TIVL;
d) Represents a transcriptional terminator sequence for the encoded reporter gene, and optional one or more untranslated 3' genetic element(s). These genetic elements may include, but are not limited to, transcript stabilizing or destabilizing elements, and unique identifier sequences;
e) Represents a second transcriptional terminator sequence for the encoded second reporter gene, oriented in the antisense direction, and optional one or more untranslated 3' genetic element(s). These genetic elements may include, but are not limited to, transcript stabilizing or destabilizing elements, and unique identifier sequences;
f) Represents the reporter gene of the TGRS, loss of which is used to indicate the integration of sequences provide by the TIVL, oriented in the antisense direction. Ideally this second reporter would be different the first;
g) Represents a Kozak sequence for efficient translational initiation of the second reporter gene that constitutively marks the presence of an intact TGRS within a T-eBPC;
h) Represents the 3' sequence responsible for targeted genomic integration to the TIVL-encoded IgH/L chain pair. Thus sequence element e) of the TGRS is matched with the equivalent 3' genetic element of the TIVL (Figure 4, element i).
i) Represents the 5' sequence of the cassette that encodes features that that regulate expression and tracking of reporter and IgH/L transcripts. These genomic elements minimally include promoter sequences that drive reporter and IgH chain expression (labelled element 1 in Figure 6);
j) Represents the 3' sequence of the cassette that encodes features that that regulate expression and tracking of reporter and IgH/L transcripts. These genomic elements minimally include promoter sequences that drive reporter and IgL chain expression (labelled element 2 in Figure 6).

Importantly, only a single copy of a TGRS cassette may be contained within the genome of a T-eBPC, such that only one IgH/L paired sequence encoded within the TIVL may be delivered to the genome of each single cell within a T-eBPC population, to create a library of T-eBPC-b, each expressing a single discrete IgH/L pair.

In the preferred embodiment, the TGRS is designed with two different receiver reporter genes encoded between the 5' and 3' genetic elements. Such an arrangement would use a sense and an antisense encoded reporter, resembling the IgH/IgL architecture within a TIVL, and would ensure that both the sense promoter (within the 5' genomic element) and the antisense promoter (within the 3' genomic element) were seen to be functional in a population of T-eBPC.

In an alternative embodiment, only a single reporter gene is encoded between the 5' and 3' genetic elements. This has the advantage of being a smaller, less complex construct and thus only a single parameter is required for selection, instead of two. However, the disadvantage of such an architecture is that it would be difficult to establish if one of the promoters is disrupted or non-functional in population of T-eBPC.

A TGRS (integrated state) represents a synthetic cassette as a single copy inserted in the genome of a T-eBPC, into which has been exchanged a IgH/IgL-encoding construct, comprising
a) a 5' genetic element
b) a first Kozak
c) a leader sequence IgH chain
d) an IGHV gene segment
e) a CDRH3 sequence
f) an IGHJ gene segment
g) an IGHC gene segment
h) a first transcriptional terminator sequence, and optionally one or more 3' untranslated genetic elements
i) a second transcriptional terminator sequence, and optionally one or more 3' untranslated genetic elements
j) an IGLC gene segment
k) an IGLJ gene segment
l) a CDRL3 sequence
m) an IGLV gene segment
n) s leader sequence IgL chain
o) a second Kozak sequence
p) a 3' genetic element
q) a 5' genomic element
r) a 3' genomic element
wherein;
a) Represents the 5' genetic element common between the TIVL and TGRS, representing the 5' sequence responsible for targeted genomic integration of the TIVL sequences to the TGRS;
b) Represents a sense direction encoded Kozak sequence for efficient translational initiation of the IgH transcript in the eBPC;
c) Represents the leader sequence of the IgH chain encoded by elements d) to g);
d) Represents an IGHV gene segment;
e) Represents an CDRH3 sequence;
f) Represents an IGHJ gene segment;
g) Represents an IGHC gene segment;
h) Represents a transcriptional terminator sequence for the sense-direction encoded IgH chain, and optional one or more untranslated 3' genetic element(s). These genetic elements may include, but are not limited to, transcript stabilizing or destabilizing elements, and unique identifier sequences;
i) Represents the transcriptional terminator sequence for the antisense-direction encoded IgL chain, and optional one or more untranslated 3' genetic element(s). These genetic elements may include, but are not limited to, transcript stabilizing or destabilizing elements, and unique identifier sequences;
j) Represents an IGLC gene segment (antisense);
k) Represents an IGLJ gene segment (antisense);
l) Represents a CDRL3 sequence (antisense);
m) Represents an IGLV gene segment (antisense);
n) Represents the leader sequence of the IgL chain (antisense) encoded by elements j) to m);
o) Represents an antisense direction encoded Kozak sequence for efficient translational initiation of the IgL transcript in the eBPC;
p) Represents the 5' genetic element common between the TIVL and TGRS, representing the 5' sequence responsible for targeted genomic integration of the TIVL sequences to the TGRS;
q) represents 5' sequence of the cassette that encodes features that that regulate expression and tracking of reporter and IgH/L transcripts. These genomic elements minimally include promoter sequences that drive reporter and IgH chain expression (labelled element 1 in Figure 6);
r) represents 3' sequence of the cassette that encodes features that that regulate expression and tracking of reporter and IgH/L transcripts. These genomic elements minimally include promoter sequences that drive reporter and IgL chain expression (labelled element 2 in Figure 6).

It is important to note that elements a through p are equivalent to the elements a through p of the TIVL depicted in Figure 4, since this is the sequence delivered by the TIVL in the process of modifying a T-eBPC to a T-eBPC-b (see Figure 2).

It is also important to note, that in this context, the IgH chain is encoded in the sense direction, and the IgL in the antisense direction. There is no practical reason why these orientations could not be reversed in the TIVL/TGRS context.

### Dual Genomic Receiver Site (DGRS) Architecture and function

Each DGRS is a synthetic sequence cassette inserted into the genome of a D-eBPC, and is designed to be paired with the DIVLs such that the IgH and IgL sequences encoded by said DIVLs may be integrated in a site-specific manner to the genome of the D-eBPC within the DGRS cassettes. Thus, a D-eBPC contains two DGRS sites, a DGRS-H **(component 2E**) and DGRS-L **(Component 2F**), which are paired with the DIVL-H and DIVL-L, respectively.

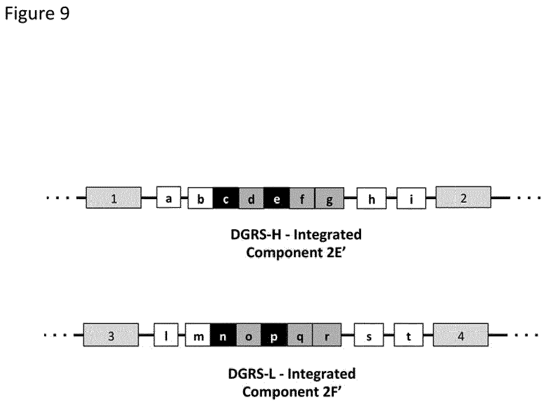

Each of the DGRS resembles closely the architecture of the TGRS cassette described above. However, unlike the TGRS, each DGRS site is designed to encode a single IgH or IgL chain sequence. Therefore, like the TGRS, each DGRS may occupy an 'open' (Figure 8) and 'integrated' (Figure 9) state. To avoid repetition, only the open state is described in detail, whereas the integrated state follows the same logic as the transition from open to integrated TGRS described above.

Importantly, only a single copy of each DGRS cassette may be contained within the genome of a D-eBPC, such that only one IgH/L paired sequence encoded within the DIVLs may be delivered to the genome of each single cell within a D-eBPC population, to create a library of D-eBPC-b, each expressing a single discrete IgH/L pair.

The DGRS-H and DGRS-L together represent a pair of synthetic cassettes, each as a single copy inserted in the genome of a D-eBPC, comprising
a) a 5' genetic element of the DGRS-H
b) a Kozak sequence
c) a first reporter gene
d) optionally one or more 3' untranslated genetic elements
e) a 3' genetic element of the DGRS-H
f) a 5' genetic element of the DGRS-L
g) a Kozak sequence
h) a second reporter gene
i) optionally one or more 3' untranslated genetic elements
j) a 3' genetic element of the DGRS-L
k) a 5' genomic element of the DGRS-H
l) a 3' genomic element of the DGRS-H
m) a 5' genomic element of the DGRS-L
n) a 3' genomic element of the DGRS-L
wherein;
a) Represents the 5' genetic element of the DGRS-H **(Component 2E**), representing the 5' sequence responsible for targeted genomic integration to the DIVL-H-encoded IgH chain. Thus, sequence element a) of the DGRS-H is matched with the equivalent 5' genetic element of the DIVL-H (Figure 5, element a);
b) Represents a Kozak sequence for efficient translational initiation of the reporter gene that constitutively marks the presence of an intact DGRS-H within a D-eBPC, and which is different from the equivalent reporter encoded by the DGRS-L (element h);
c) Represents the reporter gene of the DGRS-H, loss of which is used to indicate the integration of sequences provide by the DIVL-H;
d) Represents optional one or more untranslated 3' genetic element(s). These genetic elements may include, but are not limited to, transcript stabilizing or destabilizing elements, and unique identifier sequences.
e) Represents the 3' genetic element of the DGRS-H, representing the 3' sequence responsible for targeted genomic integration to the DIVL-H-encoded IgH chain. Thus, sequence element e) of the DGRS-H is matched with the equivalent 3' genetic element of the DIVL-H (Figure 5, element i);
f) Represents the 5' genetic element of the DGRS-L **(Component 2F**), representing the 5' sequence responsible for targeted genomic integration to the DIVL-L-encoded IgL chain. Thus, sequence element a) of the DGRS-L is matched with the equivalent 5' genetic element of the DIVL-L (Figure 5, element m);
g) Represents a Kozak sequence for efficient translational initiation of the reporter gene that constitutively marks the presence of an intact DGRS-L within a D-eBPC, and which is different from the equivalent reporter encoded by the DGRS-H (element c);
h) Represents the reporter gene of the DGRS-L, loss of which is used to indicate the integration of sequences provide by the DIVL-L;
i) Represents optional one or more untranslated 3' genetic element(s). These genetic elements may include, but are not limited to, transcript stabilizing or destabilizing elements, and unique identifier sequences;
j) Represents the 3' genetic element of the DGRS-L, representing the 3' sequence responsible for targeted genomic integration to the DIVL-L-encoded IgL chain. Thus, sequence element e) of the DGRS-L is matched with the equivalent 3' genetic element of the DIVL-L (Figure 5, element s);
k) and l) represent 5' genomic elements of the DGRS-H and DGRS-L, respectively. Each of these genomic elements encodes features that that regulate expression and tracking of reporters and IgH or L transcripts. These genomic elements minimally include promoter sequences that drive reporter and IgH chain expression in the case of k), and IgL chain expression in the case of I);
m) and n) represent 3' genomic elements of the DGRS-H and DGRS-L, respectively. Each of these genomic elements, at a minimum encode a transcription terminator, and may also encode features that that regulate expression and tracking of reporter and IgH/L transcripts. These genomic elements may thus include features to be incorporated to a 3' untranslated region, including transcript stabilising or destabilising elements, and unique identifier sequences.

Notably, in most formats of the DGRS, the DGRS-H and DGRS-L, respectively, need not be restricted in and of themselves to integration with IgH and IgL chain sequences, respectively. Indeed, this restriction is imparted by the matching of 5'/3' genetic sequences between DIVL and DGRS, which may be used interchangeably.

In addition, a TGRS/DGRS may also further comprise one or more of the following genetic or genomic elements
i. Heterospecific recombinase sites
ii. Homologous arms
iii. Eukaryotic promoter
iv. Eukaryotic conditional regulatory element
v. Eukaryotic terminator
vi. Selection marker
vii. Splice acceptor site
viii. Splice donor site
ix. Non-protein coding gene
x. Insulator
xi. Mobile genetic element
xii. Meganuclease recognition site
xiii. Internal ribosome entry site (IRES)
xiv. Viral self-cleaving peptide element
xv. A kozak consensus sequence

### Sequence Recovery Module Composition and Function

The sequence recovery module is designed to generate PCR amplicons of IgH and IgL sequences in two modes; Mode 1 are amplicons that are suitable for obtaining sequence information, and Mode 2 are for recreation of DIVLs and/or TIVLs. Operation of Mode 1, obtaining sequence information, all steps of the sequence recovery modules are conducted in a high-fidelity (HF) state, that is in conditions that ensure transcripts are faithfully amplified without error. Such HFP-Amp are considered as the terminal output of the AES, as they represent antibody sequences that have been selected on the basis of tEp engagement in the BCR context. In contrast, Mode 2 operation for recreation of DIVL and/or TIVL, the PCR steps of the sequence recovery module may be conducted in both HF and low-fidelity (LF) states. HF-state would permit regeneration of a DIVL and/or TIVL that faithfully recapitulates the IgH and/or IgL chain sequence(s) recovered into new DIVL' and/or TIVL' states. LF-state is used to impart sequence diversification upon the IgH and/or IgL chain sequence(s) recovered into new TIVL' and/or DIVL' states for subsequent re-use of the T-eBPC and D-eBPC for a further round(s) of tEp selection of the diversified Ig sequences when presented in the T-eBPC-b or D-eBPC-b contexts.

The operation of the sequence recovery module is described in detail in Figure 10 by using the example of recovering an IgH chain sequence from the integrated state of a DGRS-H (component 2E').

The sequence recovery module comprises
a) RT primer pair(s) for IgH and IgL chain sequences
b) RT amplicon product for IgH and IgL chain sequences
c) PCR primer pairs(s) for IgH and IgL chain sequences
wherein;
a) Represents two independent sets of RT primers targeting IgH chain DGRS-H/TGRS inserts **(component 1D)** and IgL chain DGRS-L/TGRS inserts **(component 1E);**
b) Represents the RT amplicon products for IgH chain **(component 1F)** and IgL chain **(component 1G)** sequences recovered with RT primer pairs of a);
c) Represents two independent sets of PCR primers targeting IgH chain RT amplicon product of b) **(component 1H)** and IgL chain RT amplicon product of b) **(component 1I)** to generate AES output module PCR amplicon products (components 1J or 1 J' and 1K and 1K').

The RT primer pairs for each of the IgH and IgL chains may be designed in a number of ways. The preferred design is that the one primer is anchored in the IgH or IgL leader sequence, which is a universal leader sequence for each chain. The second primer is then anchored in the IGHC or IGLC gene segments close to the IGHJ or IGLJ border, using shared sequence among all IgH or IgL chain members in the original TIVL or DIVL. This solution provides an universal anchor points internal to both IgH and IgL transcripts. An anchor in the universal leader sequence also ensures that this leader sequence is not mutated with subsequent diversification (LF PCR), which would otherwise reduce the overall efficiency of the system by imparting an attrition on viable IgH/IgL chain pairs during diversification procedures.

An alternative to the universal anchor in the leader sequence or constant gene segments would be to provide a library of primer pairs that targets sequence features that are more diverse among library members. Instead of the leader sequence, for example, this 5' (Forward) primer could be anchored in the IGHV or IGLV sequences. Such an approach would require provision of a primers against each variable gene segment provide in the original TIVL or DIVL library. Such an approach would reduce the overall efficiency of sequence recovery, but, could be desirable to restrict sequence diversification to sequences more focused to the CDR structures.

Another alternative is to have a universal anchor in the 5' untranslated region (UTR) of the transcripts, however, this would lead to potential diversification of the leader sequence in LF-state operations for diversification. Such an approach would reduce the overall efficiency of viable diversified chains, but, could be desirable to include mutations which improve export to the surface / extracellular matrix.

The PCR primer design closely follows the specification of RT primers. These PCR primers may either be nested inside the RT primers sites, but ideally reflect identical priming sequences and are situated so as to avoid IgLC/IgHC diversification while enabling that of the J region. The key difference between the RT and PCR primers thus becomes the primer extensions that permit application of the P-Amp products to the TIVL or DIVL recreation modules, or extensions for universal sequencing of both IgH and IgL sequences. These extensions (see Figure 10, 12 and 14) are designed in such a way as to encode single stranded DNA overhang sequences at the P-Amp product termini. These sequences are complimentary to single stranded DNA overhang sequences encoded in the TIVL and DIVL recreation module componentry (vide infra), enabling directed ligation of IgH and IgL chain sequence fragments within the TIVL or DIVL recreation process. Importantly, the single stranded DNA overhang sequences in the P-Prim extensions are directly adjacent to Type IIS restriction enzyme binding sites. Thus, Type IIS restriction enzyme action on these P-Amp products during the TIVL or DIVL recreation process generates the single stranded DNA overhang from the encoded sequence (see Figure 12 and 14).

Generally, for direct recovery of sequence information for the terminal output of the AES, the RT should be performed on partitioned cells to pair IgH and IgL chain sequences. That is, a single isolated cell is subjected to reverse transcription reaction with both IgH and IgL targeting primer pairs. The use of Fluorescence-Activated Cell Sorting (FACS) is a common approach for partitioning single cells that paired IgH/IgL sequences can be generated. However, other methods exist to partition cells, including; emulsion PCR; digital PCR approaches using microfluidic cell encapsulation, or digital PCR using physical partitioning substrates.

In a workflow where it is desirable to obtain paired IgH and IgL chains sequences as a terminal output, the PCR reactions would be performed for IgH and IgL chain sequences in parallel independent reactions. There are instances where the IgH or IgL will be a single known sequence, whereas the reciprocal chain pair is presented as a diverse library. In such instances, it may be only necessary to amplify either of IgH or IgL in the two-step process.

An alternative to recovering sequence information directly from single cells, would be to preform the sequence recovery on pools of selected T-eBPC-b or D-eBPC-b. Either the RT or PCR products in such an instance could be submitted to next-generation sequencing analysis to capture the sequences presented in the library. However, when both IgH and IgL chain sequences are presented as diverse libraries, this would not maintain the pairing of selected IgH and IgL chain sequences. However, pooled amplification of IgH and IgL chains sequences may be desirable in some workflows where pools of sequences recovered from selected T-eBPC-b or D-eBPC-b could be directly resubmitted to TIVL or DIVL recreation for re-selection on the basis of randomising IgH and IgL chain pairs within the selected pool, with or without sequence diversification.

The sequence diversification step reflects one of the two different states of performing PCR amplification step from the R-Amp products. The HFP-Amp products are generated by performing PCR with proofreading polymerase enzymes in optimised conditions, assuring faithful sequence amplification and sequence reads from the target IgH and IgL chain sequences for terminal output or for submission to the TIVL or DIVL recreation module. In contrast, the sequence diversification is imparted by use of non-proofreading polymerases in suboptimal PCR conditions. Otherwise known as error-prone PCR, such a process allows random sequence errors to accumulate in the LFP-Amp products. Such an approach is well known to those skilled in the art.

The output modules represent two different states of P-Amp products, comprising
a) HFP-Amp-H
b) HFP-Amp-L
c) LFP-Amp-H
d) LFP-Amp-L
wherein;
a) represents the high-fidelity PCR amplicon product of the IgH chain (**Component 1 J**);
b) represents the high-fidelity PCR amplicon product of the IgL chain (**Component 1 K**);
c) represents the low-fidelity PCR amplicon product of the IgH chain (**Component 1 J**');
d) represents the low-fidelity PCR amplicon product of the IgL chain (**Component 1 K**').

Considering that the TGRS contains identical IgH and IgL chain sequence architecture when compared to the DGRS-H and DGRS-L, respectively, the sequence recovery module can be cycled in the same manner to recover the same output, **Components 1J and/or 1J' and/or 1K and/or 1K'.** This is a key feature of operating the combined TSS/DSS AES, such that sequence recovery module outputs derived from TSS or DSS may be recreated in the reciprocal DIVL or TIVL context.

### TIVL Recreation Module

The TIVL recreation module is used to convert P-Amp components derived from the sequence recovery module into a recreated TIVL containing recovered IgH and IgL chain sequences, termed TIVL'. This TIVL 'recreation' process is used to cycle the AES with recovered and optionally diversified sequences, which have been selected against tEp engagement in antibody engineering workflows.

The TIVL recreation module components comprise
a) a Backbone Entry vector
b) a TaC-Only vector
wherein;
a) Represents a vector that into which the tandem IgH/IgL chain ORFs are assembled, and which provides the vector backbone and genetic elements of the final TIVL' (**Component 1L**);
b) Represents a vector that donates the elements that need to be incorporated between the P-Amp-derived IgH/IgL chain sequences, including both constant gene segment fragments of IGHC and IHLC and the antiparallel terminator sequences (**Component 1M**).

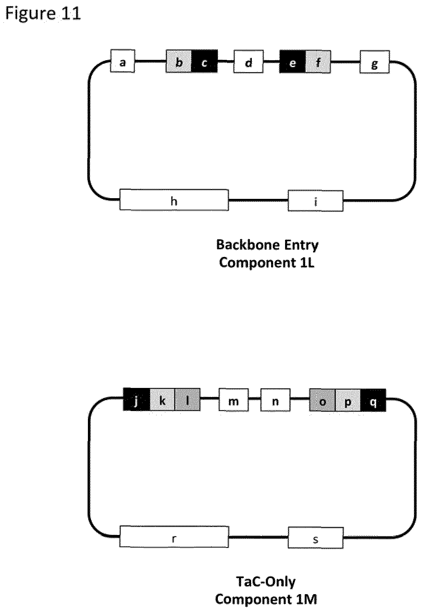

Both the Backbone Entry and TaC-Only vectors are depicted in Figure 11.

A Backbone Entry vector comprises
a) a 5' genetic element
b) a first overhang sequence
c) a first Type IIS recognition sequence,
d) a negative selection marker
e) a second Type IIS recognition sequence
f) a second overhang sequence
g) a 3' genetic element
h) a first positive selection gene
i) a plasmid origin of replication
wherein;
a) Represents the 5' genetic element of the Backbone Entry plasmid, representing the 5' sequence responsible for targeted genomic integration to the TGRS contained within a T-eBPC. This 5' sequence is identical, and fulfils the same role as the 5' genetic element of the TIVL (Figure 4, element a).
b) Represents the first overhang sequence, which encodes a unique sequence for a single-strand DNA overhang created by the action of the Type IIS enzyme binding to its recognition sequence immediately to the 3' of the overhang sequence.
c) Represents a first Type IIS recognition sequence, relating to the binding of and action of the enzyme creating the overhang encoded in element b).
d) Represents a negative selection marker used to negatively select against parental Entry Backbone vector that is undigested by Type IIS enzyme action.
e) Represents a second Type IIS recognition sequence, relating to the binding of and action of the enzyme creating the overhang encoded in element f).
f) Represents the second overhang sequence, which encodes a unique sequence for a single-strand DNA overhang created by the action of the Type IIS enzyme binding to its recognition sequence immediately to the 5' of the overhang sequence.
g) Represents the 3' genetic element of the Backbone Entry plasmid, representing the 3' sequence responsible for targeted genomic integration to the TGRS contained within a T-eBPC. This 3' sequence is identical, and fulfils the same role as the 3' genetic element of the TIVL (Figure 4, element p).
h) Represents a first positive selection gene for both Backbone Entry vector and TIVL' plasmid selection and propagation, and which is different from the second positive selection gene contained within the TaC-only vector.
i) Represents the plasmid origin of replication.

The Backbone Entry vector works in concert with the TaC-Only vector to provide all necessary sequences to the final TIVL' library, which are not carried by the P-Amp products.

A TaC-Only vector comprises
j) a third Type IIS recognition sequence
k) a third overhang sequence
l) a IGHC gene segment fragment
m) a first transcriptional terminator sequence, and optional one or more untranslated 3' genetic element(s).
n) a second transcriptional terminator sequence, and optional one or more untranslated 3' genetic element(s).
o) a IGLC gene segment fragment
p) a fourth overhang sequence
q) a fourth Type IIS recognition sequence
r) a second positive selection gene
s) a plasmid origin of replication.
wherein;
j) Represents a third Type IIS recognition sequence, relating to the binding of and action of the enzyme creating the overhang encoded in element k).
k) Represents the third overhang sequence, which encodes a unique sequence for a single-strand DNA overhang created by the action of the Type IIS enzyme binding to its recognition sequence immediately to the 5' of the overhang sequence.
l) Represents a IgHC gene segment fragment that is not encoded by the pAMP-H product.
m) Represents a first transcriptional terminator sequence encoded in the sense direction, and optional one or more untranslated 3' genetic element(s). These genetic elements may include, but are not limited to, transcript stabilizing or destabilizing elements, and unique identifier sequences.
n) Represents a second terminator sequence encoded in the antisense direction, and optional one or more untranslated 3' genetic element(s). These genetic elements may include, but are not limited to, transcript stabilizing or destabilizing elements, and unique identifier sequences.
o) Represents a IgLC gene segment fragment that is not encoded by the pAMP-H product, and in the antisense direction.
p) Represents the fourth overhang sequence, which encodes a unique sequence for a single-strand DNA overhang created by the action of the Type IIS enzyme binding to its recognition sequence immediately to the 3' of the overhang sequence.
q) Represents a fourth Type IIS recognition sequence, relating to the binding of and action of the enzyme creating the overhang encoded in element p).
r) Represents a second positive selection gene for TaC-Only plasmid selection and propagation, and which is different from the first positive selection gene contained within the Backbone Entry vector.
s) Represents the plasmid origin of replication.

The TaC-Only vector is depicted in the lower panel of Figure 11, and as for the TIVL depicted in Figure 4, the IgH-related elements are encoded in the sense direction, whereas the IgL-related elements are encoded in the antisense direction. There is no practical reason why this orientation could not be reversed, such the IgL is sense and IgH is antisense, as long as the TIVL architecture is maintained.

The process of TIVL' generation within the TIVL recreation module is depicted in Figure 12, beginning R-Amp-H (**component 1F**) and R-Amp-L (**component 1G**) that may be obtained from either a TGRS or DGRS by amplification with RT primers (**Components 1D and 1E**).

The P-Prim-H **(Component 1H)** and IgL chain P-prim-L **(component 1I),** each contain primer extension sequences that are described above. These primer extensions encode sites for generation of single stranded DNA overhang sequences, and Type IIS restriction enzyme binding sequences, both compatible with the directional ligation of the resulting P-Amp products into the final TIVL' context (vide infra).

PCR amplification of the R-Amp products using the above-defined P-Prim components results in P-Amp-H **(component 1J or 1J')** and P-Amp-L **(Component 1K or IK')** reaction products, in either HF- or LF-states as previously described, and appended with the above descried extensions.

The Backbone Entry vector, TaC-only vector and P-Amp products are combined into a one-pot restriction enzyme / ligase cycle reaction for final directional ligation step in TIVL' library creation.

The products of Backbone Entry vector **(Component 1L)** when digested with the relevant Type IIS enzyme(s) are represented by the Backbone Entry Open conformation reaction intermediate **(Component 1L')** and the excised negative selection element by-product **(component 1L").**

The products of the TaC-Only vector **(Component 1M)** when digested with the relevant Type IIS enzyme(s) are represented by the TaC-only open conformation reaction by-product (Component 1M"), and excised terminator and IGHC/IGLC elements excised as the reaction intermediate (Component 1M'). Directed ligation via single stranded DNA overhangs of these components generates the TIVL' **(Component 1N).** This incorporates the IgH chain (sense) and IgL chain (antisense) contributed by the P-Amp products, having been obtained from integrated TGRS or DGRS. These IgH and IgL chains are completed with IGHC/IGLC sequences provided by the reaction intermediate excised from the TaC-only vector, along with terminator sequences. These sequences are annealed into the Backbone Entry Open product to complete a TIVL'.

All Type IIS restriction enzyme sequences should ideally encode recognition and cutting by the same Type IIS enzyme. However, theoretically up to eight different enzymes could be used to independently cut each unique overhang site. The benefit of using a Type IIS enzyme is that considering these enzymes cut outside their recognition sequence, multiple unique single stranded overhang sequences can be generated by use of a single enzyme.

The positive selection genes contained within the Backbone Entry and TaC-only vectors should be different. The positive selection encoded in the Backbone Entry may thus be used to select the TIVL' product, while avoiding background from the TaC-only vector.

### DIVL Recreation Module

The DIVL recreation module is used to convert P-Amp components derived from the sequence recovery module into a recreated DIVL-H or DIVL-L containing recovered IgH or IgL chain sequences, respectively, termed DIVL-H' and DIVL-L'. This DIVL 'recreation' process is used to cycle the AES with recovered and optionally diversified sequences, which have been selected against tEp engagement in antibody engineering workflows.

The principle of DIVL recreation process is analogous to that of the TIVL recreation described above. The key difference of a DIVL system compared to a DIVL system, is that each IgH and IgL chain are carried on separate plasmids in a DIVL system, thus the IgH and IgL chain-related sequence elements are separated into two separate vectors.

The DIVL recreation module components comprise
a) a HC-only vector
b) a LC-Only vector
wherein;
a) Represents a vector that into which the IgH chain ORFs are assembled, and which provides the vector backbone and genetic elements of the final DIVL-H', along with constant gene segment fragments of IGHC (**Component 1O**);
b) Represents a vector that into which the IgL chain ORFs are assembled, and which provides the vector backbone and genetic elements of the final DIVL-L', along with constant gene segment fragments of IGLC **(Component 1P);**

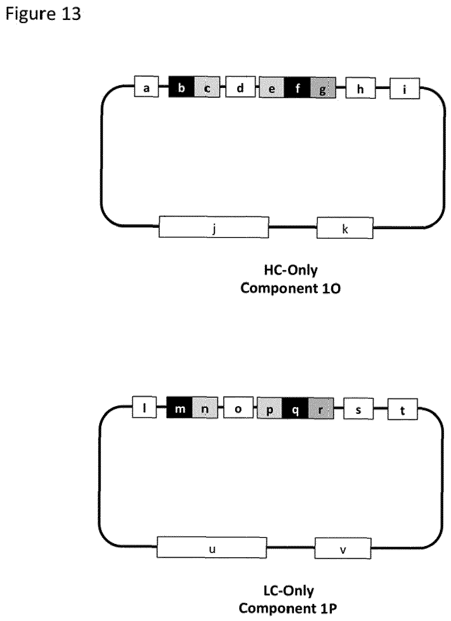

Both HC-Only and LC-Only vectors are depicted in Figure 13.

A HC-only vector comprises
a) a 5' genetic element
b) a first overhang sequence,
c) a first Type IIS recognition sequence
d) a negative selection marker
e) a second Type IIS recognition sequence
f) a second overhang sequence
g) a IGHC gene segment fragment
h) optional one or more 3' untranslated genetic elements
i) a 3' genetic element
j) a positive selection gene
k) a origin of replication
wherein;
a) Represents the 5' genetic element of the Backbone Entry plasmid, representing the 5' sequence responsible for targeted genomic integration to the DGRS-H contained within a D-eBPC. This 5' sequence is identical, and fulfils the same role as the 5' genetic element of the DIVL-H (Figure 5, element a).
b) Represents the first overhang sequence, which encodes a unique sequence for a single-strand DNA overhang created by the action of the Type IIS enzyme binding to its recognition sequence immediately to the 3' of the overhang sequence.
c) Represents a first Type IIS recognition sequence, relating to the binding of and action of the enzyme creating the overhang encoded in element b).
d) Represents a negative selection marker used to negatively select the for parental Entry Backbone vector that is undigested by Type IIS enzyme action.
e) Represents a second Type IIS recognition sequence, relating to the binding of and action of the enzyme creating the overhang encoded in element f).
f) Represents the second overhang sequence, which encodes a unique sequence for a single-strand DNA overhang created by the action of the Type IIS enzyme binding to its recognition sequence immediately to the 5' of the overhang sequence.
g) Represents a IGHC gene segment fragment that is not encoded by the P-AMP-H product.
h) Represents optional one or more untranslated 3' genetic element(s). These genetic elements may include, but are not limited to, transcript stabilizing or destabilizing elements, and unique identifier sequences.
i) Represents the 3' genetic element of the Backbone Entry plasmid, representing the 3' sequence responsible for targeted genomic integration to the DGRS-H contained within a D-eBPC. This 3' sequence is identical, and fulfils the same role as the 3' genetic element of the DIVL-H (Figure 5, element i).
j) Represents a positive selection gene for HC-Only and DIVL-H' plasmid selection and propagation.
k) Represents the plasmid origin of replication.

The HC-Only vector is generally used in a parallel independent reaction with the LC-Only vector. Both are described here together for ease of comparison.

A LC-only vector comprises
l) a 5' genetic element
m) a first overhang sequence,
n) a first Type IIS recognition sequence
o) a negative selection marker
p) a second Type IIS recognition sequence
q) a second overhang sequence
r) a IGHC gene segment fragment
s) optional one or more 3' untranslated genetic element
t) a 3' genetic element
u) a positive selection gene
v) a origin of replication
wherein;
l) Represents the 5' genetic element of the Backbone Entry plasmid, representing the 5' sequence responsible for targeted genomic integration to the DGRS-L contained within a D-eBPC. This 5' sequence is identical, and fulfils the same role as the 5' genetic element of the DIVL-L (Figure 5, element I).
m) Represents the first overhang sequence, which encodes a unique sequence for a single-strand DNA overhang created by the action of the Type IIS enzyme binding to its recognition sequence immediately to the 3' of the overhang sequence.
n) Represents a first Type IIS recognition sequence, relating to the binding of and action of the enzyme creating the overhang encoded in element m).
o) Represents a negative selection marker used to negatively select the for parental Entry Backbone vector that is undigested by Type IIS enzyme action.
p) Represents a second Type IIS recognition sequence, relating to the binding of and action of the enzyme creating the overhang encoded in element q).
q) Represents the second overhang sequence, which encodes a unique sequence for a single-strand DNA overhang created by the action of the Type IIS enzyme binding to its recognition sequence immediately to the 5' of the overhang sequence.
r) Represents a IGLC gene segment fragment that is not encoded by the P-AMP-L product.
s) Represents optional one or more untranslated 3' genetic element(s). These genetic elements may include, but are not limited to, transcript stabilizing or destabilizing elements, and unique identifier sequences.
t) Represents the 3' genetic element of the Backbone Entry plasmid, representing the 3' sequence responsible for targeted genomic integration to the DGRS-L contained within a D-eBPC. This 3' sequence is identical, and fulfils the same role as the 3' genetic element of the DIVL-L (Figure 5, element t).
u) Represents a positive selection gene for LC-Only and DIVL-L' plasmid selection and propagation.
v) Represents the plasmid origin of replication.

Generally, the HC-Only and LC-Only vectors would be treated in parallel to generate independent DIVL-H' and DIVL-L', respectively. This is to ease the operation, and preserve the advantage of utilising a DSS; that is, to handle IgH and IgL chain sequences on a selective and independent basis. Thus, there is no general requirement to harmonise the Type IIS recognition sequences or positive selection genes.

The process of DIVL-H' and DIVL-L' generation within the DIVL recreation module is depicted in Figure 14, beginning R-Amp-H **(component 1F)** and R-Amp-L **(component 1G)** that may be obtained from either a TGRS or DGRS by amplification with RT primers **(Components 1D and 1E).**

P-Prim-H **(Component 1H)** and P-prim-L **(component 1I),** each contain primer extension sequences that are described above. These primer extensions encode sites for generation of single stranded DNA overhang sequences, and Type IIS restriction enzyme binding sequences, both compatible with the directional ligation of the resulting P-Amp products into the final DIVL' context.

PCR amplification of the R-Amp products using the above-defined P-Prim components results in P-Amp-H **(component 1J or 1J')** and P-Amp-L **(Component 1K or IK')** reaction products, in either HF or LF modes as previously described, and appended with the above described extensions.

The P-Amp-H products are combined into a one-pot restriction enzyme / ligase cycle reaction with the HC-Only vector for final directional ligation step in DIVL-H' creation. Similarly, the P-Amp-L products are combined into a one-pot restriction enzyme / ligase cycle reaction with the LC-Only vector for final directional ligation step in DIVL-L' creation.

The products of HC-Only **(Component 1O),** when digested with the relevant Type IIS enzyme(s), are represented by the HC-Only Open conformation reaction intermediate **(Component 1O'),** and the excised negative selection and Type IIS recognition sequences reaction by-product **(Component 1O").**

Similarly, the products of LC-Only **(Component 1P),** when digested with the relevant Type IIS enzyme(s), are represented by the LC-Only Open conformation reaction intermediate **(Component 1P')**, and the excised negative selection and Type IIS recognition sequences reaction by-product **(Component 1P")**.

The final reaction product to create a DIVL-H' **(Component 1Q)**, representing a ligation between the P-Amp-H and the HC-Only open conformation via the single-stranded DNA overhangs. This incorporates the IgH chain contributed by the P-Amp-H products, having been obtained from a TGRS or DGRS. These IgH chain sequences are completed with IGHC sequence provided within the HC-Only Vector.

The final reaction product to create a DIVL-L' **(Component 1R)**, representing a ligation between the P-Amp-L and the LC-Only open conformation via the single-stranded DNA overhangs. This incorporates the IgL chain contributed by the P-Amp-L products, having been obtained from a TGRS or DGRS. These IgL chain sequences are completed with IGLC sequence provided within the LC-Only Vector.

### Tandem engineered BCR-presenting cells (T-eBPC)

A T-eBPC is a human cell line that is selected and/or engineered to possess a number of key features and components, and which itself comprises a central component of the TSS. The function of a T-eBPC within the TSS, is as a receptacle for the TIVL/TIVL' encoded IgH/IgL chains, and presenting a single discrete IgH/IgL pair encoded within the TIVL/TIVL' as a BCR on the cell surface. Such a process generates cell libraries presenting TIVL/TIVL' encoded IgH/IgL chain pairs on the cell surface, termed T-eBPC-b, that may be contacted with tEp for purposes of performing a selection of one or more cells for recovery of IgH/IgL chain pairs that have a desirable engagement properties with said tEp.

The components and different states of a T-eBPC are depicted in Figure 15.

The T-eBPC, **component 2A,**
i. Lacks endogenous expression of IgH and IgL chains and
ii. Expresses CD79a and CD79b and
iii. Contains further **component 2B,** as a synthetic genomic receiver site for integration of a single tandem construct encoding IgH and IgL chain ORFs
wherein
i) May be obtained by selection of a naturally occurring cell population lacking said expression, or may be engineered to lack such expression;
ii) May be obtained by selection of a naturally occurring cell population comprising said expression, or may be engineered to comprise such expression;
iii) May be introduced by means of genetic engineering.

Engineering of such a cell line is achievable with techniques known to those skilled in the art. The selection of cell lines lacking expression of IgH and IgL chains but expressing CD79a and CD79b is straightforward, and indeed most cell lines lack such expression. The addition of a genomic receiver site may be achieved through a number of genome engineering techniques, but preferably is site-directed.

A genomic receiver site of a T-eBPC, **component 2B,** is termed a TGRS. The architecture and function of which is described above (see Figure 6 and 7).

A T-eBPC is designed to assay engagement of the presented BCR with a tEp. The readout of engagement may be achieved through detectable labelling of T-eBPC-b cells a given tEp, or through induction of a synthetic reporter element that responds to BCR engagement. Therefore, the T-eBPC **component 2A,** may further contain a component **designated 2H,** a synthetic genomic BCR-stimulation response element selected from
i. A single component synthetic construct containing at least one native promoter and/or at least one synthetic promoter and at least one reporter
ii. A multi-component synthetic construct designed with at least one native promoter and/or at least one synthetic promoter and at least one reporter
wherein activation of i and/or ii is dependent on at least one signal transduction pathway selected from a synthetic pathway, a native pathway or a combination thereof.

Synthetic BCR-stimulation response elements **(component 2H)** with synthetic as opposed to native promoters are less likely to mimic a natural BCR stimulation response and thus represent a more distant approximation to natural B-cell stimulation. However, synthetic promoters provide greater flexibility for tuning and adjustment of the reporter response for the robust identification of eBPC-b presenting BCR that productively engage tEp.

Single component synthetic constructs provide limited space for amplification or modulation of the signal reporter response, but are more straightforward to implement and predict the outcome. Multi-component constructs have the advantage of having a virtually unlimited space to construct complex response networks, however, this comes with the cost of being more difficult to implement and predict. A network also provides instances to initiate feedback loops, repression and activation of secondary response reporters.

Synthetic promoters can be linked to artificial synthetic signalling components, which can be either downstream of the initial natural BCR signalling pathway or substitute it. In such an instance, the CD79a/b complex can be coupled in-part or wholly to an artificial synthetic signalling pathway and response network. Such artificial pathways provide the advantage of being very modular and adaptable, for greater fine tuning or increased variety of responses.

The preferred embodiment utilises a multi-component synthetic construct with synthetic promoters and at least a partial synthetic signalling pathway. This provides the most robust means to ensure limited resting state signal response but with high coupled reporter signal when ligation of tEp to the BCR occurs.

Regardless of the exact architecture of coupling the BCR stimulation to a component 2H the desired end outcome is a detectable reporter. In the preferred embodiment, a reporter that is amenable to fluorescent detection (i.e. FACS) and/or physical selection methods such as Magnetic Activated Cell Soting (MACS) is desired. Alternatively, the reporter could be a secreted or intracellular molecule for detection by spectrometric, fluorescent or luminescent assays, preferably in a non-destructive nature, wherein the populations can subsequently be selected based on the presence or absence of the reporter molecule. In addition, the response is not limited to a single reporter type but could be a combination of one or more different reporters.

In one context, a multi-component synthetic construct with synthetic promoters and a partial synthetic signalling pathway (Driver-Activator/Repressor + Amplifier-Reporter) would comprise of:
Driver-Activator/Repressor:
   a) a synthetic promoter
   b) a Kozak sequence
   c) a transcription factor,
   d) a first terminator
Amplifier-Reporter
   e) a second synthetic promoter
   f) a Kozak sequence
   g) a reporter
   h) a second terminator
wherein;
   a) Represents a synthetic promoter (Driver) that is designed to be coupled to the initial natural signalling pathway generated by BCR ligation to the tEp. A minimum driver comprises of one or more transcription factor binding site and a core promoter, wherein the core promoter comprises, at a minimum, of a TATA Box, Initiator element (Inr) and transcriptional start site.
   b) Represents a Kozak sequence for efficient translational initiation of the transcription factor
   c) Represents an ORF encoding a synthetic or natural transcription factor (Activator, or Repressor), which can bind to the DNA sequence of the second synthetic promoter
   d) Represents a transcriptional terminator for efficient transcription and polyadenylation of the transcription factor mRNA transcript, and optional one or more untranslated 3' genetic element(s). These genetic elements may include, but are not limited to, transcript stabilizing or destabilizing elements, and unique identifier sequences
   e) Represents a second synthetic promoter (Amplifier), which encodes one or more recognition sites for binding of the c) transcription factor and a core promoter, wherein the core promoter comprises, at a minimum, of a TATA Box, Inr and transcriptional start site.
   f) Represents a Kozak sequence for efficient translational initiation of the reporter
   g) Represents an ORF encoding a reporter protein
   h) Represents a transcriptional terminator for efficient transcription and polyadenylation of the reporter mRNA transcript, and optional one or more untranslated 3' genetic element(s). These genetic elements may include, but are not limited to, transcript stabilizing or destabilizing elements, and unique identifier sequences

It is important to recognise that the Driver-Activator/Repressor coupled to Amplifier-Reporter paradigm can be extended to include additional Driver-Activator/Repressor and Amplifier-Reporter pairs and/or additional Amplifier-Reporter units. Furthermore, Activators can be replaced with Repressors to shutdown Amplifier-Reporter units and/or Driver-Activator/Repressor units, for negative selection methods and/or negative-feedback loops.

In a second context, a single-component synthetic construct with synthetic promoters (Driver-Reporter) would comprise of:
a) a synthetic promoter
b) a Kozak sequence
c) a reporter
d) a terminator
wherein;
a) Represents a synthetic promoter (Driver) that is designed to be coupled to the initial natural signalling pathway generated by BCR ligation to the tEp. A minimum driver comprises of one or more transcription factor binding site and a core promoter, wherein the core promoter comprises, at a minimum, of a TATA Box, Inr and transcriptional start site.
b) Represents a Kozak sequence for efficient translational initiation of the reporter
c) Represents an ORF encoding a reporter protein
d) Represents a transcriptional terminator for efficient transcription and polyadenylation of the reporter mRNA transcript, and optional one or more untranslated 3' genetic element(s). These genetic elements may include, but are not limited to, transcript stabilizing or destabilizing elements, and unique identifier sequences

Without the use of a synthetic reporter the preferred way of generating a selected T-eBPC-b library **(component 2C')** from a T-eBPC-b library would be engagement with a tEp that has be labelled with a fluorophore and selection of T-eBPC-b that bind the tEp through FACS. The use of a synthetic reporter additionally allows eBPC-b selection from unlabelled tEp, from tEp that are immobilized or from tEp expressed contextually in complex with other proteins and/or on the surface of a eTp-presenting cell.

### Dual engineered BCR-presenting cells (D-eBPC)

A D-eBPC (component 2D) is analogous to the T-eBPC described above in all aspects, except for the nature of genomic receiver sites. That is, rather than a single genomic receiver designed to receive a tandem IgH/IgL chain construct, the D-eBPC harbours dual genomic receiver sites (DGRS; components 2E and 2F), each set to receive a IgH or IgL chain ORF. Thus, the D-eBPC is central to the DSS, and is a receptacle for DIVL-H or DIVL-H' (Components 1B or 1Q) encoded IgH chain sequences, and DIVL-L or DIVL-L' (Components 1C or 1 R) encoded IgL chain sequences. Integration of such sequences must result in the cells presenting a single discrete IgH/IgL pair encoded within the DIVL as a BCR on the cell surface, termed a D-eBPC-b, that may be contacted with tEp for purposes of performing a selection of one or more cells for recovery of IgH/IgL chain pairs that have a desirable engagement properties with said tEp.

The components and different states of a T-eBPC are depicted in Figure 16.

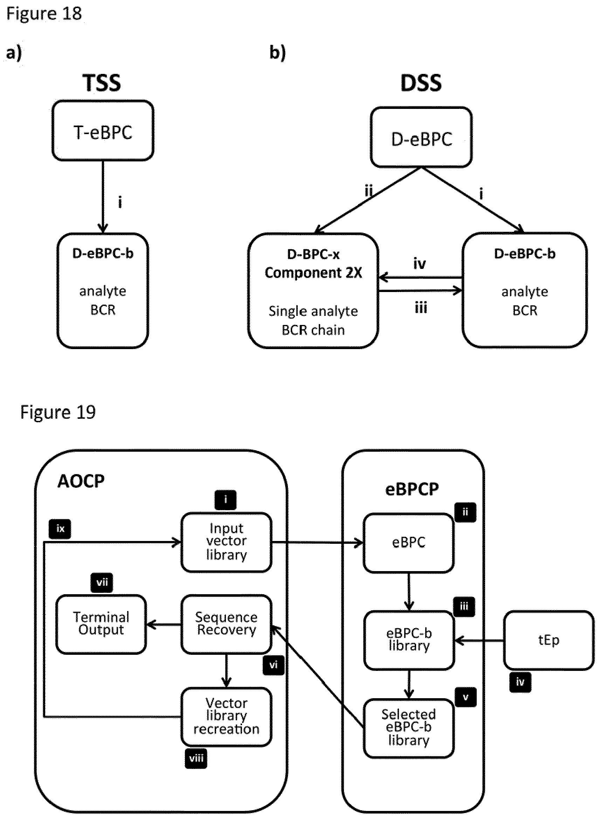

A key difference between the T-eBPC and D-eBPC operations to generate eBPC-b libraries, is that T-eBPC necessarily proceeds through a single step to the T-eBPC-b library (Figure 18a), whereas the D-eBPC can proceed in one or two steps to D-eBPC-b, and may be recycled back to intermediate states (Figure 18b).

The recycling of DGRS sites within the D-eBPC-b is a key part of the utility of the DSS. This key part is related to aspect of the DSS, where each IgH and IgL chain library, or individual sequences, may be manipulated independently. For example, selected D-eBPC-b may indeed be recycled back to an intermediate D-eBPC-x state that contains either IgH or IgL chain **(Component 2X;** see Figure 18b). This leaves either the DGRS-H or DGRS-L in the open conformation such that new DIVL-H/DIVL-H' or DIVL-L/DIVL-L' sequence can be integrated at this site.

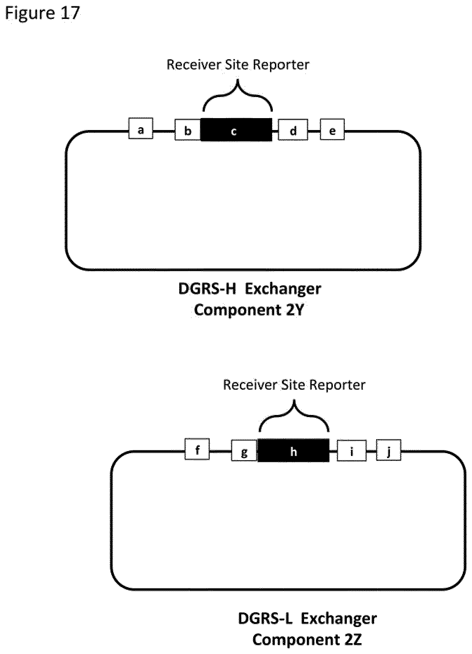

To achieve the recycling of an integrated DGRS to an open DGRS conformation, integration vectors are required to target either the DGRS-H or DGRS-L sites, and ideally carry with then reporter genes to mark the exchange of these sites. These components are termed DGRS-H Exchanger (**Component 2Y**) and DGRS-L Exchanger (**Component 2Z**), targeting the DGRS-H and DGRS-L sites, respectively (Figure 17).

A DGRS-H exchanger comprises
a) a 5' genetic element
b) a Kozak sequence
c) a first reporter gene
d) optional one or more untranslated 3' genetic element(s)
e) a 3' genetic element
f) a positive selection marker
g) an origin of replication
   a) Represents the 5' genetic element of the DGRS-H Exchanger **(Component 2Y),** representing the 5' sequence responsible for targeted genomic integration to the integrated DGRS-H. Thus, sequence element a) of the DGRS-H Exchanger is matched with the equivalent 5' genetic element of the DGRS-H;
   b) Represents a Kozak sequence for efficient translational initiation of the reporter gene that marks the integration of DGRS-H Exchanger sequences to the DGRS-H site;
   c) Represents the reporter gene of the DGRS-H Exchanger, gain of which is used to indicate the integration of sequences provide by the DGRS-H Exchanger, and is generally different to that of the DGRS-L exchanger reporter;
   d) Represents optional one or more untranslated 3' genetic element(s). These genetic elements may include, but are not limited to, transcript stabilizing or destabilizing elements, and unique identifier sequences.
   e) Represents the 3' genetic element of the DGRS-H Exchanger, representing the 3' sequence responsible for targeted genomic integration to the DGRS. Thus, sequence element e) of the DGRS-H Exchanger is matched with the equivalent 3' genetic element of the DGRS-H.
   f) Represents a positive selection gene for DGRS-H Exchanger plasmid selection propagation.
   g) Represents the plasmid origin of replication.

A DGRS-L exchanger comprises
a) a 5' genetic element
b) a Kozak sequence
c) a first reporter gene
d) optional one or more untranslated 3' genetic element(s)
e) a 3' genetic element
f) a positive selection marker
g) an origin of replication
wherein;
a) Represents the 5' genetic element of the DGRS-L Exchanger **(Component 2Z),** representing the 5' sequence responsible for targeted genomic integration to the integrated DGRS-L. Thus, sequence element a) of the DGRS-L Exchanger is matched with the equivalent 5' genetic element of the DGRS-L;
b) Represents a Kozak sequence for efficient translational initiation of the reporter gene that marks the integration of DGRS-L Exchanger sequences to the DGRS-L site;
c) Represents the reporter gene of the DGRS-L Exchanger, gain of which is used to indicate the integration of sequences provide by the DGRS-L Exchanger, and is generally different to that of the DGRS-H exchanger reporter;
d) Represents optional one or more untranslated 3' genetic element(s). These genetic elements may include, but are not limited to, transcript stabilizing or destabilizing elements, and unique identifier sequences.
e) Represents the 3' genetic element of the DGRS-L Exchanger, representing the 3' sequence responsible for targeted genomic integration to the DGRS. Thus, sequence element e) of the DGRS-L Exchanger is matched with the equivalent 3' genetic element of the DGRS-L.
f) Represents a positive selection gene for DGRS-H Exchanger plasmid selection propagation.
g) Represents the plasmid origin of replication.

### AOCP and eBPCP lists of components

**Table 1 Antibody ORF Construction Platform (ACOP) component summary**

| **Component** | **Short Name** | **Role** |
|---|---|---|
| 1A | TIVL | Delivery of tandem IgH/IgL chain constructs to T-eBPC |
| 1B | DIVL-H | Delivery of IgH chain constructs to D-eBPC |
| 1C | DIVL-L | Delivery of IgL chain constructs to D-eBPC |
| 1D | R-Prim-H | Reverse transcription primer pair(s) for recovery of IgH chain transcript amplicon from T/D-eBPC-b |
| 1E | R-Prim-L | Reverse transcription primer pair(s) for recovery of IgL chain transcript amplicon from T/D-eBPC-b |
| 1F | R-Amp-H | Reverse transcriptase amplicon of IgH chain amplicon recovered from T/D-eBPC-b |
| 1G | R-Amp-L | Reverse transcriptase amplicon of IgL chain amplicon recovered from T/D-eBPC-b |
| 1H | P-Prim-H | PCR primer pair(s) for amplification of IgH chain reverse transcriptase amplicon recovered from T/D-eBPC-b |
| 1I | P-Prim-L | PCR primer pair(s) for amplification of IgL chain reverse transcriptase amplicon recovered from T/D-eBPC-b |
| 1J | HFP-Amp-H | High-fidelity PCR product of IgH chain |
| 1J' | LFP-Amp-H | Low-fidelity PCR product of IgH chain |
| 1K | HFP-Amp-L | High-fidelity PCR product of IgL chain |
| 1K' | LFP-Amp-L | Low-fidelity PCR product of IgL chain |
| 1L | Backbone Entry | Vector backbone into which TIVL is recreated from PCR amplicons |
| 1M | Tac-Only | Donor vector donating constant gene segments and terminator sequences to reconstruct TIVL from PCR amplicons |
| 1N | TIVL' | The TIVL recreated from PCR amplicons |
| 1O | HC-Only | A vector backbone encoding constant gene segments for reconstruction of DIVL-H from PCR amplicons |
| 1P | LC-Only | A vector backbone encoding constant gene segments for reconstruction of DIVL-L from PCR amplicons |
| 1Q | DIVL-H' | A DIVL-H recreated from PCR amplicons |
| 1R | DIVL-L' | A DIVL-L recreated from PCR amplicons |

**Table 2 Engineered BCR presenting cell platform (eBPCP) component summary**

| **Component** | **Short Name** | **Role** |
|---|---|---|
| 2A | T-eBPC | A BCR-presenting cell that contains a tandem genomic receiver sites |
| 2B | TGRS (open) | The tandem genomic receiver sites contained within a T-eBPC that contains no IgH or IgL chain sequences |
| 2B' | TGRS (integrated) | The tandem genomic receiver sites contained within a T-eBPC that is integrated with IgH or IgL chain sequences from a TIVL or TIVL' |
| 2C | T-eBPC-b library | The library of T-eBPC cells created by integration of TIVL or TIVL' IgH/IgL chains sequences |
| 2C' | Selected T-eBPC library | A selection of one or more cells from the T-eBPC-b library on the basis of BCR engagement with target epitope (tEp) |
| 2D | D-eBPC | A BCR-presenting cell that contains dual genomic receiver sites |
| 2E | DGRS-H (open) | The dual genomic receiver sites contained within a D-eBPC that contains no IgH chain sequences |
| 2E' | DGRS-H (integrated) | The dual genomic receiver site contained within a D-eBPC that is integrated with IgH from a DIVL-H or DIVL-H' |
| 2F | DGRS-L (open) | The dual genomic receiver sites contained within a D-eBPC that contains no IgL chain sequences |
| 2F' | DGRS-L (integrated) | The dual genomic receiver site contained within a D-eBPC that is integrated with IgL from a DIVL-L or DIVL-L' |
| 2G | D-eBPC-b library | The library of D-eBPC cells created by integration of DIVL-H and DIVL-L and/or DIVL-H' and DIVL-L' IgH/IgL chains sequences |
| 2G' | Selected D-eBPC library | A selection of one or more cells from the D-eBPC-b library on the basis of BCR engagement with target epitope (tEp) |
| 2H | Synthetic reporter | A synthetic reporter construct integrated to the T/D-eBPC genome for reporting BCR signalling |
| 2X | D-eBPC-x | The library of D-eBPC cells created by integration of DIVL-H or DIVL-L, thus expressing a single IgH or IgL chain |
| 2Z | DGRS-H exchanger | The integration vector used to recycle the DGRS-H integrated conformation back to the DGRS-H open conformation |
| 2Y | DGRS-L exchanger | The integration vector used to recycle the DGRS-L integrated conformation back to the DGRS-L open conformation |

### AES operation

The architecture of the overall AES, and a detailed description of the components it is comprised of, has been provided above. This section will describe, in generic terms, many of the methodologies and considerations required to operate an AES to generate antibodies or BCR against a tEp. The generic operation of the AES is depicted in Figure 19.

The first consideration of an AES, is the synthesis and preparation of TIVL and/or DIVL input libraries. This may be achieved through a variety of methodologies, including direct synthesis, or combinatorial vector assembly. As described above, there are key considerations on what level of diversity is to be provided, and indeed, what form this diversity takes. There is much literature available to those skilled in the art that discuss positional amino acid biases in the CDR3 regions of IgH and IgL chains as well the selection and number of V and J segments. The AES as described in the present innovation can be used with combinatorial synthetic libraries based on human germline sequences and diverse CDR3s but also use native human antibody repertoires as a starting point. Furthermore, it should be noted, that while the main utility of the AES described in the present invention is to rapidly generate full-length native-like human antibodies, there same principles of this system could be applied and recapitulated with antibody sequences from other organisms, or partially artificial antibody V, J and C sequences. Finally, to achieve the large-scale integration workflows, significant amounts of DNA are required with which to transfect the eBPC. This is most conveniently achieved through production of plasmidic DNA in a microbial host. The selection and application of culture conditions to maintain the diversity of the synthesised TIVL or DIVL within the host organism is well known to those skilled in the art.

The preparation of the eBPC that act as receptacles for the TIVL and DIVL are constructed with features as described above. These cells should ideally be suspension cells to support high-density growth, and should be adapted to a minimal media, at least during selection procedures, as to avoid false-positive signals from expressed BCR engaging culture media components. A further consideration when the tEp represents a human-produced biomolecule, particular those that are surface-presented, is that the gene encoding the biomolecule, or the enzymes that produce, may in some instances need to be deleted from the eBPC cell to avoid the elimination of specific BCR during negative selection rounds (vide infra).

The generation of eBPC-b libraries using the input vector libraries may be achieved via a variety of methods. RMCE is the preferred format of TIVL/TGRS and DIVL/DGRS couples. In this case, eBPC must be transfected with large amounts of TIVL/DIVL along with a matched recombinase enzyme expression construct. This transfection can be achieved through a variety of methods well known to those skilled in the art. These methods include chemical transfection, electroporation or use of viral vectors, among others. The key consideration here is to achieve the maximum level of transfection to a sufficiently large culture. Considering that the theoretical diversity within a TIVL or DIVL library will generally far exceed the practical scale of an eBPC production culture, maximising efficiencies in this step will assure the largest cross-section of TIVL/DIVL-encoded sequence diversity is ultimately presented as BCR by eBPC-b. This procedure may also include selection of cells based on BCR expression, and subsequent outgrowth of the selected cell populations prior to selection. Such outgrowth procedures must be optimised as to maintain the BCR diversity of generated eBPC-b library. These relatively large-scale cell cultures may also be cryopreserved, such that they may be recalled and used in independent AES workflows against distinct tEp.

The tEp may represent essentially any molecule, macromolecule, or substrate that is not toxic to the eBPC cells at the quantities required to detect labelling of the eBPC by said tEp, or stimulation of the synthetic reporter via BCR engagement by the tEp.

An tEp entity may be provided as
a) a monomeric unlabelled reagent
b) a monomeric labelled reagent
c) a multimerised unlabelled reagent
d) a multimerised labelled reagent
e) a substrate immobilised reagent
Wherein, labelling can represent both fluorescent or other reporter motifs or affinity motifs for various selection purposes. Multimerisation and/or substrate immobilisation may be used for increasing sensitivity of labelling and/or BCR stimulation detection, and substrate immobilisation may be used for specific affinity enrichment selection workflows.

A tEp must impart a selection on an eBPC-b library, which may be achieved through a variety of means, including
a) Labelling of eBPC-b for selective sorting
b) Labelling of eBPC-b for affinity enrichment
c) Direct affinity enrichment
d) Stimulation of BCR response element for selective sorting or affinity enrichment
wherein; a) Represents FACS, or other high content single-cell selection technique, which selects positively (positive selection against tEp) or negatively labelled cells (negative selection against tEP). The label may for example be fluorescent for direct detection, or may present an epitope for secondary labelling with an independent reagent; b) represents MACS or other affinity enrichment technique for positive selection after secondary labelling; c) represents MACS or other affinity enrichment technique wherein the affinity enrichment leverages directly substrate-immobilised tEp.

One important aspect of the AES, is the ability to use the BCR response element to detect cells presenting a BCR that have engaged productively with a tEp. This provides the ability to select eBPC-b populations in a label-free manner, which is a significant advantage for small molecules or difficult to label entities.
A response element in this context, as described above, induces the expression of a reporter 'label', e.g. fluorescent protein and/or surface-exposed unique protein epitope, which can be employed in any of the above-mentioned selection approaches. Alternatively, the reporter could be a secreted molecule for detection by spectrometric, fluorescent or luminescent assays, wherein the populations could be selected based on the presence or absence of the reporter molecule. In addition, the response could be a combination of one or more different reporters. Indeed, there are advantages for such combination reporters, where a single line could be used for both FACS, MACS and traditional multi-well assay formats.

It is critical to note, that any given workflow is likely to include negative selection rounds, particularly in cases leveraging the BCR response element. In the case of a BCR response element, a naïve negative selection on the eBPC-b library not contact with an tEp would be desirable to eliminate background and increase sensitivity of the system. In any case, negative selection rounds may also be conducted with components such as multimerisation substrate, or affinity substrate, such that false positives recognising these entities are eliminated.

Any selected eBPC-b population may be managed in several non-exclusive ways. A typical approach would see the selected population directly partitioned to single cells and submitted for sequence recovery. This would see single-cell sorting using FACS selection directly deposit these cells in reaction vessels, for example. Partitioning of cells is required for pairing of IgH and IgL chain sequences in the context of most workflows. Alternatively, selected bulk populations can be submitted to single-cell partitioning methods such as microfluidic encapsulation, followed by encapsulated RT/PCR for sequence recovery. Such methods allow for ultrahigh-throughput pairing of IgH/IgL in a similar method as single cell sorting. Additionally, selected bulk populations may be reapplied to negative or positive selection rounds prior to further processing.

In any workflow, there may be advantages to a period of outgrowth of selected eBPC-b populations subsequent to selection and prior to re-selection of submission to sequence recovery. This outgrowth period is most likely to be a bulk population, but may also represent generation of monoclonal lines from the selected population for more detailed screening and characterisation. Monoclonal populations may be manipulated in a DSS context by exchanging a single DGRS to generate a polyclonal D-eTPC-x containing a selected population of IgH or IgL chain sequences. In many instances, it is likely a combination of approaches is employed.

Submission to the sequence recovery process is subsequent to any given selection and cell handling procedure. As outlined above, a typical workflow will see at least a proportion of the selected population submitted to high-fidelity sequence recovery for obtaining sequence information as an AES terminal output. This may be performed by any sequencing method that is able to account for IgH/IgL chain pairing from single cells. Generally, this will include Sanger sequencing of HFP-Amp pairs, but may also include batched next-generation sequencing approaches using nucleotide barcodes incorporated into the P-Prim components. Next-generation sequencing may also be used for general monitoring of eBPC-b libraries at any stage, and may also be readily employed for counting frequency of IgH or IgL clonotype occurrence, particular in selected D-eBPC-b, where one of the Ig chains is fixed, and therefore pairing of sequences is not necessary.

In most instances, a single round of selection will be insufficient to recover high-quality antibody sequences as a terminal output. Therefore, the sequence recovery module will typically be further employed to generate HFP-Amp and LFP-Amp products for recreation of TIVL and/or DIVL based on selected sequences. These TIVL and/or DIVL are then recycled as an input to the eBPC system for subsequent round of selection and recovery. Largely, this process will generate LFP-Amp products to impart sequence diversification on the resulting TIVL or DIVL. Such products may equally be drawn from pools of selected eBPC-b, or eBPC-b monoclones, in different workflow scenarios. The extraction of diversified sequence from a pool of cells will result in a higher content screen in the next round, whereas the focusing on one or more monoclones would restrict this pool in rounds where clear candidates have been cloned as eBPC-b and characterised. There is also a clear role for HFP-Amp products to be employed in the DSS context where one chain should be faithfully recapitulated from the selected cells, and the other chain diversified. The inclusion of HFP-Amp is also useful in multiple-cycle DSS workflows where high-quality pairs have been recovered, and a clonal D-eBPC-b is generated to faithfully recapitulate a single selected IgH/IgL chain pair. Such an approach would see detailed characterisation prior to D-eBPC-x generation for iterative diversification of either pair.

It should be noted that the AES system described herein may also be adapted for construction of cell lines that secrete a soluble variant of the BCR identified as the terminal product of the AES. In such a system, the AOCP includes derivatives of the TVIL/DIVL construction vectors encoding the IgH constant regions, (Components 1M, 1Q), wherein the IgH constant regions lack sequence encoding the transmembrane domain. Without transmembrane domain the expressed IgH/IgL chains are incapable of being imbedded in the cell membrane, and thus are secreted into the culture media as soluble antibodies. Indeed, the AOCP can incorporate derivatives of the Component 1M, 1Q wherein constant regions for the complete repertoire of human IGH are represented. The extension of the AOCP for soluble secretion enables rapid screening of putative IgH/IgL candidates for suitability as soluble production products or indeed different applications, for example where IgA, IgE or IgM might be preferable to IgG. Furthermore, the eBPC in principle can also operate as a production line in such instances, and so could be considered an T/D-eBPC expressing antibodies (T/D-eBPC-a), as these cells do not present the encoded IgH/IgL chains as BCR but soluble antibody instead.

### Legends to Figures

The invention is further illustrated in the following non-limiting figures.
*Figure 1**. Operation of the combined two-part Antibody Engineering System (AES) comprising combined modules of the Tandem-Site Subsystem (TSS) and Dual-Site Subsystem (DSS).*
   The modules of each of the four parts of the overall antibody engineering system (AES) are depicted, comprising of a tandem-site subsystem (TSS) and a dual-site subsystem (DSS). The different parts are represented by 'input part' (IP; white boxes), 'manufacturing part 1' (MP1; light grey boxes), 'output part' (OP; black box) and 'manufacturing part 2' (MP2; dark grey boxes). Arrows represent the processes executed within the system workflows, and thus denote the connectivity of modules in operation of the overall system, and the component TSS and DSS subsystems. The TSS is represented in the top half of the figure, and the DSS represented in the bottom half, where dedicated TSS and DSS are contained within the grey dashed boxes. The TSS and DSS share a universal sequence recovery module and output part that enables transition between the two subsystems during operation of the AES.
   In operation of the TSS, a Tandem integration vector library **(TIVL),** representing **Module 1 of the Input Part,** carrying both Immunoglobulin heavy (IgH) and Immunoglobulin light (IgL) chain open reading frames (ORF) (**Component 1A; see** **Figure 2**) is generated. This TIVL is combined with a Tandem-engineered BCR-presenting cell **(T-eBPC; Component 2A**), representing **Module 2 of the Input Part,** harboring a tandem genomic receiver site (**TGRS; Component 2B**) matched to the TIVL. The combination of these two inputs within **Module 1 of Manufacturing Part 1** (process i/ii), generates a library of T-eBPC expressing a BCR on the cell surface (T-eBPC-b; Component 2C), the BCR being encoded by the IgH and IgL chains carried by the TIVL.
   Within **Module 1 of Manufacturing Part 1,** a selection is made on the generated T-eBPC-b library on the basis of engagement of a target epitope **(tEp)** with the expressed BCR on the T-eBPC-b cell surface **(Component 2C'; see** **Figure 2****).** This selection restricts the T-eBPC-b pool to those cells expressing BCR that engage the tEp in a desirable manner. This restricted pool of T-eBPC-b is then submitted to **Module 2 of Manufacturing Part 1** for recovery of the IgH and IgL chain sequences (process iii).
   **Module 2 of Manufacturing Part 1** comprises the primer and amplicon toolset for IgH and IgL chain sequence recovery (**components 1D, 1E, 1F, 1G, 1H, 1I; see** **Figure 2** **and** **3****).** This module generates the PCR amplicons of the **Output Part modules 1 and 2,** in high fidelity (process iv) and low fidelity (process v) modes, respectively.
   **Module 1 of the Output Part** represents the high-fidelity PCR amplicons **(HF P-Amp, components 1J and 1K; see** **Figures 2** **and** **3**). These components represent the terminal outputs of the AES, as they represent heavy- and light- chain sequences selected on the basis of tEp engagement. In contrast, **Module 2 of the Output Part** represents the low-fidelity PCR amplicons (**LF P-Amp, components 1J' and 1K'; see** **Figures 2** **and** **3**), represent collections of diversified heavy- and light- chain sequences that are primarily used for to TIVL (and/or DIVL) recreation for further rounds of T-eBPC-b (and/or D-eBPC-b) selection of these diversified sequences on the basis tEp engagement. It is important to note that both HF P-Amp and LF P-Amp may be submitted to TIVL (and/or dual integration vector library; **DIVL**) recreation (processes vi and xi), as it is often desirable to hold either the IgH or IgL chain constant while diversifying the other chain (via LF P-Amp) in AES workflows.
   **Module 1 of the Manufacturing Part 2** represents a vector set (**components 1L and 1M; see** **Figure 2**) for recreation of a TIVL from HF P-Amp and/or LF P-Amp (**termed TIVL', component 1N; see** **Figure 2**). This TIVL' may be used as a TIVL in Module 1 of the Input Part to recycle the T-eBPC-b selection.
   In operation of the DSS, a DIVL, representing **Module 3 of the Input Part,** each library carrying IgH or IgL- chain ORFs **(DIVL-H and DIVL-L, respectively, Components 1B and 1C, respectively; see** **Figure 3****)** are generated. These DIVL-H and DIVL-L are combined with a dual engineered BCR-presenting cell **(D-eBPC; Component 2D),** representing **Module 4 of the Input Part.** These D-eBPC harbour a pair of genomic receiver sites, termed dual genomic receiver site **(DGRS; Components 2E and 2F),** matched to the DIVL. These sites are distinguished by their pairing, wherein the DGRS-H and DGRS-L are matched to the DIVL-H and DIVL-L, respectively. The combination of these two inputs within **Module 3 of Manufacturing Part 1** (process viii/ix), generates a library of D-eBPC expressing a BCR on the cell surface **(D-eBPC-b; Component 2G; see** **Figure 3****),** the BCR being encoded by the IgH and IgL chains carried by the DIVL-H and DIVL-L, respectively.
   Within **Module 3 of Manufacturing Part 1,** a selection is made on the generated D-eBPC-b library on the basis of engagement of a tEp with the expressed BCR on the D-eBPC-b cell surface **(Component 2G'; see** **figure 3****).** This selection restricts the T-eBPC-b pool to those cells expressing BCR that engage the tEp in a desirable manner. This restricted pool of D-eBPC-b is then submitted to **Module 2 of Manufacturing Part 1** for recovery of the IgH and IgL chain sequences (process iii).
   **Module 2 of Manufacturing Part** 1 and the modules of the **Output Part** are identical in operation of the DSS as for the TSS described above. These universal sequence recovery and output modules allow transition between TSS and DSS in placing HF P-Amp and/or LF P-Amp derived from the TSS into the DSS, or vice versa, for recycling of the systems during antibody engineering workflows.
   **Module 2 of the Manufacturing Part** 2 represents a vector set **(components 10 and 1P; see** **figure** 3) for recreation of a DIVL-H and DIVL-L from HF P- and/or LF P- Amp **(termed DIVL-H' and DIVL-L', respectively, component 1Q and 1R, respectively; see** **figure** 2). This pair of DIVL-H' and DIVL-L' may be used as a DIVL in **Module 1 of the Input Part** to recycle the D-eBPC-b selection.
*Figure 2**. Schematic representation of operation of the TSS*
   The tandem-site subsystem (TSS) modules are designed to operate in the context of both IgH and IgL chain ORFs being contained within a single vector backbone, and a single genomic receiver site in the eBPC. There are two fundamental elements of the overall TSS (and DSS). The first element represents the **engineered BCR presenting cell platform (eBPCP),** which is depicted within the grey shaded section. This represents the engineered cell platform and its products. The second element is the **antibody ORF construction platform (AOCP),** which are all components depicted outside of the grey shaded section, and represent vector, primer and amplicon sets used for genetic manipulation of the IgH and IgL chain antibody sequences.
   **Component 1A** represents the TIVL that encodes the desired IgH and IgL chain diversity for a given antibody engineering workflow. **Component 2A** represents the T-eBPC that is matched to the TIVL, in that the T-eBPC contains a TGRS; designated **component 2B.**
   The combination of the TIVL and T-eBPC **(process i/ii)**, generates a library of T-eBPC presenting a BCR (T-eBPC-b), **component 2C,** wherein the IgH and IgL chains are encoded in the converted TGRS, **component 2B'.** Each cell in the library of generated T-eBPC-b encodes a single pair of IgH and IgL chains that were encoded in a single vector contained within the originating TIVL.
   Contact of the T-eBPC-b with tEP **(process iii/iv),** permits the selection of a library of T-eBPC-b on the basis of tEP engagement, **component 2C'.** This selection process restricts the T-eBPC-b population to a sub-set of cells that express desired IgH and IgL chain combinations with regard to tEP engagement.
   **Components 1D and 1E** represent pairs of Reverse-transcriptase primers **(R-Prim)** that target either IgH and IgL chain sequences contained within the integrated TGRS of the selected T-eBPC-b library. Thus, the combination of the R-Prim and selected T-eBPC-b library **(process v/vi)** is used to generate reverse transcriptase amplicons **(R-Amp)** for both IgH and IgL chains, **components 1F and 1G, respectively**.
   R-Amp products are submitted to a further amplification step by way of nested PCR with PCR primers **(P-Prim)** specific for the IgH and IgL chain R-Amp products, **components 1H and 1I.**
   The process of nested PCR may be performed in two modes. Firstly, a high-fidelity PCR can be used **(process vii/viii)** to produce high-fidelity PCR amplicons (**HFP-Amp), components 1J and 1K**, for direct use in sequence analysis of recovered IgH and IgL chain sequences. Secondly, low-fidelity or high-fidelity PCR can be used **(process ix/x)** to produce PCR amplicons for submission to TIVL recreation for the selected IgH and IgL chain combinations recovered from the selected T-eBPC library. Furthermore, Process ix/x can be run in two different states to produce, low-fidelity PCR amplicons (**LFP-Amp**), **components 1J' and 1K'** or HFP-Amp, components 1J and 1K. The LFP-Amp and HFP-Amp products can be used in different IgH and IgL combinations in the TIVL recreation process to produced fully diversified (LFP-Amp IgH+IgL), partially diversified (one chain LFP-Amp, one chain HFP-Amp) or faithful recreation (HFP-Amp, IgH+IgL) of the BCR ORF from the selected T-eBPC.
   **Components 1L and 1M** represent a backbone entry vector and Tandem-constant-only vector, respectively, which are used to recreate the TIVL from HFP-Amp and/or LFP-Amp **(process xi/xii).** This process recreates a variant TIVL', **component 1N,** that represents a selected, and optionally sequence-diversified, library of IgH and IgL chain ORFs based on the measured engagement of tEp contacted with the original T-eBPC-b library.
   This TIVL' may then be combined with a T-eBPC **(process ii/xiii)** to iteratively generate a new T-eBPC-b library expressing the selected complementary BCR chains. These processes may then be repeatedly cycled until desired IgH and IgL chain combination characteristics are achieved with regard to tEp engagement.
*Figure 3**. Schematic representation of operation of the DSS*
   The dual-site subsystem (DSS) modules are designed to operate in the context of IgH and IgL chain ORFs being contained within separate vector backbone pairs, and separate genomic sites in the eBPC. There are two fundamental elements of the overall DSS (and TSS). The first element represents the **eBPCP,** which is depicted within the grey shaded section. This represents the engineered cell platform and its products. The second element is the **AOCP,** which are all component depicted outside of the grey shaded section, and represent vector, primer and amplicon sets used for genetic manipulation of the IgH and IgL chain antibody sequences.
   **Component 1B and 1C** represent the DIVL for DIVL-H and DIVL-L, respectively. Each vector library encodes the desired IgH and IgL chain diversity for a given antibody engineering workflow. **Component 2D** represents the D-eBPC that is matched to the DIVL-H/DIVL-L pair, in that the D-eBPC contains a DGRS; designated **components 2E and 2F.**
   The combination of the DIVL-H and DIVL-L and D-eBPC **(process i/ii),** generates a library D-eBPC presenting a BCR (D-eBPC-b), **component 2G,** wherein the IgH and IgL chains are encoded in the converted DGRS, **component 2E' and 2F'.** Each cell in the library of generated D-eBPC-b encodes a single pair of IgH and IgL chains that were encoded in a random pair of vectors contained within the originating DIVLs.
   Contact of the D-eBPC-b with a tEP **(process iii/iv),** permits the selection of a library of D-eBPC-b on the basis of tEP engagement, **component 2G'.** This selection process restricts the D-eBPC-b population to a sub-set of cells that express desired IgH and IgL combinations with regard to tEP engagement.
   **Components 1D and 1E** represent pairs of R-Prim that target either IgH and IgL chain sequences contained within the integrated DGRS of the selected D-eBPC-b library.
   Thus, the combination of the R-Prim and selected D-eBPC-b library **(process v/vi)** is used to generate R-Amp for both IgH and IgL chains, **components 1F and 1G.**
   R-Amp products are submitted to a further amplification step by way of nested PCR with PCR primers (P-Prim) specific for the IgH and IgL chain R-Amp products, **components 1H and 1I.**
   The process of nested PCR may be performed in two modes. Firstly, a high-fidelity PCR can be used **(process vii/viii)** to produce high-fidelity PCR amplicons (HF P-Amp), **components 1J and 1K**, for direct use in sequence analysis of recovered IgH and IgL chain sequences. Secondly, low-fidelity or high-fidelity PCR can be used **(process ix/x)** to produce PCR amplicons for submission to DIVL recreation for the selected IgH and IgL chain combinations recovered from the selected D-eBPC library. Furthermore, Process ix/x can be run in two different states to produce, low-fidelity PCR amplicons (**LFP-Amp**), **components 1J' and 1K'** or HFP-Amp, components 1J and 1K. The LFP-Amp and HFP-Amp products can be used in different IgH and IgL combinations in the DIVL recreation process to produced fully diversified (LFP-Amp IgH+IgL), partially diversified (one chain LFP-Amp, one chain HFP-Amp) or faithful recreation (HFP-Amp, IgH+IgL) of the BCR ORF from the selected D-eBPC.
   **Components 1O and 1P** represent heavy-constant-only (HC-only) and light-constant-only (LC-only) vectors, respectively, which are used to recreate the DIVL-H and DIVL-L from HFP-Amp and/or LFP-Amp **(processes xi/xii and process xiii/xiv, respectively).** This process recreates a variant DIVL-H' and DIVL-L', **component 1Q and 1R,** respectively. These libraries represent a selected and optionally sequence-diversified library of IgH and IgL chain ORFs based on the measured engagement of tEp contacted with the original D-eBPC-b library.
   These DIVL' may then be combined with a D-eBPC **(process ii/xv)** to iteratively generate a new D-eBPC-b library expressing the selected complementary BCR chains. These processes may then be repeatedly cycled until desired IgH and IgL chain combination characteristics are achieved with regard to tEp engagement.
*Figure 4**. TIVL Plasmid Architecture*
   A schematic representation of the TIVL plasmid architecture is depicted, representing **component 1A** of the TSS. A TIVL is a library of one or more vectors that represents the desired IgH and IgL sequence diversity to be inserted to a selected eBPC. Thus, a TIVL is the primary input to a TSS system; containing the IgH/L sequences to be presented by a T-eBPC.
   The TIVL plasmid is depicted as a closed plasmid, with each lettered box representing a key element of the plasmid architecture. The TIVL plasmid has the IgH and IgL chain sequences encoded in opposite sense. a) Represents the 5' genetic element of the TIVL plasmid, representing the 5' sequence responsible for targeted genomic integration to the TGRS (component 2B) contained within a T-eBPC (component 2A). b) Represents a Kozak sequence for efficient translational initiation of the IgH transcript in the eBPC. c) Represents the leader sequence for the IgH chain encoded by elements d) to g). d) Represents the IGHV region. e) Represents the CDRH3 region. f) Represents the IGHJ region, g) Represents the IGHC region. h) Represents a transcriptional terminator sequence for the sense-direction encoded IgH chain, and optionally one or more 3' untranslated genetic elements. These genomic elements may include, but are not limited to, transcript stabilizing or destabilizing elements, and unique identifier sequences. i) Represents the transcriptional terminator sequence for the antisense-direction encoded IgL chain, and optionally one or more 3' untranslated genetic elements. These genomic elements may include, but are not limited to, transcript stabilizing or destabilizing elements, and unique identifier sequences. j) Represents the IGLC region. k) Represents the IGLJ region. I) Represents the CDRL3 region. m) Represents the IGLV region, n) Represents the leader sequence IgL chain encoded by elements j) to m). o) Represents a Kozak sequence for efficient translational initiation of the IgL transcript in the eBPC p) Represents the 3' genetic element of the TIVL plasmid, representing the 3' sequence responsible for targeted genomic integration to the TGRS contained within a T-eBPC. q) Represents a positive selection gene for TIVL plasmid selection and propagation. r) Represents the plasmid origin of replication.
*Figure 5**. DIVL Plasmid Architecture*
   A schematic representation of the DIVL plasmid architecture is depicted, representing **components 1B and 1C** of the DSS. A DIVL is comprised of a DIVL-H, encoding the Ig heavy chain (IgH), and a DIVL-L encoding the Ig light chain (IgL). Each of the DIVL-H and DIVL-L comprises a library of one or more vectors that represents the desired IgH and IgL sequence diversity, respectively, to be inserted to a selected D-eBPC. Thus, a pair of DIVL are the primary input to a DSS system; containing the IgH/L sequences to be presented by a D-eBPC.
   The DIVL plasmids are depicted as closed plasmids, with each lettered box representing a key element of the plasmid architecture. The key difference of a DIVL system compared to the TIVL plasmid system described in Figure 4, is that IgH and IgL are encoded on separate plasmids within the paired DIVL libraries, rather than encoded in single plasmid as is the case for the TIVL. Both DIVL forms have the IgH and IgL encoded in the sense direction. a) Represents the 5' genetic element of the DIVL-H plasmid, representing the 5' sequence responsible for targeted genomic integration to the first Dual genomic receiver site heavy chain **(DGRS-H; component 2E)** contained within a T-eBPC **(component 2D).** b) Represents a Kozak sequence for efficient translational initiation of the IgH transcript in the D-eBPC. c) Represents the leader sequence IgH chain encoded by elements d) to g). d) Represents the IGHV region. e) Represents the CDRH3 region, f) Represents the IGHJ region. g) Represents the IGHC region. h) Represents an optional one or more untranslated 3' genetic element(s). These genetic elements may include, but are not limited to, transcript stabilizing or destabilizing elements, and unique identifier sequences. i) Represents the 3' genetic element of the DIVL-H plasmid, representing the 3' sequence responsible for targeted genomic integration to the DGRS-H **(component 2E)** contained within a D-eBPC. j) Represents a positive selection gene for DIVL-H plasmid selection propagation DIVL-H. k) Represents the plasmid origin of replication.
   I) Represents the 5' genetic element of the DIVL-L plasmid, representing the 5' sequence responsible for targeted genomic integration to the second Dual genomic receiver site light-chain **(DGRS-L; component 2F)** contained within a T-eBPC **(component 2D).** m) Represents a Kozak sequence for efficient translational initiation of the IgL transcript in the D-eBPC. n) Represents the leader sequence IgL chain encoded by elements o) to r). o) Represents the IGLV region. p) Represents the CDRL3 region. q) Represents the IGLJ region, r) Represents the IGLC region. t) Represents the 3' genetic element of the DIVL-L plasmid, representing the 3' sequence responsible for targeted genomic integration to the DGRS-L **(component 2E)** contained within a D-eBPC. u) Represents a positive selection gene for DIVL-H plasmid selection propagation DIVL-H. v) Represents the plasmid origin of replication.
   In this paired library system, the 3'/5' genetic elements contained within each of the DIVL-H and DIVL-L must be different, as to provide insulated integration of interposed sequence into the DGRS-H and DGRS-L, respectively, contained within the D-eBPC.
*Figure 6**. TGRS Architecture* - *Open*
   A schematic representation of the open TGRS architecture is depicted, representing **component 2B** of the TSS. A TGRS is a synthetic sequence cassette inserted into the genome of a T-eBPC, and is designed to be paired with the TIVL such that the IgH and IgL sequences encoded by said TIVL may be integrated in a site-specific manner to the genome of the T-eBPC within the TGRS cassette. This figure depicts the 'open' confor-mation of this site, encoding a constitutive reporter gene. Importantly, only a single copy of a TGRS cassette may be contained within the genome of a T-eBPC, such that only one IgH/L paired sequence encoded within the TIVL may be delivered to the genome of each single cell within a T-eBPC population, to create a library of T-eBPC-b, each expressing a single discrete IgH/L pair.
   The TGRS cassette is depicted as a section of genomic DNA, with the ellipsis at either end indicating that this cassette comprises a small internal sequence of a given chromosome, with each lettered box representing a key element of the interaction of the TGRS with TIVL elements, and the cloning process. The numbered boxes represent flanking genomic features of the TGRS, which affect expression of the internal elements. a) Represents the 5' genetic element of the TGRS, representing the 5' sequence responsible for targeted genomic integration to the TIVL-encoded IgH/L chain pair. Thus, sequence element a) of the TGRS is matched with the equivalent 5' genetic element of the TIVL (Figure 4, element a). b) Represents a Kozak sequence for efficient translational initiation of the reporter gene that constitutively marks the presence of an intact TGRS within a T-eBPC. **c**) Represents a reporter gene of the TGRS, loss of which is used to indicate the integration of sequences provide by the TIVL. **d**) Represents a transcriptional terminator sequence for encoded reporter gene. Note, in second embodiment b-c-d components may be replicated **(b'-c'-d')** and inserted immediately downstream of b-c-d in the antisense direction, but before e, to mimic the TIVL architecture. In such an instance, it is preferred that reporter genes **c** and **c'** are different. e) Represents the 3' genetic element of the TGRS, representing the 3' sequence responsible for targeted genomic integration to the TIVL-encoded IgH/L chain pair. Thus, sequence element e) of the TGRS is matched with the equivalent 3' genetic element of the TIVL (Figure 4, element i). 1) and 2) represent 5' and 3' genomic elements, respectively. Each of these genomic elements encodes features that that regulate expression and tracking of reporter and IgH/L transcripts. These genomic elements minimally include promoter sequences that drive reporter c and IgH chain expression in the case of 1), and IgL chain expression, and optional c', in the case of 2).
*Figure 7**. TGRS Architecture* - *Integrated*
   A schematic representation of the integrated TGRS architecture is depicted, representing **component 2B'** of the TSS. A TGRS is a synthetic sequence cassette inserted into the genome of a T-eBPC, and is designed to be paired with the TIVL such that the IgH and IgL sequences encoded by said TIVL may be integrated in a site-specific manner to the genome of the T-eBPC within the TGRS cassette. This figure depicts the 'integrated' conformation of this site, encoding the pair of IgH/L chains delivered by the TIVL. Importantly, only a single copy of a TGRS cassette may be contained within the genome of a T-eBPC, such that only one IgH/L paired sequence encoded within the TIVL may be delivered to the genome of each single cell within a T-eBPC population, to create a library of T-eBPC-b, each expressing a single discrete IgH/L pair.
   The TGRS cassette is depicted as a section of genomic DNA, with the ellipsis at either end indicating that this cassette comprises a small internal sequence of a given chromosome, with each lettered box representing an element delivered to the TGRS via the TIVL. The numbered boxes represent flanking genomic features of the TGRS, which affect expression of the internal elements.
   a) Represents the 5' genetic element common between the TIVL and TGRS, representing the 5' sequence responsible for targeted genomic integration of the TIVL sequences to the TGRS (i.e. Figures 4 and 6, element a in each). b) Represents a Kozak sequence for efficient translational initiation of the IgH transcript in the eBPC. c) Represents the leader sequence IgH chain encoded by elements d) to g). d) Represents the IGHV region. e) Represents the CDRH3 region. f) Represents the IGHJ region. g) Represents the IGHC region. h) Represents a transcriptional terminator sequence for the sense-direction encoded IgH chain, and optionally one or more 3' untranslated genetic elements. These genetic elements may include, but are not limited to, transcript stabilizing or destabilizing elements, and unique identifier sequences. i) Represents the transcriptional terminator sequence for the antisense-direction encoded IgL chain, and optionally one or more 3' untranslated genetic elements. These genetic elements may include, but are not limited to, transcript stabilizing or destabilizing elements, and unique identifier sequences. j) Represents the IGLC region. k) Represents the IGLJ region. I) Represents the CDRL3 region. m) Represents the IGLV region. n) Represents the leader sequence IgL chain encoded by elements j) to m). o) Represents a Kozak sequence for efficient translational initiation of the IgL transcript in the eBPC p) Represents the 5' genetic element common between the TIVL and TGRS, representing the 5' sequence responsible for targeted genomic integration of the TIVL sequences to the TGRS (i.e. Figures 4 and 6, element p and e, respectively). It is important to note that elements a through p are equivalent to the elements a through p of the TIVL depicted in Figure 4, since this is the sequence delivered by the TIVL.
      1) and 2) represent 5' and 3' genomic elements, respectively. Each of these genomic elements encodes features that that regulate expression and tracking of IgH/L transcripts. These genomic elements minimally include promoter sequences that drive IgH chain expression in the case of 1), and IgL chain expression in the case of 2).
*Figure 8**. DGRS Architecture* - *Open*
   A schematic representation of the open DGRS architecture is depicted, representing **components 2E and 2F** of the DSS. Each DGRS is a synthetic sequence cassette inserted into the genome of a D-eBPC, and is designed to be paired with the DIVLs such that the IgH and IgL sequences encoded by said DIVLs may be integrated in a site-specific manner to the genome of the D-eBPC within the DGRS cassettes. Thus, a D-eBPC contains two DGRS sites, a DGRS-H and DGRS-L, which are paired with the DIVL-H and DIVL-L, respectively. This figure depicts the 'open' conformation of each site, encoding constitutive reporter genes. Importantly, only a single copy of each DGRS cassette may be contained within the genome of a D-eBPC, such that only one IgH/L paired sequence encoded within the DIVLs may be delivered to the genome of each single cell within a D-eBPC population, to create a library of D-eBPC-b, each expressing a single discrete IgH/L pair.
   The DGRS cassettes are depicted as a section of genomic DNA, with the ellipsis at either end indicating that these cassettes comprise a small internal sequence of a given chromosome, with each lettered box representing a key element of the interaction of the DGRS with DIVL elements, and the cloning process. The numbered boxes represent flanking genomic features of the DGRS, which affect expression of the internal elements. Each of the DGRS resembles closely the architecture of the TGRS cassette described in Figure 6.
   a) Represents the 5' genetic element of the DGRS-H **(Component 2E),** representing the 5' sequence responsible for targeted genomic integration to the DIVL-H-encoded IgH chain. Thus, sequence element a) of the DGRS-H is matched with the equivalent 5' genetic element of the DIVL-H (Figure 5, element a). b) Represents a Kozak sequence for efficient translational initiation of the reporter gene that constitutively marks the presence of an intact DGRS-H within a D-eBPC, and which is different from the equivalent reporter encoded by the DGRS-L (element h). c) Represents the reporter gene of the DGRS-H, loss of which is used to indicate the integration of sequences provide by the DIVL-H d) Represents optionally one or more 3' untranslated genomic elements. These genomic elements may include, but are not limited to, transcript stabilizing or destabilizing elements, and unique identifier sequences. e) Represents the 3' genetic element of the DGRS-H, representing the 3' sequence responsible for targeted genomic integration to the DIVL-H-encoded IgH chain. Thus, sequence element e) of the DGRS-H is matched with the equivalent 3' genetic element of the DIVL-H (Figure 5, element i).
   f) Represents the 5' genetic element of the DGRS-L **(Component 2F),** representing the 5' sequence responsible for targeted genomic integration to the DIVL-L-encoded IgL chain. Thus, sequence element f) of the DGRS-L is matched with the equivalent 5' genetic element of the DIVL-L (Figure 5, element I). g) Represents a Kozak sequence for efficient translational initiation of the reporter gene that constitutively marks the presence of an intact DGRS-L within a D-eBPC, and which is different from the equivalent reporter encoded by the DGRS-H (element c). h) Represents the reporter gene of the DGRS-L, loss of which is used to indicate the integration of sequences provide by the DIVL-L. i) Represents optionally one or more 3' untranslated genomic elements. These genomic elements may include, but are not limited to, transcript stabilizing or destabilizing elements, and unique identifier sequences. j) Represents the 3' genetic element of the DGRS-L, representing the 3' sequence responsible for targeted genomic integration to the DIVL-L-encoded IgL chain. Thus, sequence element j) of the DGRS-L is matched with the equivalent 3' genetic element of the DIVL-L (Figure 5, element t).
      1) and 3) represent 5' genomic elements of the DGRS-H and DGRS-L, respectively. Each of these genomic elements encodes features that that regulate expression and tracking of reporters and IgH or IgL transcripts. These genomic elements minimally include promoter sequences that drive reporter c and IgH chain expression in the case of 1), and reporter h and IgL chain expression in the case of 3).
      2) and 4) represent 3' genomic elements of the DGRS-H and DGRS-L, respectively, and includes at least a transcriptional terminator sequence. These genomic elements may also include, but are not limited to, transcript stabilizing or destabilizing elements, and unique identifier sequences.
*Figure 9**. Dual Genomic Receiver Site (DGRS) Architecture* - *Integrated*
   A schematic representation of the integrated DGRS architecture is depicted, representing **components 2E' and 2F'** of the DSS. Each DGRS is a synthetic sequence cassette inserted into the genome of a D-eBPC, and is designed to be paired with the DIVLs such that the IgH and IgL sequences encoded by said DIVLs may be integrated in a site-specific manner to the genome of the D-eBPC within the DGRS cassettes. Thus, a D-eBPC contains two DGRS sites, a DGRS-H and DGRS-L, which are paired with the DIVL-H and DIVL-L, respectively. This figure depicts the 'integrated' conformation of each site, encoding the pair of IgH and IgL chains at the DGRS-H and DGRS-L, respectively, delivered by the DIVL-H and DIVL-L, respectively. Importantly, only a single copy of each DGRS cassette may be contained within the genome of a D-eBPC, such that only one IgH/L paired sequence encoded within the DIVLs may be delivered to the genome of each single cell within a D-eBPC population, to create a library of D-eBPC-b, each expressing a single discrete IgH/L pair.
   The DGRS cassettes are depicted as a section of genomic DNA, with the ellipsis at either end indicating that these cassettes comprise a small internal sequence of a given chromosome, with each lettered box representing an element delivered to the DGRS sites via the DIVLs. The numbered boxes represent flanking genomic features of each DGRS, which affect expression of the internal elements.
   a) Represents the 5' genetic element common between the DIVL-H and DGRS-H, representing the 5' sequence responsible for targeted genomic integration of the DIVL-H sequences to the DGRS-H (i.e. Figures 5 and 8, element a in each). b) Represents a Kozak sequence for efficient translational initiation of the IgH transcript in the eBPC. c) Represents the leader sequence IgH chain encoded by elements d) to g). d) Represents the IGHV region. e) Represents the CDRH3 region. f) Represents the IGHJ region. g) Represents the IGHC region. h) Represents an optional one or more untranslated 3' genetic element(s). These genetic elements may include, but are not limited to, transcript stabilizing or destabilizing elements, and unique identifier sequences. i) Represents the 3' genetic element common between the DIVL-H and DGRS-H, representing the 3' sequence responsible for targeted genomic integration of the DIVL-H sequences to the DGRS-H (i.e. Figures 5 and 8, element i and e, respectively). It is important to note that elements a through i are equivalent to the elements a through i of the DIVL-H depicted in Figure 5, since this is the sequence delivered by the DIVL-H.
   I) Represents the 5' genetic element common between the DIVL-L and DGRS-L, representing the 5' sequence responsible for targeted genomic integration of the DIVL-L sequences to the DGRS-L (i.e. Figures 5 and 8, elements I and f, respectively). m) Represents a Kozak sequence for efficient translational initiation of the IgL transcript in the eBPC. n) Represents the leader sequence IgL chain encoded by elements o) to r). o) Represents the IGLV region. p) Represents the CDRL3 region. q) Represents the IGLJ region. r) Represents the IGLC region. s) Represents an optional one or more untranslated 3' genetic element(s). These genetic elements may include, but are not limited to, transcript stabilizing or destabilizing elements, and unique identifier sequences. t) Represents the 3' genetic element common between the DIVL-L and DGRS-L, representing the 3' sequence responsible for targeted genomic integration of the DIVL-L sequences to the DGRS-L (i.e. Figures 5 and 8, element t and j, respectively). It is important to note that elements i through t are equivalent to the elements i through t of the DIVL-L depicted in Figure 5, since this is the sequence delivered by the DIVL-L.
      1) and 3) represent 5' genomic elements of the DGRS-H and DGRS-L, respectively. Each of these genomic elements encodes features that that regulate expression and tracking of reporters and IgH or IgL transcripts. These genomic elements minimally include promoter sequences that drive reporter c and IgH chain expression in the case of 1), and reporter h and IgL chain expression in the case of 3).
      2) and 4) represent 3' genomic elements of the DGRS-H and DGRS-L, respectively, and includes at least a transcriptional terminator sequence. These genomic elements may also include, but are not limited to, transcript stabilizing or destabilizing elements, and unique identifier sequences.
*Figure 10**. Composition and function of the sequence recovery module*
   The components and function of the sequence recovery module is depicted schematically, using the recovery of IgH chain sequence from a DGRS-H cassette that would be contained within a D-eBPC-b, as an example.
   The sequence recovery module is designed to recover PCR amplicons of IgH and IgL sequences in two modes; Mode 1, amplicons that are suitable for obtaining sequence information, and Mode 2 amplicons that are suitable for recreation of DIVLs and/or TIVLs. In obtaining sequence information (Mode 1), all steps of the sequence recovery modules are conducted in HF-state conditions that ensure high-fidelity (HF) transcripts. For recreation of DIVL and/or TIVL (Mode 2), the PCR steps of the sequence recovery module may be conducted in both HF- and low-fidelity (LF-) states. HF-state would permit regeneration of a DIVL and/or TIVL that faithfully recapitulates the IgH and/or IgL chain sequence(s) recovered into new DIVL' and/or TIVL' states. LF-state is used to impart sequence diversification upon the IgH and/or IgL chain sequence(s) recovered into new DIVL' and/or TIVL' states for subsequent re-use of the D-eBPC and T-eBPC for a further round(s) of tEp selection on the diversified Ig sequences.
   Constructs and products are depicted as linear sequences, with each lettered box representing a key element of the sequence architecture, whereas primers are depicted as black arrows. The top panel depicts component 2E' described in Figure 9. a) Represents the 5' genetic element common between the DIVL-H and DGRS-H, representing the 5' sequence responsible for targeted genomic integration of the DIVL-H sequences to the DGRS-H (i.e. Figures 5 and 8, element a in each). b) Represents a Kozak sequence for efficient translational initiation of the IgH transcript in the eBPC. c) Represents the leader sequence IgH chain encoded by elements d) to g). d) Represents the IGHV region. e) Represents the CDRH3 region. f) Represents the IGHJ region. g) Represents the IGHC region. h) Represents an optional one or more untranslated 3' genetic element(s). These genetic elements may include, but are not limited to, transcript stabilizing or destabilizing elements, and unique identifier sequences. i) Represents the 5' genetic element common between the DIVL-H and DGRS-H, representing the 5' sequence responsible for targeted genomic integration of the DIVL-H sequences to the DGRS-H (i.e. Figures 5 and 8, element i and e, respectively). It is important to note that elements a through i are equivalent to the elements a through i of the DIVL-H depicted in Figure 5, since this is the sequence donated by the DIVL-H.
   1) represents 5' genomic elements of the DGRS-H. Each of these genomic elements encodes features that that regulate expression and tracking of IgH transcripts. These genomic elements minimally include a promoter sequence that drive IgH chain expression. 2) represents 3' genomic elements of the DGRS-H, and includes at least a transcriptional terminator sequence. These genomic elements may also include, but are not limited to, transcript stabilizing or destabilizing elements, and unique identifier sequences.
   The first step of the sequence recovery module uses R-Prim, which preferentially runs in HF-state, that are specific for 5' and 3' sequences of the IgH chain integrated to the DGRS-H, R-Prim-H F and R-Prim-H R (**Component 1D**), respectively (F designates forward and R designates reverse primer binds). The R-Prim-H F binds to a universal sequence centred on the IgH leader sequence (element c), while the R-Prim-H R binds sequence 3' of the IGHJ/IGHC boundary. A RT reaction using the above-described elements generates the RT amplicon depicted in the centre panel, spanning IgH sequence from the IgH chain leader through the IGHJ (Component 1F, R-Amp-H).
   The R-Amp-H product is then subjected to nested PCR using primers binding the same or internal sequence (P-Prim-H F and Prim-H R), but with extensions depicted as elements w and x (**Component 1H**). Thus, submission of these components to a PCR reaction yields a PCR amplicon depicted in the lower panels as P-Amp-H **(Component 1J or 1J'**), which represents an internal IgH chain sequence flanked by single-stranded DNA overhang sequence, elements w and x. These overhang sequences have extended architecture that enables operation within the DIVL and TIVL recreation modules. A HF-state PCR process generates a HFP-Amp product designated **Component 1J,** whereas a LF-state PCR process generates LFP-Amp product designated **Component 1 J'.**
   A completely analogous system exists for the recovery of the IgL sequences from the DGRS-L cassette. R-Prim-L are designated as **Component 1E,** and the R-Amp-L product as **Component 1G.** P-Prim-L are designated **Component 1I**, and P-Amp-L designated as **Component 1K or 1K'**. (not depicted, see Figures 2 and 3).
   Considering that the TGRS contains identical IgH and IgL chain sequence architecture when compared to the DGRS-H and DGRS-L, respectively, the sequence recovery module can be cycled in the same manner to recover the same output, **Components 1J and/or 1J' and/or 1K and/or 1K'**. This is a key feature of operating the AES, wherein the sequence recovery module outputs derived from TSS or DSS are interchangeable and compatible as inputs into Module 1 and/or Module 2 of MP2 for recreation of TIVL or DIVL contexts.
*Figure 11**. TIVL' recreation module plasmid architecture*
   A schematic representation of the TIVL recreation module plasmid architecture is depicted, representing **components 1L and 1M** of the TSS.
   The TIVL recreation module is used to convert P-Amp components (1J, 1K, 1J', 1K') derived from the sequence recovery module into a TIVL, termed TIVL'. This TIVL 'recreation' process is used to cycle the AES with recovered and/or diversified sequences, which have been selected against tEp engagement in antibody engineering workflows.
   The TIVL recreation module plasmids are depicted as closed plasmids, with each lettered box representing a key element of the plasmid architecture. A TIVL recreation module comprises of two plasmids. The Backbone Entry Vector **(Component** 1L), and the Tandem Constant-Only Vector (**TaC-Only; Component 1M**). The Backbone Entry vector provides the vector backbone and genetic elements of the final TIVL', whereas the TaC-Only vector provides the elements that need to be incorporated between the P-Amp-derived sequences, including both invariant (constant) segments of the IGHC and IHLC and the terminator sequences.
   The Backbone Entry vector is depicted in the upper panel. a) Represents the 5' genetic element of the Backbone Entry plasmid, representing the 5' sequence responsible for targeted genomic integration to the TGRS contained within a T-eBPC. This 5' sequence is identical, and fulfills the same role as the 5' genetic element of the TIVL (Figure 4, element a). b) Represents the first overhang sequence, which encodes a unique sequence for a single-strand DNA overhang created by the action of the Type IIS enzyme binding to its recognition sequence immediately to the 3' of the overhang sequence. c) Represents a first Type IIS recognition sequence, relating to the binding of and action of the enzyme creating the overhang encoded in element b). d) Represents a negative selection marker used to negatively select for parental Entry Backbone vector that is undigested by Type IIS enzyme action. e) Represents a second Type IIS recognition sequence, relating to the binding of and action of the enzyme creating the overhang encoded in element f). f) Represents the second overhang sequence, which encodes a unique sequence for a single-strand DNA overhang created by the action of the Type IIS enzyme binding to its recognition sequence immediately to the 5' of the overhang sequence. g) Represents the 3' genetic element of the Backbone Entry plasmid, representing the 3' sequence responsible for targeted genomic integration to the TGRS contained within a T-eBPC. This 3' sequence is identical, and fulfills the same role as the 3' genetic element of the TIVL (Figure 4, element p). h) Represents a first positive selection gene for both Backbone Entry vector and TIVL' plasmid selection and propagation, and which is different from the second positive selection gene contained within the TaC-only vector. i) Represents the plasmid origin of replication.
   The TaC-Only vector is depicted in the lower panel. As for the TIVL depicted in Figure 4, the IgH-related elements are encoded in the sense direction, whereas the IgL-related elements are encoded in the antisense direction. j) Represents a third Type IIS recognition sequence, relating to the binding of and action of the enzyme creating the overhang encoded in element k). k) Represents the third overhang sequence, which encodes a unique sequence for a single-strand DNA overhang created by the action of the Type IIS enzyme binding to its recognition sequence immediately to the 5' of the overhang sequence. I) Represents a IGHC gene segment fragment that is not encoded by the pAMP-H product. m) Represents a first transcriptional terminator sequence encoded in the sense direction, and optionally one or more 3' untranslated genetic elements. These genetic elements may include, but are not limited to, transcript stabilizing or destabilizing elements, and unique identifier sequences. n) Represents a second terminator sequence encoded in the antisense direction and optionally one or more 3' untranslated genetic elements. These genetic elements may include, but are not limited to, transcript stabilizing or destabilizing elements, and unique identifier sequences. o) Represents a IGLC gene segment fragment that is not encoded by the pAMP-H product, and in the antisense direction. p) Represents the fourth overhang sequence, which encodes a unique sequence for a single-strand DNA overhang created by the action of the Type IIS enzyme binding to its recognition sequence immediately to the 3' of the overhang sequence. q) Represents a fourth Type IIS recognition sequence, relating to the binding of and action of the enzyme creating the overhang encoded in element p). r) Represents a second positive selection gene for TaC-Only plasmid selection and propagation, and which is different from the first positive selection gene contained within the Backbone Entry vector. s) Represents the plasmid origin of replication.
*Figure 12**. TIVL'generation from P-Amp products*
   The process of TIVL' generation within the TIVL recreation module is depicted. Constructs and RT/PCR products are depicted as linear sequences, with each lettered or numbered box representing a key element of the sequence architecture, whereas primers are depicted as black arrows, plasmids are depicted in both open (digested) and circularized conformations with lettered and numbered boxes represent key sequence elements.
   i) Depicts R-Amp-H (component 1F) and R-Amp-L (component 1G) that may be obtained from either a TGRS or DGRS by amplification with RT primers (Components 1D and 1E, not depicted, see Figure 10). The R-Amp elements have been numbered whereas; 1) represents the leader sequence IgH chain; 2) represents the IGHV region; 3) represents the CDRH3 region; 4) Represents the IGHJ region; 5) represents the leader sequence IgL chain; 6) represents the IGLV region; 7) represents the CDRL3 region; 8) Represents the IGLJ region. The P-Prim-H and P-prim-L components are depicted with overhang sequences w/x and y/z, respectively (see Figure 10).
   ii) Depicts the P-Amp-H (component 1J or 1J') and P-Amp-L (Component 1K or IK') reaction products generated with the above components, and each appended with the primer extensions encoding single-stranded DNA overhangs and Typel IIs binding sites (elements w, x, y and z). The action of the Type IIs enzyme on these extensions generates a P-Amp product intermediate with appended signel stranded DNA overhangs that permit directional cloning (see panel vii)
   iii) and iv) depict the products of Backbone Entry vector (Component 1L) when digested with the relevant Type IIS enzyme(s). Panel iii) represents the Backbone Entry Open conformation, with the negative selection and Type IIS recognition sequences excised as depicted in Panel iv). Product iii) is a reaction intermediate, whereas product iv) is a reaction by-product. These elements are labelled the same as in Figure 11.
   v) and vi) depict the products of the TaC-Only vector (Component 1M) when digested with the relevant Type IIS enzyme(s). Panel vi) represents the TaC-only open conformation, with the terminators and IGHC-IGLC elements excised as depicted in Panel vi). Product v) is a reaction intermediate, whereas product vi) is a reaction by-product. These elements are labelled the same as in Figure 11.
   vi) depicts the final reaction product, a TIVL' (Component 1N), representing a ligation between products ii), iii) and v) via the single-stranded DNA overhangs. This incorporates the IgH chain (sense) and IgL chain (antisense) contributed by the P-Amp products (product ii), having been obtained from a TGRS or DGRS. These IgH and IgL chains are completed with IGHC/IGLC sequences provided by the reaction intermediate excised from the TaC-only vector, along with terminator sequences (product v). These sequences are annealed into the Backbone Entry Open product (product iii) to complete a TIVL'. The paired boxes indicate the elements that represent the paired single-stranded DNA ligation points that permit directed ligation of the final TIVL'.
*Figure 13**. DIVL' recreation module plasmid architecture*
   A schematic representation of the DIVL recreation module plasmid architecture is depicted, representing **components 1O and 1P** of the DSS.
   The DIVL recreation module is used to convert P-Amp components derived from the sequence recovery module into a DIVL-H and DIV-L, termed DIVL-H' and DIVL-L', respectively. This DIVL 'recreation' process is used to cycle the AES with recovered and/or diversified sequences, which have been selected against tEp engagement in antibody engineering workflows.
   The DIVL recreation module plasmids are depicted as closed plasmids, with each lettered box representing a key element of the plasmid architecture. The principle of DIVL recreation process is analogous to that of the TIVL recreation depicted in Figures 11 and 12. The key difference of a DIVL system compared to a DIVL system, is that each IgH and IgL chain are carried on separate plasmids in a DIVL system, thus the IgH and IgL chain-related sequence elements are separated into two separate vectors.
   A DIVL recreation module comprises of two plasmids. The Heavy-Constant-C-Only (HC-Only, **Component 1O**), and the Light-Constant-Only (LC-Only; **Component 1P**). The HC-Only vector provides the vector backbone and genetic elements of the final DIVL-H', including the terminator sequence, along with invariant (constant) segments of the IGHC. Thus, a full DIVL-H encoding a full IgH chain is created by insertion of P-Amp-H (Component 1J or 1J', see Figure 10). The LC-Only vector provides the vector backbone and genetic elements of the final DIVL-L', including the optional untranslated 3' genetic elements (s), along with invariant (constant) segments of the IGLC. Thus, a full DIVL-L encoding a full IgL chain is created by insertion of P-Amp-L (Component 1K or 1K', see Figure 10).
   The HC-Only is depicted in the upper panel. a) Represents the 5' genetic element of the Backbone Entry plasmid, representing the 5' sequence responsible for targeted genomic integration to the DGRS-H contained within a D-eBPC. This 5' sequence is identical, and fulfills the same role as the 5' genetic element of the DIVL-H (Figure 5, element a). b) Represents the first overhang sequence, which encodes a unique sequence for a single-strand DNA overhang created by the action of the Type IIS enzyme binding to its recognition sequence immediately to the 3' of the overhang sequence. c) Represents a first Type IIS recognition sequence, relating to the binding of and action of the enzyme creating the overhang encoded in element b). d) Represents a negative selection marker used to negatively select the for parental Entry Backbone vector that is undigested by Type IIS enzyme action. e) Represents a second Type IIS recognition sequence, relating to the binding of and action of the enzyme creating the overhang encoded in element f). f) Represents the second overhang sequence, which encodes a unique sequence for a single-strand DNA overhang created by the action of the Type IIS enzyme binding to its recognition sequence immediately to the 5' of the overhang sequence. g) Represents a IGHC gene segment fragment that is not encoded by the P-AMP-H product. h) Represents optionally one or more 3' untranslated genetic elements. These genetic elements may include, but are not limited to, transcript stabilizing or destabilizing elements, and unique identifier sequences. i) Represents the 3' genetic element of the Backbone Entry plasmid, representing the 3' sequence responsible for targeted genomic integration to the DGRS-H contained within a D-eBPC. This 3' sequence is identical, and fulfills the same role as the 3' genetic element of the DIVL-H (Figure 5, element i). j) Represents a positive selection gene for HC-Only and DIVL-H' plasmid selection and propagation. k) Represents the plasmid origin of replication.
   The LC-Only is depicted in the upper panel. I) Represents the 5' genetic element of the Backbone Entry plasmid, representing the 5' sequence responsible for targeted genomic integration to the DGRS-L contained within a D-eBPC. This 5' sequence is identical, and fulfills the same role as the 5' genetic element of the DIVL-H (Figure 5, element l). m) Represents the first overhang sequence, which encodes a unique sequence for a single-strand DNA overhang created by the action of the Type IIS enzyme binding to its recognition sequence immediately to the 3' of the overhang sequence. n) Represents a first Type IIS recognition sequence, relating to the binding of and action of the enzyme creating the overhang encoded in element m). o) Represents a negative selection marker used to negatively select the for parental Entry Backbone vector that is undigested by Type IIS enzyme action. p) Represents a second Type IIS recognition sequence, relating to the binding of and action of the enzyme creating the overhang encoded in element q). q) Represents the second overhang sequence, which encodes a unique sequence for a single-strand DNA overhang created by the action of the Type IIS enzyme binding to its recognition sequence immediately to the 5' of the overhang sequence. r) Represents a IGLC gene segment fragment that is not encoded by the P-AMP-L product. s) Represents optionally one or more 3' untranslated genetic elements. These genetic elements may include, but are not limited to, transcript stabilizing or destabilizing elements, and unique identifier sequences. t) Represents the 3' genetic element of the Backbone Entry plasmid, representing the 3' sequence responsible for targeted genomic integration to the DGRS-L contained within a D-eBPC. This 3' sequence is identical, and fulfills the same role as the 3' genetic element of the DIVL-H (Figure 5, element t). u) Represents a positive selection gene for LC-Only and DIVL-L' plasmid selection and propagation. v) Represents the plasmid origin of replication.
*Figure 14**. DIVL'generation from P-Amp products*
   The process of DIVL' generation within the DIVL recreation module is depicted. Constructs and RT/PCR products are depicted as linear sequences, with each lettered or numbered box representing a key element of the sequence architecture, whereas primers are depicted as black arrows, plasmids are depicted in both open (digested) and circularized conformations with lettered and numbered boxes represent key sequence elements.
   i) Depicts R-Amp-H (component 1F) and R-Amp-L (component 1G) that may be obtained from either a TGRS or DGRS by amplification with RT primers (Components 1D and 1E, not depicted, see Figure 10). The P-Amp elements have been numbered whereas; 1) represents the leader sequence IgH chain; 2) represents the IGHV region; 3) represents the CDRH3 region; 4) Represents the IGHJ region; 5) represents the leader sequence IgL chain; 6) represents the IGLV region; 7) represents the CDRL3 region; 8) Represents the IGLJ region. The P-Prim-H and P-prim-L components are depicted with primer extensions encoding overhang sequences w/x and y/z, respectively (see Figure 10).
   ii) Depicts the P-Amp-H (component 1J or 1J') and P-Amp-L (Component 1K or 1K') reaction products generated with the above components, and each appended with the primer extensions encoding single-stranded DNA overhangs and Type IIS binding sites (elements w, x, y and z). The action of the Type IIS enzyme on these extensions generates a P-Amp product intermediate with appended signal stranded DNA overhangs that permit directional cloning (see panels vii and viii)
      The P-Amp elements have been numbered whereas; 1) represents the leader sequence IgH chain; 2) represents the IGHV region; 3) represents the CDRH3 region; 4) Represents the IGHJ region; 5) represents the leader sequence IgL chain; 6) represents the IGLV region; 7) represents the CDRL3 region; 8) Represents the IGLJ region. The P-Prim-H and P-prim-L components are depicted with overhang sequences w/x and y/z, respectively (see Figure 10).
   iii and v) depict the products of HC-Only (Component 1O), when digested with the relevant Type IIS enzyme(s). Panel iii) represents the HC-Only Open conformation (Componet 1O'), with the negative selection and Type IIS recognition sequences excised as depicted in Panel v). Product iii) is a reaction intermediate, whereas product v) is a reaction by-product. These elements are labelled the same as in Figure 13.
   iv) and vi) depict the products of LC-Only (Component 1P), when digested with the relevant Type IIS enzyme(s). Panel iv) represents the LC-Only Open conformation (Componet 1P'), with the negative selection and Type IIS recognition sequences excised as depicted in panel vi). Product iv) is a reaction intermediate, whereas product vi) is a reaction by-product. These elements are labelled the same as in Figure 13.
   vii) depicts the final reaction product, a DIVL-H' (Component 1Q), representing a ligation between products ii) and iii) via the single-stranded DNA overhangs. This incorporates the IgH chain contributed by the P-Amp products (panel ii), having been obtained from a TGRS or DGRS. These IgH chain sequences are completed with IGHC sequence provided within the HC-Only Vector. The P-Amp-H sequences are annealed into the HC-Only Open conformation (product iii) to complete a DIVL-H'. The paired boxes indicate the elements that represent the paired single-stranded DNA ligation points that permit directed ligation of the final DIVL-H'.
   viii) depicts the final reaction product, a DIVL-H' (Component 1R), representing a ligation between products ii) and iv) via the single-stranded DNA overhangs. This incorporates the IgL chain contributed by the P-Amp products (panel ii), having been obtained from a TGRS or DGRS. These IgL chain sequences are completed with IGLC sequence provided within the LC-Only Vector. The P-Amp-L sequences are annealed into the LC-Only Open conformation (product iv) to complete a DIVL-L'. The paired boxes indicate the elements that represent the paired single-stranded DNA ligation points that permit directed ligation of the final DIVL-L'.
*Figure 15* *Description of the T-eBPC and operation*
   The upper panel depicts a schematic representation a T-eBPC comprising five components. The first component **2A** is the **T-eBPC** line itself with all required engineered features of that cell. The **T-eBPC** contains two further components, one of which is component **2B,** representing the TGRS. The second component optionally included in the **T-eBPC,** is a synthetic reporter construct that is induced upon BCR ligation with tEp, **2H.** Two additional independent components **1A** and **1N** represent a TIVL and TIVL', respectively. Each of the tandem integration vector libraries is matched with the TGRS, to enable site-directed integration of the IgH and IgL chain sequences to the genome of 2A (**Step1**).
   Integration of the TIVL- or TIVL'- encoded IgH and IgL sequences into the TGRS coverts this TGRS (2B) to an integrated state (2B'). This results in the conversion of an T-eBPC (2A), into a T-eBPC-b library (2C); depicted in the middle panel. Each member of the T-eBPC-b library expresses a BCR at the cell surface, which is encoded by the IgH and IgL sequences delivered by the TIVL or TIVL' to the TGRS.
   In step 2, a selection of the T-eBPC-b library is conducted by contacting the cell library with a tEp. On the basis of tEp binding (depicted in bottom panel) a sub-population of T-eBPC-b library (2C) may be isolated as a selected T-eBPC-b library (2C'). Such a selection may equally be conducted on the basis of the isolated population being unable to bind tEp in negative selection procedures.
*Figure 16* *Description of the D-eBPC and operation*
   The upper panel depicts a schematic representation a D-eBPC comprising eight components. The first component **2D** is the **D-eBPC** line itself with all required engineered features of that cell. The **D-eBPC** contains three further components, two of which are components **2E and 2F,** representing the DGRS-H and DGRS-L. The third component optionally included in the **D-eBPC,** is a synthetic reporter construct that is induced upon BCR ligation with tEp, **2H.** Four additional independent components **1B/1Q and 1C/1R,** represent a DIVL-H/DIVL-H' and DIVL-L/DIVL-L', respectively.
   The DIVL-H/DIVL-H' vector libraries are matched with the DGRS-H, whereas the DIVL-L/DIVL-L' vector libraries are matched with the DGRS-L, to enable site-directed integration of the IgH and IgL chain sequences to the genome of 2D (**Step1**).
   Integration of the DIVL encoded IgH and IgL sequences into the DGRS coverts each DGRS-H and DGRS-L (2E and 2F, respectively) to an integrated state (2E' and 2F, respectively). This results in the conversion of a D-eBPC (2D), into a D-eBPC-b library (2G); depicted in the middle panel. Each member of the D-eBPC-b library expresses a BCR at the cell surface, which is encoded by the IgH and IgL sequences delivered by the DIVL to the DGRS.
   In step 2, a selection of the D-eBPC-b library is conducted by contacting the cell library with a tEp. On the basis of tEp binding (depicted in bottom panel) a sub-population of D-eBPC-b library (2G) may be isolated as a selected T-eBPC-b library (2G'). Such a selection may equally be conducted on the basis of the isolated population being unable to bind tEp in negative selection procedures.
*Figure 17* *Architecture of DGRS exchangers*
   A schematic representation of the DGRS-H and DGRS-L exchanger vector architectures is depicted, representing **components 2Y and 2Z** of the DSS, respectively. Each DGRS exchangers are vectors that carry a reporter gene bounded by 5' and 3' genetic elements, which is used to regenerate the integrated state of DGRS, back to the open state, within a D-eBPC. Thus, a D-eBPC-b contains two DGRS sites, a DGRS-H and DGRS-L, which are paired with the DGRS-H exchanger and DIVL-L exchanger, respectively.
   The DIVL plasmids are depicted as closed plasmids, with each lettered box representing a key element of the plasmid architecture. The elements of each DGRS exchanger essentially reflects the composition of a DGRS, encoding a reporter gene flanked by 5' and 3' genetic elements that target the DGRS.
   a) Represents the 5' genetic element of the DGRS-H Exchanger **(Component 2Y),** representing the 5' sequence responsible for targeted genomic integration of the reporter gene (element c) to the integrated DGRS-H. Thus, sequence element a) of the DGRS-H Exchanger is matched with the equivalent 5' genetic element of the DIVL-H (Figure 5, element a) and DGRS (Figure 8 element a). b) Represents a Kozak sequence for efficient translational initiation of the reporter gene that constitutively marks the presence of an intact DGRS-H within a D-eBPC, and which is different from the equivalent reporter encoded by the DGRS-L Exchanger (element h). c) Represents the reporter gene of the DGRS-H, gain of which is used to indicate the integration of sequences provide by the DGRS-H Exchanger d) Represents optionally one or more 3' untranslated genetic elements. These genetic elements may include, but are not limited to, transcript stabilizing or destabilizing elements, and unique identifier sequences. e) Represents the 3' genetic element of the DGRS-H exchanger, representing the 3' sequence responsible for targeted genomic integration to the reporter gene (element c). Thus sequence element e) of the DGRS-H Exchanger is matched with the equivalent 3' genetic element of the DIVL-H (Figure 5, element i) and DGRS-H (Figure 8, element e) . k) Represents a positive selection gene for DIVL-H plasmid selection propagation DIVL-H. I) Represents the plasmid origin of replication.
   f) Represents the 5' genetic element of the DGRS-L Exchanger **(Component 2Z),** representing the 5' sequence responsible for targeted genomic integration of the reporter gene (element h) to the integrated DGRS-L. Thus sequence element f) of the DGRS-L Exchanger is matched with the equivalent 5' genetic element of the DIVL-L (Figure 5, element I) and DGRS (Figure 8 element f). g) Represents a Kozak sequence for efficient translational initiation of the reporter gene that constitutively marks the presence of an intact DGRS-L within a D-eBPC, and which is different from the equivalent reporter encoded by the DGRS-H Exchanger (element c). h) Represents the reporter gene of the DGRS-L, gain of which is used to indicate the integration of sequences provide by the DGRS-L Exchanger i) Represents optionally one or more 3' untranslated genetic elements. These genetic elements may include, but are not limited to, transcript stabilizing or destabilizing elements, and unique identifier sequences. j) Represents the 3' genetic element of the DGRS-L exchanger, representing the 3' sequence responsible for targeted genomic integration to the reporter gene (element h). Thus sequence element j) of the DGRS-L Exchanger is matched with the equivalent 3' genetic element of the DIVL-L (Figure 5, element t) and DGRS-L (Figure 8, element j). m) Represents a positive selection gene for DIVL-H plasmid selection propagation DIVL-H. n) Represents the plasmid origin of replication.
*Figure 18* *Compilation of different analyte BCRsp presenting eBPC forms*
   Depicted are schematic representations of operation of the TSS (a) and DSS (b) to generated different analyte eBPC libraries.
   a) The operation of the TSS to prepare analyte **T-eBPC** populations begins with the **T-eBPC** and uses **TIVL/TIVL'** to create cells expressing analyte **BCR.** A **T-eBPC** presenting **BCR** is termed **T-eBPC-b,** and in the case of a T-eBPC may only be created by introduction of **IgH and IgL** chain sequences in a single step via TIVL/TIVL' **(process i).**
   b)The operation of the DSS to prepare analyte **D-eBPC** populations begins with the **D-eBPC** and uses **DIVL-H/DIVL-H'** and/or **DIVL-L/DIVL-L'** to create cells expressing analyte **BCR,** or only one of IgH or IgL. A **D-eBPC** presenting **BCR** is termed **D-eBPC-b,** and in the case of a D-eBPC may be created by introduction of **IgH and IgL** chain sequences in a single step via **DIVL-H/DIVL-H'** and/or **DIVL-L/DIVL-L' (process i).** Alternatively, a two-step process may be used to generate a D-eBPC-x intermediate **(process ii)** by first integrating either IgH or IgL chain sequences via **DIVL-H/DIVL-H'** or **DIVL-L/DIVL-L',** respectively. Subsequently a D-eBPC-b may be generated by the integration of the reciprocal IgH or IgL chain sequence **(process iii).** A D-eBPC-b may also be reverted to a D-eBPC-x by use of an integration vector to replace either the encoded IgH or IgL sequence **(process iv).**
*Figure 19**. Generic AES workflow*
   Depicts the overall generic workflow of the AES, both DSS and TSS. The left-hand panel represents AOCP processes, and the right-hand panel the eBPCP processes. The tEp preparation is an input that operates outside the system, and which is used to select eBPC-b libraries. i) The preparation of an input vector library is represented by a TIVL or pair of DIVL-H/DIVL-L, and reflects the desired IgH/IgL chain diversity. ii) The T-eBPC and D-eBPC lines that act as receptacles for the TIVL and DIVL, respectively. iii) Preparation of an eBPC-b library is a result of integrating the TIVL or DIVL to the T-eBPC or D-eBPC, respectively. This creates a cell library that expresses a set of BCRs on the cell surface, which are encoded by the TIVL or DIVL. iv) Preparation of a tEp may occur in a variety of manners, depending on the selection methods to be employed, and may include negative selection tEp entities. v) A subset of eBPC-b is selected on the basis desirable engagement of tEp with BCR expressed on the eBPC-b cell surface. vi) IgH and/or IgL sequences are recovered from selected cells. vii) Recovered high-fidelity sequences may represent terminal outputs of the AES, representing antibodies or BCR with desired tEp engagement properties. viii) Further selection rounds on selected IgH/IgL chain are facilitated by inserting high-fidelity and/or low-fidelity recovered sequences to recreation of the TIVL and/or DIVL, referred to as TIVL' and DIVL', to recreate input vector libraries with or without sequence diversification. ix) Reapplication of TIVL' and/or DIVL' to AES workflow cycle.

### List of abbreviations

**AES:** Antibody engineering system.
**AOCP:** Antibody ORF Construction Platform.
**BCR:** B-cell receptor.
**C-segment:** Constant segment.
**CAR-T:** Chimeric antigen receptor T-cell.
**CDR:** Complementarity determining region.
**CDRH3:** Complementarity determining region heavy 3.
**CDRL3:** Complementarity determining region light 3.
**CHO:** Chinese Hamster Ovarian cell.
**D-eBPC:** Dual engineered BCR-presenting cell.
**D-eBPC-b:** Library of BCR-presenting D-eBPC.
**D-eTPC-x:** D-eBP expressing one of the antibody chains.
**DGRS:** Dual genomic receiver sites.
**DGRS-H:** Dual genomic receiver sites heavy.
**DGRS-L:** Dual genomic receiver sites light.
**DIVIL:** Dual integration vector library.
**DIVIL':** DIVL recreated.
**DIVL-H:** Dual integration library heavy.
**DIVL-L:** Dual integration library light.
**DNA:** Desoxyribonucleic acid.
**D-segment:** Diversity segment.
**DSS:** Dual site subsystem.
**eBPC:** Engineered BCR-presenting cell.
**eBPC-a.** Engineered BCR-presenting cells expressing a soluble antibody.
**eBPC-b:** Engineered BCR-presenting cells expressing a BCR on the cell surface.
**eBPCP:** Engineered BCR-presenting cell platform.
**FACS:** Fluorescence-activated cell sorting.
**GRS:** Genomic receiver site.
**HC-only:** Heavy-constant-only vector.
**HFP-Amp:** High-Fidelity PCR amplicon.
**Ig:** Immunoglobulin.
**IgH:** Immunoglobulin heavy.
**IgHC:** Immuglobulin heavy constant.
**IgHV:** Immunoglobulin heavy variable.
**IgL:** Immunoglobulin light:
**IgLC:** Immunoglobulin light constant.
**IgLV:** Immunoglobulin light variable.
**IgSF:** Immunoglobulin superfamily domain.
**Inr:** Initiation element.
**ITAM:** Immunoreceptor tyrosin-based activation motif.
**IP:** Input part.
**IVL:** Integration vector library:
**J-segment:** Joining segment.
**LC-only:** Light-constant-only vector.
**LFP-Amp:** Low-Fidelity PCR amplicon.
**MACS:** Magnetic activated cell sorting.
**MP:** Manufacturing part.
**OP:** Output part.
**ORF:** Open reading frame.
**P-Amp:** PCR amplicon.
**P-Prim:** PCR primer.
**PCR:** Polymerase chain reaction.
**R-Prim:** RT primer component.
**R-Amp:** RT amplicon.
**RT:** Reverse transcription.
**RMCE:** Recombinase-mediated cassette exchange.
**TaC-only:** Tandem constant-only vector.
**T-eBPC:** Tandem genomic receiver site containing eBPC.
**T-eBPC-b:** Library of BCR-presenting T-eBPC. created by combination with TIVL.
**tEp:** Target epitope
**TGRS:** Tandem genomic receiver site.
**TIVL:** Tandem Integration Vector Library.
**TIVL':** TIVL recreated.
**TSS:** Tandem site subsystem.
**UTR:** Untranslated region.
**V-segment:** Variable segment.

### Definitions

**AES:** Antibody engineering system. The name for the overall system.

**Amplicon:** A piece of DNA or RNA that is the source and/or product of artificial amplification using various methods including PCR.

**Amplifier-Reporter:** Synthetic or native promoter bound by a transcription factor from an amplifier/suppresser unit driving the expression of a reporter.

**Antibody:** Affinity molecule that is expressed by specialized cells of the immune system called B-cells and that contains of two chains. B-cells express a very large and very diverse repertoire of antibodies that do generally not bind self-proteins but can bind and neutralize pathogens or toxins that would threaten the host. Natural or artificially engineered antibodies are often used as affinity reagents.

**Antibody repertoire:** All antibodies produced by any given individual.

**AOCP:** Antibody ORF Construction Platform. The overall description of the vector, primer and product sets required for all ORF construction and manipulation .

**Backbone Entry:** Original assembly backbone vector used to remake diversified H/L vector library from P-Amp

**B-cell:** Specialized cell of the immune system. Each B-cell expresses one specific BCR on its surface and may secrete it as a soluble antibody upon stimulation in the context of an infection.

**BCR:** B-cell receptor. Membrane bound version of an antibody in context with signalling molecules CD79a/b.

**CAR-T:** Chimeric antigen receptor T-cell. Engineered T-cell expressing a synthetic variant of a T-cell receptor or related affinity reagent.

**CDR:** Complementarity determining region: Short and highly variable region in the variable segments of antibodies that determines target binding properties of the antibody. Each chain has three CDR.

**CDRH3:** Complementarity determining region heavy 3. Third CDR of the heavy chain. The most variable CDR of the heavy chain

**CDRL3:** Complementarity determining region light 3. Third CDR of the light chain. The most variable CDR of the light chain.

**CHO:** Chinese Hamster Ovarian cell. Human cell line commonly used for the production of antibodies.

**Contextual epitope:** Epitope that is formed by and/or depended on two or more different proteins

**C-segment:** Constant segment. One of the gene segments that is used to assemble an antibody. There are five different classes of c-segments (A, D, E, G, M). with a number of subclasses. These do not affect the antigen-binding site but have differing roles in immune defence.

**CD79a / CD79b:** Surface proteins expressed by B-cells that form the BCR together with the membrane-bound form of the antibody. Contain ITAM for signalling.

**D-eBPC:** Dual engineered BCR-presenting cell. eBPC containing DGRS for insertion of antibody chains from a DIVL

**D-eBPC-b:** Library of BCR-presenting D-eBPC created by combination of D-eBPC with a DIVIL.

**D-eTPC-x:** D-eBPC having integrated and expressing either light or heavy chain.

**Donor vector:** A genetic based vector for delivery of genetic material.

**Driver-Activator/Repressor.** Synthetic or native promotor driving the expression of a synthetic or native transcription factor that in turn drives or supresses gene expression of a secondary promoter.

**DGRS:** Dual genomic receiver sites. Dual system GRS contained within an D-eBPC.

**DGRS-H:** Dual genomic receiver sites heavy. Dual system GRS for integration of the heavy chain.

**DGRS-L:** Dual genomic receiver sites light. Dual system GRS for integration of the light chain.

**DGRS-H Exchanger:** Dual genomic receiver sites heavy exchanger. Donor vector for the replacement of a heavy chain integrated into DGRS-H with a reporter or placeholder.

**DGRS-L Exchanger:** Dual genomic receiver sites light exchanger. Donor vector for the replacement of a light chain integrated into DGRS-L with a reporter or placeholder.

**DIVIL:** Dual integration vector library. The dual IVL preassembled as starting material for insertion to D-eBPC.

**DIVIL':** The DIVL recreated from P-Amp for recreation of a D-eBPC-b Library **DIVL-H:** Dual integration library heavy. Dual heavy chain IVL for insertion to D-eBPC.

**DIVL-L:** Dual integration library light. Dual light chain IVL for insertion to D-eBPC.

**DNA:** Desoxyribonucleic acid. Chemical name of the molecule that forms genetic material encoding genes and proteins.

**D-segment:** Diversity segment. One of the gene segments that is used to assemble an antibody. Each individual has a large number of different variations of these regions making it possible for each individual to have a very large variety of antibodies.

**DSS:** Dual site subsystem, describes all AES modules that treat H- and L- chain ORFs in separate paired constructs and genomic sites.

**eBPC:** Engineered BCR-presenting cell. Engineered cell with single instances of TGRS or DGRS for the integration of heavy and light antibody chain and antibody expression.

**eBPC-a.** Engineered BCR-presenting cells expressing a soluble antibody.

**eBPC-b:** Engineered BCR-presenting cells expressing a BCR on the cell surface.

**eBPCP:** Engineered BCR-presenting cell platform. Incorporates both tandem and dual integration site versions.

**Endogenous:** Substance that originated from within a cell

**Engineered Cell:** A cell whereby the genome has been engineered through genetic modification.

**Epitope:** Region on a antibody target that is bound by the antibody.

**FACS/Flow Cytometry:** Fluorescence-activated cell sorting. Analytical technique by which individual cells can be analysed for the expression of specific cell surface and intracellular markers. A variation of that technique, cell sorting, allows cells that carry a defined set of markers to be retrieved for further analysis.

**Fluorescent (protein) marker:** Molecule that has specific extinction and emission characteristics and can be detected by Microscopy, FACS and related techniques.

**Germline:** Genetic material as encoded in the inherited genome.

**GRS:** Genomic receiver site. A site within the genome for targeted integration of donor genetic material encoded within a genetic donor vector.

**HC-only:** Heavy-constant-only vector to remake faithful or diversified DIVL-H from P-Amp.

**High fidelity:** Mode of operating PCR, which minimizes the introductions of errors during sequence amplification.

**HFP-Amp:** High-Fidelity PCR amplicons.

**Ig:** Immunoglobulin. Alternative name for antibody.

**IgH:** Immunoglobulin heavy. Antibody heavy chain.

**IgHC:** Immuglobulin heavy constant. Constant segment of the antibody heavy chain.

**IgHV:** Immunoglobulin heavy variable. Variable segment of the antibody heavy chain.

**IgL:** Immunoglobulin light. Antibody kappa or lambda light chain.

**IgLC:** Immunoglobulin light constant. Constant segment of the antibody light chain.

**IgLV:** Immunoglobulin light variable. Variable segment of the antibody light chain.

**IgSF:** Immunoglobulin superfamily domain. Three-dimensional structure comprised of 70-100 amino acids and shared among a large number of cell surface and soluble proteins.

**IP:** Input part. Input into the AES. DIVL, DIVL', TIVL, TIVL', D-eBPC, T-eBPC.

**Inr:** Initiation element. Essential part of a promotor.

**Integration:** The physical ligation of a DNA sequence into a chromosome of a cell **ITAM:** Immunoreceptor tyrosine-based activation motif. Short protein sequence associated with receptors, which generates a stimulatory signal following ligand engagement.

**IVL:** Integration vector library: Vector library representing desired antibody chain diversity for integration into e-BPC via TGRS/DGRS.

**J-segment:** Joining segment. One of the gene segments that is used to assemble an antibody. Each individual has a large number of different variations of these regions making it possible for each individual to have a very large variety of antibodies.

**Kozak Sequence:** Short sequence required for the efficient initiation of translation **LC-only:** Light-constant-only vector to remake faithful or diversified DIVL-L from P-Amp

**Low fidelity:** Mode of operating PCR, which either tolerates or specifically favours the introduction of errors (error-prone PCR) during sequence amplification.

**LFP-Amp:** Low-Fidelity PCR amplicons

**MACS:** Magnetic activated cell sorting: Cellular isolation technique in which cells are labelled with affinity molecules that contain magnetic particles for separation using magnetic fields.

**Matched:** When two components encode genetic elements that direct and restrict the interaction between the complemented components.

**Monoclone cell line:** A defined group of cells produced from a single ancestral cell by repeated cellular replication.

**Native:** A entity that is naturally occurring in the cell

**ORF:** Open reading frame. Stretch of genetic material that encodes a translation frame for synthesis of a protein by the ribosome.

**Ortholog:** Gene in a related species that shares a common ancestor.

**P-Amp:** PCR amplicon.

**Plasmid:** circular DNA structure that contains genetic material as well as selection markers and replicates independently of the chromosomal DNA. Also referred to as Vector.

**P-Prim:** PCR primer.

**PCR:** Polymerase chain reaction in which a specific target DNA molecule is exponentially amplified.

**Primary Input:** Initial input into the AES. DIVL, TIVL, D-eBPC, T-eBPC.

**Primer:** Short DNA sequence that allows specific recognition of a target DNA sequence for example during a PCR.

**Promoter:** Regulatory DNA element for the controlled initiation of gene expression.

**R-Prim:** RT primer component.

**R-Amp:** RT amplicon.

**RT:** Reverse transcription. Process by which RNA is enzymatically transcribed into DNA.

**Recombinase:** Protein that catalyses RMCE.

**Reporter:** Protein or chemical compound that is produced by a cell in response to a defined signal.

**RMCE:** Recombinase-mediated cassette exchange. Exchange of genetic material at a GRS catalysed by a recombinase.

**Selected D-eBPC-b:** Selected D-eBPC-b single cell or library based on tEp binding or stimulation.

**Selected T-eBPC-b:** Selected T-eBPC-b single cell or library based on tEp binding or stimulation

**Synthetic:** An entity that is artificially generated.

**TaC-only:** Tandem constant-only vector to remake faithful or diversified TIVL from P-Amp.

**TATA-box:** Essential part of a promotor.

**T-cell:** T lymphocyte. White blood cell that expresses a T-cell receptor on its surface. Selected by the immune system to not react with the own body but have the potential to recognize infections and malignancies as well as reject grafts from most members of the same species.

**T-eBPC:** Tandem genomic receiver site containing eBPC.

**T-eBPC-b:** Library of BCR-presenting T-eBPC created by combination with TIVL.

tEp: Target epitope

**Terminal Outputs:** HF-Amp-H and HF-Amp-L with specific tEp binding properties

**Terminator/Signal terminator:** A DNA sequence that are recognized by protein factors that are associated with the RNA polymerase II and which trigger the termination process of transcription. It also encodes the poly-A signal

### TGRS: Tandem genomic receiver site. Tandem system GRS contained within an T-eBPC

**TIVL:** Tandem Integration Vector Library. The H/L bidirectional IVL representing desired antibody chain diversity preassembled as starting material for insertion to T-eBPC.

**TIVL':** The TIVL recreated from P-Amp for recreation of a T-eBPC-b Library **TSS:** Tandem site subsystem, describes all AED modules that treat H- and L- chain ORFs in a single construct or genomic site.

**Transcription factor:** Protein that binds to a promoter and regulates gene expression.

**UTR:** Untranslated region. Part of a gene transcript that is not encoding a protein sequence but can contain regulatory elements.

**Vector:** circular DNA structure that contains genetic material as well as selection markers and replicates independently of the chromosomal DNA. Also referred to as Plasmid.

**V-segment:** Variable segment.. One of the gene segments that is used to assemble an antibody. Each individual has a large number of different variations of these regions making it possible for each individual to have a very large variety of antibodies.

**Embodiments of the invention are given in the following items**
1. A tandem-manufacturing system for generating antibody sequences, comprising
   a. an input part (IP), comprising:
      i. IP Module 1, which is a tandem integration vector library (TIVL), each vector comprises a pair of IgH and IgL sequences each pair encoding together an antibody/BCR;
      ii. IP Module 2, which is a tandem-engineered BCR presenting cell (T-eBPC) containing a Tandem Genomic Receiver Site (TGRS) and optionally a synthetic reporter construct to report BCR engagement;
   b. a first manufacturing part (MP1), comprising
      a. MP1 Module 1, which is a module for combining IP Module 1 and IP module 2 whereby the sequences of a vector from IP Module 1 is inserted into the Tandem Genomic Receiver Site (TGRS) of a T-eBPC of IP Module 2, to obtain T-eBPC cells presenting a BCR (T-eBPC-b), wherein the BCR is encoded by those IgH and IgL sequences transferred from the TIVL of IP Module 1 to the TGRS of IP Module 2;
      b. MP1 Module 2, which is a 2-step sequence recovery module for recovering said IgH and/or IgL sequences of the T-eBPC-b, said sequence recovery module comprises reverse transcription primers (R-Prim) for deriving reverse transcriptase amplicons (R-Amp) from IgH and/or IgL chain sequences encoded in the TGRS of the T-eBPC-b in a first sequence recovery step, and further comprising polymerase chain reaction primers (P-Prim) to amplify the R-Amp products in second sequence recovery step; wherein said second sequence recovery step comprises either high-fidelity or low-fidelity polymerase chain reaction components;
   c. an output part (OP), which contains the product resulting from MP1 Module 2.
2. A tandem-manufacturing system according to item 1, wherein the output part (OP) is OP Module 1 or OP Module 2, wherein OP Module 1 comprises a high-fidelity polymerase chain reaction amplicon (HFP-Amp), and wherein OP Module 2 comprises a low-fidelity polymerase chain reaction amplicon (LFP-Amp).
3. A tandem-manufacturing system according to item 1 or 2 further comprising MP2 Module 1, which is a second manufacturing part containing a Backbone entry vector and a tandem Constant only (TaC-only) vector for combining with the amplicons of the OP Module(s).
4. A method for human antibody engineering, the method comprises using a tandem-manufacturing system as defined in items 1-3, wherein combination of inputs from IP Module 1 and IP Module 2, which is integration of IgH and IgL chain sequences encoded by vectors of said TIVL into the TGRS, results in the creation of a library of T-eBPC-b expressing BCR at the cell surface.
5. A method according to item 4, wherein the MP1 Module 1 of the tandem-manufacturing system provides a library of one or more T-eBPC-b expressing single discrete pairs of IgH and IgL chains.
6. A method according to item 4 or 5, wherein MP1 Module 2 of the tandem-manufacturing system provides a high-fidelity polymerase chain reaction amplicon (HFP-Amp) for IgH and IgL chain sequences from selected T-eBPC-b.
7. A method according to any of items 4-6, wherein the low-fidelity polymerase chain reaction amplicons (LFP-Amps) derived from MP1 Module 2 are used in MP2 Module 1 of the tandem-manufacturing system for the re-creation of vectors of TIVL to obtain a TIVL with introduced IgH/ IgL sequence diversity (TIVL').
8. A method according to item 7, wherein one or more vectors of the TIVL' is used as IP Module 1.
9. A method for human antibody engineering, the method comprises
   i) reacting a vector of a TIVL, the vector comprises a pair of IgH and IgL sequences each pair encoding together an antibody/BCR with a tandem-engineered BCR presenting cell (T-eBPC) containing a Tandem Genomic Receiver Site (TGRS) and optionally a synthetic reporter construct to report BCR engagement to obtain a T-eBPC-b;
   ii) contacting the T-eBPC-b with a target epitope (tEp) to select one or more T-eBPC-b on the basis of tEp engagement;
   iii) subjecting in a first sequence recovery step (MP1 Module 2), the one or more T-eBPC-b selected to reverse transcription primers (R-Prim), for deriving reverse transcriptase amplicons (R-Amp) from IgH and/or IgL chain sequences encoded in the TGRS of the T-eBPC-b;
   iv) subjecting in a second sequence recovery step (MP1 Module 2), the R-Amp to polymerase chain reaction primers (P-Prim) to amplify the R-Amp products, wherein said second recovery step comprises either high-fidelity or low-fidelity polymerase chain reaction components to obtain HFP-Amps and LFP-Amps, respectively.
10. A method according to item 9 further comprising combining amplicons from step iv) with a Backbone entry vector and a tandem Constant only (TaC-only) vector.
11. A method according to item 9, wherein said LFP-Amps or HFP-Amps are used for re-creation of a TIVL with IgH/ IgL sequences amplified from selected T-eBPC-b cells, in order to recreate TIVL vectors encoding faithfully those selected sequences (using HFP-Amps) or with introduced sequence diversity (using LFP-Amps), wherein the resulting vector library is termed a TIVL'.
12. A method according to item 11, wherein one or more vectors of TIVL' is used as starting material in step i) of item 9.
13. A dual-manufacturing system for generating antibody sequences, comprising
   a. an input part (IP), comprising:
      i. IP Module 3, which is a dual integration vector library (DIVL), each vector comprises either a IgH (DIVL-H) or a IgL (DIVL-L) sequence, wherein the IgH (DIVL-H) and the IgL (DIVL-L) sequences together encode an antibody/BCR;
      ii. IP Module 4, which is a dual-engineered BCR presenting cell (D-eBPC) containing a pair of Dual Genomic Integration Sites (DGRS), which is Heavy (DGRS-H) and Light (DGRS-L) and optionally a synthetic reporter construct to report BCR engagement.
   b. a first manufacturing part (MP1), comprising
      i. MP1 Module 3, which is a module for combining IP Module 3 and IP Module 4, whereby the sequences of a DIVL-H and DIVL-L vector from IP Module 3 are inserted into the DGRS-H and DGRS-L, respectively, of a D-eBPC of IP Module 4 to obtain D-eBPC cells presenting a BCR (D-eBPC-b), wherein the BCR is encoded by those IgH and IgL sequences transferred from the DIVL-H and DIVL-L of IP Module 3 to the DGRS of IP Module 4;
      ii. MP1 Module 2, which is a 2-step sequence recovery module for recovering said IgH and/or IgL sequences of said D-eBPC-b, said sequence recovery module comprises reverse transcription primers (R-Prim) for deriving reverse transcriptase amplicons (R-Amp) from IgH and IgL chain sequences encoded in the DGRS-H and DGRS-L of the D-eBPC-b in a first sequence recovery step, and further comprising polymerase chain reaction primers (P-Prim) to amplify the R-Amp products in a second sequence recovery step, wherein said second sequence recovery step comprises either high-fidelity or low-fidelity polymerase chain reaction components;
   c. an output part (OP), which contains the products resulting from MP 1 Module 2.
14. A dual-manufacturing system according to item 13, wherein the output part (OP) is OP Module 1 or OP Module 2, wherein OP Module 1 comprises a high-fidelity polymerase chain reaction amplicon (HFP-Amp), and wherein OP Module 2 comprises a low-fidelity polymerase chain reaction amplicon (LFP-Amp).
15. A dual-manufacturing system according to item 13 or 14 further comprising MP2 Module 2, which is a second manufacturing part containing either a Heavy Constant-Only (HC-only), or a Light Constant-only (LC-only) vector, for combining with the amplicons of the OP Modules.
16. A method for human antibody engineering, the method comprises using a dual-manufacturing system as defined in any of item 13-15, wherein combination of input from IP Module 3 and IP Module 4, which is integration of IgH and IgL chain sequences encoded by vectors of said DIVL-H and DIVL-L, respectively, results in the creation of a library of D-eBPC-b expressing BCR at the cell surface.
17. A method according to item 16, wherein the MP1 Module 3 of the dual-manufacturing system provides a library of one or more D-eBPC-b expressing single discrete pairs of IgH and IgL chains.
18. A method according to items 16 or 17, wherein MP1 Module 2 of the dual-manufacturing system provides a high-fidelity polymerase chain reaction amplicon (HFP-Amp) for IgH and IgL chain sequences from selected D-eBPC-b.
19. A method according to any of items 16-18, wherein the low-fidelity polymerase chain reaction amplicons (LFP-Amps) derived from MP1 Module 2 is used in MP2 Module 2 of the dual-manufacturing system for the re-creation of vectors of DIVL-H or DIVL-L to obtain a DIVL-H or DIVL-L with introduced IgH/ IgL sequence diversity (DIVL-H' or DIVL-L').
20. A method according to item 19, wherein one or more vectors of the DIVL' is used as IP Module 3.
21. A method for human antibody engineering, the method comprises
   i) reacting a) a vector of DIVL-H, the vector comprising a IgH sequence, and b) a vector of DIVL-L, the vector comprising a IgL sequence, the sequences encoding together an antibody/BCR, with c) a dual-engineered BCR presenting cell (D-eBPC) containing a Dual Genomic Receiver Site (DGRS) and optionally a synthetic reporter construct to report BCR engagement to obtain a D-eBPC-b;
   ii) contacting the D-eBPC-b with a target epitope (tEp) to select one or more D-eBPC-b on the basis of tEp engagement;
   iii) subjecting in a first sequence recovery step (MP1 Module 2), the one or more D-eBPC-b selected to reverse transcription primers (R-Prim), for deriving reverse transcriptase amplicons (R-Amp) from IgH and/or IgL chain sequences encoded in the DGRS of the D-eBPC-b;
   iv) subjecting in a second sequence recovery step (MP1 Module 2), the R-Amp to polymerase chain reaction primers (P-Prim) to amplify the R-Amp products, wherein said second sequence recovery step comprises either high-fidelity or low-fidelity polymerase chain reaction components to obtain HFP-Amps and LFP-Amps, respectively.
22. A method according to item 21 further comprising combining at least one amplicon from step iv) with at least one of a Heavy Constant-Only (HC-only) and a Light Constant-only (LC-only) vector.
23. A method according to item 21, wherein said LFP-Amps or HFP-Amps are used for re-creation of at least one DIVL with a IgH or a IgL sequence amplified from selected D-eBPC-b cells, in order to recreate DIVL vectors encoding faithfully those selected sequences (using HFP-Amps) or with introduced sequence diversity (using LFP-Amps), wherein the resulting vector library is termed a DIVL-H' or DIVL-L'.
24. A method according to item 23, wherein one or more vectors of DIVL-H' or DIVL-L' is used as starting material in step i) of item 21.
25 A combined tandem-dual manufacturing system comprising a tandem-manufacturing system as defined in any one of items 1-3 and a dual-manufacturing system as defined in any one of items 13-15, wherein the second manufacturing part, MP2 Module 2, defined in item 15 is included.
26. A method for human antibody engineering, the method comprises using the combined tandem-dual manufacturing system according to item 25.
27. A method according to item 26, wherein said LFP-Amps or HFP-Amps are used for re-creation of at least one TIVL with IgH and IgL sequence amplified from selected D-eBPC-b cells, in order to recreate TIVL vectors encoding faithfully those selected sequences (using HFP-Amps) or with introduced sequence diversity (using LFP-Amps), wherein the resulting vector library is termed a TIVL'.
28. A method according to item 26, wherein said LFP-Amps or HFP-Amps are used for re-creation of at least one DIVL with IgH and/ or IgL sequence amplified from selected T-eBPC-b cells, in order to recreate DIVL vectors encoding faithfully those selected sequences (using HFP-Amps) or with introduced sequence diversity (using LFP-Amps), wherein the resulting vector library is termed a DIVL'.
29. A method according to items 27, wherein one or more vectors of TIVL' is used as starting material in step i) of item 9.
30. A method according to items 28, wherein one or more vectors of TIVL' is used as starting material in step i) of item 13.
31. A manufacturing system as defined in any one of items 1-3, 13-15 or 25 to provide one or more antibodies against tEp.

## Claims

1. A tandem-manufacturing system for generating antibody sequences, comprising
c. an input part (IP), comprising:
iii. IP Module 1, which is a tandem integration vector library (TIVL), each vector comprises a pair of IgH and IgL sequences each pair encoding together an antibody/BCR;
iv. IP Module 2, which is a tandem-engineered BCR presenting cell (T-eBPC) containing a Tandem Genomic Receiver Site (TGRS) and optionally a synthetic reporter construct to report BCR engagement;
d. a first manufacturing part (MP1), comprising
a. MP1 Module 1, which is a module for combining IP Module 1 and IP module 2 whereby the sequences of a vector from IP Module 1 is inserted into the Tandem Genomic Receiver Site (TGRS) of a T-eBPC of IP Module 2, to obtain T-eBPC cells presenting a BCR (T-eBPC-b), wherein the BCR is encoded by those IgH and IgL sequences transferred from the TIVL of IP Module 1 to the TGRS of IP Module 2;
b. MP1 Module 2, which is a 2-step sequence recovery module for recovering said IgH and/or IgL sequences of the T-eBPC-b, said sequence recovery module comprises reverse transcription primers (R-Prim) for deriving reverse transcriptase amplicons (R-Amp) from IgH and/or IgL chain sequences encoded in the TGRS of the T-eBPC-b in a first sequence recovery step, and further comprising polymerase chain reaction primers (P-Prim) to amplify the R-Amp products in second sequence recovery step; wherein said second sequence recovery step comprises either high-fidelity or low-fidelity polymerase chain reaction components;
c. an output part (OP), which contains the product resulting from MP1 Module 2.

2. A tandem-manufacturing system according to claim 1, wherein the output part (OP) is OP Module 1 or OP Module 2, wherein OP Module 1 comprises a high-fidelity polymerase chain reaction amplicon (HFP-Amp), and wherein OP Module 2 comprises a low-fidelity polymerase chain reaction amplicon (LFP-Amp).

3. A tandem-manufacturing system according to claim 1 or 2 further comprising MP2 Module 1, which is a second manufacturing part containing a Backbone entry vector and a tandem Constant only (TaC-only) vector for combining with the amplicons of the OP Module(s).

4. A method for human antibody engineering, the method comprises
i) reacting a vector of a TIVL, the vector comprises a pair of IgH and IgL sequences each pair encoding together an antibody/BCR with a tandem-engineered BCR presenting cell (T-eBPC) containing a Tandem Genomic Receiver Site (TGRS) and optionally a synthetic reporter construct to report BCR engagement to obtain a T-eBPC-b;
ii) contacting the T-eBPC-b with a target epitope (tEp) to select one or more T-eBPC-b on the basis of tEp engagement;
iii) subjecting in a first sequence recovery step (MP1 Module 2), the one or more T-eBPC-b selected to reverse transcription primers (R-Prim), for deriving reverse transcriptase amplicons (R-Amp) from IgH and/or IgL chain sequences encoded in the TGRS of the T-eBPC-b;
iv) subjecting in a second sequence recovery step (MP1 Module 2), the R-Amp to polymerase chain reaction primers (P-Prim) to amplify the R-Amp products, wherein said second recovery step comprises either high-fidelity or low-fidelity polymerase chain reaction components to obtain HFP-Amps and LFP-Amps, respectively.

5. A method according to claim 4 further comprising combining amplicons from step iv) with a Backbone entry vector and a tandem Constant only (TaC-only) vector.

6. A method according to claim 4, wherein said LFP-Amps or HFP-Amps are used for re-creation of a TIVL with IgH/ IgL sequences amplified from selected T-eBPC-b cells, in order to recreate TIVL vectors encoding faithfully those selected sequences (using HFP-Amps) or with introduced sequence diversity (using LFP-Amps), wherein the resulting vector library is termed a TIVL'.

7. A method according to claim 6, wherein one or more vectors of TIVL' is used as starting material in step i) of claim 9.

8. A dual-manufacturing system for generating antibody sequences, comprising
c. an input part (IP), comprising:
i. IP Module 3, which is a dual integration vector library (DIVL), each vector comprises either a IgH (DIVL-H) or a IgL (DIVL-L) sequence, wherein the IgH (DIVL-H) and the IgL (DIVL-L) sequences together encode an antibody/BCR;
ii. IP Module 4, which is a dual-engineered BCR presenting cell (D-eBPC) containing a pair of Dual Genomic Integration Sites (DGRS), which is Heavy (DGRS-H) and Light (DGRS-L) and optionally a synthetic reporter construct to report BCR engagement.
d. a first manufacturing part (MP1), comprising
i. MP1 Module 3, which is a module for combining IP Module 3 and IP Module 4, whereby the sequences of a DIVL-H and DIVL-L vector from IP Module 3 are inserted into the DGRS-H and DGRS-L, respectively, of a D-eBPC of IP Module 4 to obtain D-eBPC cells presenting a BCR (D-eBPC-b), wherein the BCR is encoded by those IgH and IgL sequences transferred from the DIVL-H and DIVL-L of IP Module 3 to the DGRS of IP Module 4;
ii. MP1 Module 2, which is a 2-step sequence recovery module for recovering said IgH and/or IgL sequences of said D-eBPC-b, said sequence recovery module comprises reverse transcription primers (R-Prim) for deriving reverse transcriptase amplicons (R-Amp) from IgH and IgL chain sequences encoded in the DGRS-H and DGRS-L of the D-eBPC-b in a first sequence recovery step, and further comprising polymerase chain reaction primers (P-Prim) to amplify the R-Amp products in a second sequence recovery step, wherein said second sequence recovery step comprises either high-fidelity or low-fidelity polymerase chain reaction components;
c. an output part (OP), which contains the products resulting from MP 1 Module 2.

9. A dual-manufacturing system according to claim 8, wherein the output part (OP) is OP Module 1 or OP Module 2, wherein OP Module 1 comprises a high-fidelity polymerase chain reaction amplicon (HFP-Amp), and wherein OP Module 2 comprises a low-fidelity polymerase chain reaction amplicon (LFP-Amp).

10. A dual-manufacturing system according to claim 8 or 9 further comprising MP2 Module 2, which is a second manufacturing part containing either a Heavy Constant-Only (HC-only), or a Light Constant-only (LC-only) vector, for combining with the amplicons of the OP Modules.

11. A method for human antibody engineering, the method comprises
i) reacting a) a vector of DIVL-H, the vector comprising a IgH sequence, and b) a vector of DIVL-L, the vector comprising a IgL sequence, the sequences encoding together an antibody/BCR, with c) a dual-engineered BCR presenting cell (D-eBPC) containing a Dual Genomic Receiver Site (DGRS) and optionally a synthetic reporter construct to report BCR engagement to obtain a D-eBPC-b;
ii) contacting the D-eBPC-b with a target epitope (tEp) to select one or more D-eBPC-b on the basis of tEp engagement;
iii) subjecting in a first sequence recovery step (MP1 Module 2), the one or more D-eBPC-b selected to reverse transcription primers (R-Prim), for deriving reverse transcriptase amplicons (R-Amp) from IgH and/or IgL chain sequences encoded in the DGRS of the D-eBPC-b;
iv) subjecting in a second sequence recovery step (MP1 Module 2), the R-Amp to polymerase chain reaction primers (P-Prim) to amplify the R-Amp products, wherein said second sequence recovery step comprises either high-fidelity or low-fidelity polymerase chain reaction components to obtain HFP-Amps and LFP-Amps, respectively.

12. A method according to claim 11 further comprising combining at least one amplicon from step iv) with at least one of a Heavy Constant-Only (HC-only) and a Light Constant-only (LC-only) vector.

13. A method according to claim12, wherein said LFP-Amps or HFP-Amps are used for re-creation of at least one DIVL with a IgH or a IgL sequence amplified from selected D-eBPC-b cells, in order to recreate DIVL vectors encoding faithfully those selected sequences (using HFP-Amps) or with introduced sequence diversity (using LFP-Amps), wherein the resulting vector library is termed a DIVL-H' or DIVL-L'.

14. A method according to claim 13, wherein one or more vectors of DIVL-H' or DIVL-L' is used as starting material in step i) of claim 11.

15. A combined tandem-dual manufacturing system comprising a tandem-manufacturing system as defined in any one of claims 1-3 and a dual-manufacturing system as defined in any one of claims 9-10, wherein the second manufacturing part, MP2 Module 2, defined in claim 15 is included.

16. A method for human antibody engineering, the method comprises using the combined tandem-dual manufacturing system according to claim 15.

17. A method according to claim 16, wherein said LFP-Amps or HFP-Amps are used for re-creation of at least one TIVL with IgH and IgL sequence amplified from selected D-eBPC-b cells, in order to recreate TIVL vectors encoding faithfully those selected sequences (using HFP-Amps) or with introduced sequence diversity (using LFP-Amps), wherein the resulting vector library is termed a TIVL'.

18. A method according to claim 16, wherein said LFP-Amps or HFP-Amps are used for re-creation of at least one DIVL with IgH and/ or IgL sequence amplified from selected T-eBPC-b cells, in order to recreate DIVL vectors encoding faithfully those selected sequences (using HFP-Amps) or with introduced sequence diversity (using LFP-Amps), wherein the resulting vector library is termed a DIVL'.

19. A method according to claims 17, wherein one or more vectors of TIVL' is used as starting material in step i) of claim 4.

20. A method according to claims 18, wherein one or more vectors of TIVL' is used as starting material in step i) of claim 8.

21. A manufacturing system as defined in any one of claims 1-3, 8-10 or 15 to provide one or more antibodies against tEp.
